# EUROPEAN PATENT APPLICATION

(11) **EP 2 837 381 A1**
(43) Date of publication of application: **18.02.2015**
(21) Application number: 12856740.1
(22) Date of filing: 13.12.2012
(51) Int. Cl.: A61K 31/445, A61P 35/00

(54) **USE OF AGENTS THAT ALTER THE PERITUMORAL ENVIRONMENT FOR THE TREATMENT OF CANCER**

(30) Priority: 13.12.2011 ES 201101311
(71) Applicant: Servicio Andaluz de Salud, 41071 Sevilla (ES)
(72) Inventor: SALINAS MARTÍN, Manuel Vicente, E-41002 Sevilla (ES); LARA RUIZ, María Carmen, E-41002 Sevilla (ES)
(74) Representative: Illescas, Manuel
(86) International application number: PCT/ES2012/070865
(87) International publication number: WO 2013/087964

(57) **Abstract**

The invention relates to the use of agents that alter the peritumoral environment, specifically non-peptide NK1 receptor antagonists, for the treatment of cancer. The peritumoral environment is formed by the stromal cells, the stromal matrix, intra- and peri-tumoral vascularization and the cells responsible for the inflammatory and/or immune response around the tumor. The result of the alteration to the peritumoral environment is a reduction in the size of the tumor, the prevention of its development and, optionally, the induction of its disappearance. The invention also relates to pharmaceutical compositions containing said peritumoral-environment-altering agents, either alone or combined with at least one other active ingredient, for the treatment of cancer.

## Description

### FIELD OF THE INVENTION

The present invention relates to the field of medicine. More specifically in molecular biology applied to medicine, pharmacology and oncology. Specifically, it relates to the use of agents that alter the peritumoral environment, preferably non-peptidic antagonists of the NK1 receptors, for the manufacture of medicaments useful in the treatment of cancer in humans.

### BACKGROUND OF THE INVENTION

NK1 receptors (neuropeptide receptor for substance P and the tachykinins), are widely distributed in the body cells. Its presence has been found in the central and peripheral nervous system of mammals, in the digestive tract, the circulatory system, hematopoietic and inflammatory and/or immune response cells, as well as in soft tissue, in particular in the vascular endothelium. Multiple biological processes are currently known where NK1 receptors are involved in their regulation.

Substance P (PS) is a naturally occurring undecapeptide, which belongs to the family of tachykinins, it is produced in mammals and its sequence was described by Veber et al, (U.S. 4,680,283). Tachykinins also includes other peptides as Neurokinin A, Neurokinin B, Neuropeptide K, Neuropeptide gamma and Hemokinina I, among others. The involvement of SP and other tachykinins in the aetiopathogenesis of several diseases has been widely reported in scientific literature. In this regard, the action of tachykinins has been related to the aetiopathogenesis of human nervous system diseases, such as Alzheimer's Disease, Multiple Sclerosis, Parkinson's Disease, anxiety, and depression (Barker R. et al., 1996; Kramer MS, et al., 1998). The involvement of tachykinins has been also been evidenced in the aetiopathogenesis of several diseases with inflammatory component, such as rheumatoid arthritis, asthma, allergic rhinitis, inflammatory bowel diseases like ulcerative colitis and Crohn's disease (Maggi CA, et al., 1993).

In this sense, non-peptide antagonists of NK1 receptors have been developed as medicaments for the treatment of several central nervous system disorders, such as depression, psychosis and anxiety (WO 95/16679, WO 95/18124, WO 95/23798, and WO 01/77100). It has been described that the use of selective NK1 receptor antagonists is useful for the treatment of nausea and vomiting induced by anticancer chemotherapy agents, as well as for the treatment of some forms of urinary incontinence (Quartara L. et al., 1998; Doi T. et al., 1999).

In a study published in 2003 (Giardina G, et al., 2003), a review was made of the most recent patents on NK1, NK2 and NK3 receptor antagonists. The molecules from the most important world manufacturers are described, indicating their possible applications, including mainly: antidepressive, anti-inflammatory, anxiolytic, antiemetic, treatment of ulcerative colitis, and others.

The article published by Antal Orosz et al., (Orosz A, et al., 1995) describes the use of several PS antagonists to inhibit the proliferation of lung carcinoma cells (e.g. target cells NCI-H69). Also, in the article by Bunn PA Jr (Bunn PA Jr. et al., 1994) describes the SP antagonists capable of inhibiting the growth *in vitro* several lung cancer cell lines (e.g. designated NCI-H510 cells NCI-H345 and SHP-77).

In the article published by Palma C et al. (Palma C. et al., 2000), it is described that the astrocytes of the central nervous system express functional receptors for several neurotransmitters, including NK1 receptors. In brain tumors, malignant glial cells derived from astrocytes trigger - under the action of tachykinins and by mediation of NK1 receptors - the secretion of mediators increasing their proliferation rate. Consequently, selective NK1 antagonists can be very useful as therapeutic agents for the treatment of malignant gliomas.

Patent EP 773026 (Pfizer) makes reference to the use of non-peptide NK1 receptor antagonists for the treatment of cancer in mammals. In particular in the treatment of small lung carcinoma, APUDOMAS (Amine Precursor Uptake and Decarboxylation, enterochromaffin cell tumors located mainly in the digestive tube mucosa), neuroendocrine tumor, and small extra-pulmonary carcinoma.

On the other hand, patent WO 2001001922 describes the use of NK1 receptor antagonists for the treatment of adenocarcinoma and very specifically, prostate carcinoma.

Several studies with specific antagonists of neurokinin NK receptors such as CP-96341-1-(Pfizer), MEN 11467, SR 48968 (Sanofi) and MEN 11420 (Nepadutant) have shown their efficacy in blocking cell proliferation (Singh D et al., 2000; and Bigioni M. et al., 2005).

Several studies with specific antagonists of neurokinin receptors such as NK CP-96341-1 (Pfizer), MEN 11467, SR 48968 (Sanofi) and MEN 11420 (Nepadutant) have demonstrated their effectiveness in blocking cell proliferation (Singh D et al., 2000; and Bigioni M. et al., 2005).

It has been described that non-peptide NK1 receptor antagonists induce apoptosis (cell death) in tumor cells from various tumors, such as stomach carcinoma, colon carcinoma (Rosso M, et al., 2008) or melanoma (Muñoz M, et al. 2010). Furthermore, it has been described that these receptors are over-expressed in cancer cells.

Patent ES 2 246 687 claims the use of non-peptide antagonists of NK1 and SP in the preparation of a pharmaceutical composition for the induction of apoptosis in mammalian tumor cells. Furthermore, the patent application WO2012020162 describes the use of antibodies or fragments thereof, against NK1, NK2 and/or NK3 receptors, useful in the treatment of cancer by induction of apoptosis in tumor cells. In this regard, it is noteworthy that there are two types of peptide antagonists of NK1 receptors: the polypeptides (amino acid sequences -10 to 20) that are the first to be synthesized, but were abandoned, - we must take into account that agonists as substance P are polypeptides, but in this case synthesized by the body-) and monoclonal antibodies. The advantages of non-peptidic antagonists of the NK1 receptor compared to the peptide antagonist are mainly that the non-peptidic have potentially fewer side effects (peptide or monoclonal antagonists can generate the body's immune response against themselves) and additionally, non-peptidic antagonists can be administered orally and are easier to synthesize.

On the genesis and development of cancer, not only the molecular mechanisms of tumor cells are involved, but cells surrounding the tumor have great importance, specifically stromal cells and inflammatory cells, as well as the interactions between the tumor cells and the cells surrounding the tumor (stromal cells and inflammatory cells) (McAllister SS. Et al. 2010; Ikushima H. et al. 2010). Also, some substances, for example the nuclear factor NF-KB (nuclear factor kappa B), TGF-β (transforming growth factor β), the SPARC (the English term "secreted protein acidic, cysteine-rich") or TGF-α (transforming growth factor alpha) have been demonstrated to be present in the tumor microenvironment and are important in the genesis and progression of tumors (Coussens L et al. 2002). TGF can inhibit the passage of lymphocyte precursor forms of CD8 + effector cell forms (Berzofsky JA, et al., J Clin Invest. 2004, 113: 1515-1525). SPARC plays a role associated with tumor neoangiogenesis (Carmeliet P et al. 2000). In this regard, neoangiogenesis, immunity and inflammation are key factors for tumor progression (Hanahan D et al. 2000).

The importance of some molecules produced by fibroblasts in cancer progression is well known and the use of them as therapeutic targets has been even proposed for the treatment of cancer by the use of monoclonal antibodies specific against them (Welt S, et al. 1994).

Specifically, and related to the microenvironment around the tumor, metalloproteinases (MMPs) are enzymes which contain a metal (zinc) and degrade extracellular matrix proteins (collagen IV, laminin, elastin, fibronectin, proteoglycans, etc.). They are expressed physiologically in some situations, such as wound healing, transition from cartilage to bone, and placental development. They are expressed pathologically in the process of invasion and development of metastasis in tumors (Clin Cancer Res 2000, 6: 2349). The MMPs most commonly involved in tumor development are: MMP-2 (gelatinase A), MMP-3 (Stromelysin 1), MMP-7 (matrilysin) MMP-9 (gelatinase B), MMP-11 (Stromelysin 3), MMP-13 (Collagenase 3) and MMP-14. There are several drugs known as metalloprotease inhibitors that are currently tested for the treatment of cancer: Batimastat (whose targets are peptidomimetic MMP-1, 2, 3, 7 and 9), Marimastat (peptidomimetic with MMP-1,2,3,7 and 9 as targets), Prinomastat (non-peptidomimetic, with MMP-2,3,9,13 and 14 as targets), Bay-129566 (non-peptidomimetic, with MMP-2,3 and 9 as targets), metastat (tetracycline with MMP-2 and 9 as targets), BMS 275291 (non-peptidomimetic with MMP-2 and 9 as targets), and Neovastat (obtained from shark cartilage, with MMP-1,2,7,9,12 and 13 as targets). Specifically, Marimastat has completed Phase I clinical trials on breast cancer and non-small cell type lung, Prinomastat has completed Phase I clinical trials in prostate cancer, Bay-129566 has completed Phase I clinical trials in several solid tumors, and BMS 275291 has completed Phase I clinical trials in non-small cell lung cancer. In addition, it has been reported that the use of Marimastat has been shown to be effective for the treatment of advanced pancreatic cancer (Rosemurgy et al., PROCC ASCO 1999), for the treatment of advanced gastric cancer (Fieldng et al., ASCO PROCC 2000), for glioblastoma (Puphanich et al., ASCO PROCC 2001) and for breast cancer after first-line chemotherapy treatment (Sparano et al., ASCO PROCC 2002).

Therefore, in conclusion, the following facts are currently known in the state of art:
1. NK1 receptors are widespread in the human organism.
2. Tachykinins and specifically Substance P act on the NK1 receptor.
3. Non-peptide NK1 receptors antagonists can be used for the manufacture of a medicament for the treatment of several central nervous system disorders, such as depression, psychosis and anxiety, which has been the subject of claim in several patent applications (WO 95/16679, WO 95/18124, WO 95/23798, and WO 01/77100)..
4. The use of non-peptide NK1 receptor antagonists has shown effects on tumor cells, resulting in apoptosis (cell death) thereof (ES 2246687).
5. That, as stated, patent ES 2 246 687 claims the use of non-peptide NK1 receptor antagonists and substance P (and lists a given number of them, specifically). Therefore, it does not include that the effects of non-peptidic antagonists of the NK1 receptor can be performed at the level of cells and substances that compose the tumor microenvironment and are of crucial importance for the genesis, development and progression of tumors.
6. The presence of NK1 receptors has been demonstrated in the blood cells involved in the inflammatory and/or immune response, in stromal matrix cells and in the cells of vascularization around the tumor cells. It is known that stromal cells, the blood cells involved in inflammatory and/or immune response and the cells of vascularization influence tumor progression.

However, the known prior art, including using non-peptide NK1 receptors antagonists for inducing death and/or apoptosis in tumor cells, the objective of this invention, as well as the technical advantage contributed, is the use of modulating agents of peritumoral environment, preferably selected from non-peptide receptor NK1 antagonists for cancer treatment, thanks to the ability to modify the environment shown by inducing peritumoral changes in cells that make up this environment and substances secreting them, in order to prevent or hinder the genesis, development or progression of tumors, and reduce the size thereof. Therefore, the present invention discloses the use of modifying peritumoral environment agents, preferably non-peptidic NK1 antagonists for the manufacture of a medicament or pharmaceutical composition useful in the therapeutic treatment of cancer by direct administration to a mammal, including humans.

In the prior art, it is known that the NK1 receptor activates cell proliferation, preferably via the MAP kinases pathway, specifically through its efferent "downstream" such as ERK. ERK can modulate cell proliferation through various efferent turns "downstream" of great importance as Fos/Jun or p90rsk, among others. Moreover, is also known in the prior art that the NK1 receptor activates cell proliferation, preferably via the route of the PI3 Kinase. Activation of the PI3 Kinase pathway causes increased afferent "downstream" as AKT. AKT exerts an anti-apoptotic effect through several cell efferent turns "downstream" of great importance as Bcl2 or a stimulating effect on cell proliferation, for example through the Cyclin D. The present invention demonstrates that non-peptide NK1 antagonists are capable of inhibiting tumor growth and proliferation in those types of tumors in which the signaling pathways of MAP kinases and PI3 kinases are upright in these cells, i.e., are active, and therefore treatment with these antagonists inhibit the proliferation of tumor cells through the MAP kinases and PI3 kinases pathway, preferably by inhibiting the expression of different effectors "downstream" of these routes, including ERK and AKT, respectively. In contrast, the present invention demonstrates that exits specific tumors where the treatment with non-peptide NK1 receptors antagonists is unable to inhibit either their growth, or the activation of the MAP kinases and PI3 Kinase signaling pathways. But when this type of tumors are cultured in the presence of the cells that shap the peritumoral microenvironment (stromal cells - fibroblasts or immunity cells/inflammatory-leukocytes and macrophages, and vascular endothelial cells) inhibition of their proliferation occurs, irrespective of MAP kinases or PI3 kinases signaling pathways. This fact shows that the non-peptide NK1 receptor antagonists inhibit the survival of tumor cells by mechanisms related to blocking the secretion of molecules characteristic of the interaction of tumor cells with other cells characteristic of the tumor microenvironment, being such different mechanisms known in the prior art.

The cell pathway which, starting from NK1 receptors ends in the inhibition of cell apoptosis may be altered, for example due to "downstream" mutations in the signaling pathway of the NK1 receptor itself. In these cases, the administration of non-peptide NK1 receptor antagonists would have no predictable effect in induction of apoptosis of tumor cells. In these cases, the antitumor action should be exerted:
(i) Inducing apoptosis by means other than through NK1 and/or
(ii) Influencing the peritumoral environment to reduce tumor size and/or prevent their development and/or to disappear.

In any case, determine the integrity of the metabolic pathway NK1 receptor mediated in cancer cases will determine:
a) Adjusting the effective dose NK1 antagonists, in particular and/or,
b) Adjusting the effective dose of agents that alter the peritumoral environment in general and/or,
c) Selecting the type of antitumoral to administer in combination, possibly with the agent that alter the peritumoral environment in general or, more specifically, with the non-peptide antagonist NK1 receptor.

The fact that the invention is aligned in the treatment of cancer through peritumoral environmental modification to reduce tumor size, inhibit its development and eventually induce their disappearance, allows adjustment of the effective doses of antitumoral agents, both chemo-radiotherapy based, as well as combinations with the same effect. This implies a more effective treatment, applied to a wider range of tumors and with tighter doses, which means fewer associated side effects and a higher quality of life for patients during and after treatment.

### DESCRIPTION OF THE INVENTION

### Brief description of the invention

An object of the present invention is the use of at least one modifying peritumoral environment agent, preferably non-peptide NK1 receptor antagonists, for the manufacture of a medicament or pharmaceutical composition useful in the treatment of cancer.

The microenvironment around the tumor is formed by all the cells surrounding the tumor, preferably said environment, is formed by the stromal cells, preferably fibroblasts, stromal matrix, vascular endothelial cells and peritumoral intra and inflammatory cells and/or surrounding the immune tumor, preferably mono- and polymorphonuclear leukocytes and macrophages. The modification of the peritumoral microenvironment is carried out by inducing changes in the stromal matrix and peritumoral cells, as well as inflammatory and/or immune response, leading to inhibition of tumor development and/or progression, thus being useful in the treatment of cancer. Changes in the peritumoral microenvironment produced by treatment - with at least one agent that alters the peritumoral environment being preferred non-peptide receptor NK1 antagonists - can be summarized as the modification of the immunophenotype of cells that make up this microenvironment, preferably fibroblasts, inflammatory cells and vascular endothelial cells, preferably in relation to the synthesis of key molecules in the progression of tumors, such as MMPs, NF-KB, TGF and SPARC. Another modification of the microenvironment around the tumor, by treatment with non-peptide receptor NK1 antagonists, refers to the inhibition of neo-angiogenesis by inhibiting the proliferation of vascular endothelial cells, determinant of tumor progression. Therefore, the use of non-peptide receptor NK1 antagonists leads to changes in the cells that comprise the tumor microenvironment, fibroblastic cells (stroma), vascular endothelial cells (vessels) and cells involved in the inflammatory and immune response (which cause the growth and perpetuation of tumors through the interaction between stromal cells and cancer) such modifications being beneficial for the treatment of cancer. These changes in the tumor microenvironment are designed to reduce tumor size or their complete removal, as well as preventing the development and progression thereof.

Therefore, for purposes of the present invention the agents that alter the peritumoral environment is any substance of peptidic or non-peptidic nature, having the ability to modify the peritumoral environment as previously defined. Modifying agents are preferably peritumoral environment non-peptide NK1 receptors. As used herein "non-peptide NK1 receptor antagonist" means any non-peptide substance of sufficient size and conformation suitable for binding at the NK1 receptor and thus inhibit its normal operation, including [the fact prevent] or other SP agonists of these receptors bind to said receptors. Preferably, in the present invention the following commercial non-peptide NK1 were tested: L-733,060 ((2S, 3S) -3- [(3,5-bis (Trifluoromethyl) phenyl) methoxy]-2-phenylpiperidine hydrochloride) (Sigma-Aldrich), L-732, 138 (N-Acetyl-L-tryptophan 3,5-bis (trifluoromethyl) benzyl ester) (Sigma-Aldrich), L-703.606 (cis-2-(Diphenylmethyl) - N-[(2-iodophenyl) methyl]-I-azabicyclo [2.2.2] octan-3-amine oxalate salt) (Sigma-Aldrich), WIN 62,577 (Sigma-Aldrich), CP-122721 (Pfizer), Aprepitant or MK 869 or L-754 030 (MSD), TAK-637 (Takeda/Abbot), Vestipitant or GW597599 (GSK), Casopitant or GW679769 (GSK) and R673 (Roche). CP-100263, WIN 51708, CP-96345, L-760 735. Similarly, other compounds can be used non-peptide NK1 and SP such as Vofopitant or GR-205171 (Pfizer), or CJ Ezlopitant - January 1974 (Pfizer), CP-122721 (Pfizer), L- 758 298 (MSD), L-741 671, L-742 694, CP-99994, Lanepitant or LY-303870, T-2328, LY-686 017. Preferred are compounds: aprepitant or MK-869 or L 754030 (MSD), Vestipitant or GW597599 (GSK) and Casopitant or GW679769 (GSK).

"Cancer" refers to a malignant tumor of potentially unlimited growth that expands locally by invasion and systemically by metastasis. According to the present invention, the non-peptide antagonist of the NK1 receptor is administered to individuals with a cancer.

Another of the objects described in the present invention refers to a method of treatment directed to the modification of the microenvironment around the tumor by administering to a patient suffering from cancer an effective amount of at least one agent that alters the peritumoral environment, preferably a peptide NK1 receptor antagonist. The treatment method described herein is useful for patients suffering from cancer in the asymptomatic, symptomatic, in neoadjuvant therapy (treatment before surgery) in adjuvant therapy (adjuvant treatment after surgery, when no detectable macroscopic tumor is present) and in treatment of metastatic stage disease.

Another object of the present invention is a composition, preferably pharmaceutical comprising at least one modifying agent selected from peritumoral environment:
(i) an agent capable of inhibiting cell mediated neoangiogenesis of the seed and/or vascular
(ii) an agent capable of inhibiting the synthesis, by fibroblastic cells of markers selected from any of the following: TGF-α, TGF-β 1, TGF-β 2, TGF-β 3 SPARC MMP-3, MMP-7, MMP-9, MMP-11, MMP-13 and MMP-14 and/or
(iii) an agent capable of inhibiting the synthesis, by cells of the immune lineage and/or inflammatory, of markers selected from any of the following: TGF-β, NF-kB, EGF, MMP-9, VEGF and TNF-α, or combinations thereof, and is useful in cancer treatment.

The aforesaid pharmaceutical composition may further comprise carriers and/or excipients agents that alter the environment agents are preferred peritumoral non-peptide NK1 receptor. The pharmaceutical composition or medicament comprising at least one agent that alters the peritumoral environment, preferably a non-peptide NK1 receptor antagonist, is present in a pharmaceutically acceptable form for administration to an individual directly, preferably by intravenous, oral, parenteral, or any other means. Intravenous administration relates directly to the application of the antagonist or pharmaceutical composition comprising it, directly into the patient's bloodstream. Oral administration may involve swallowing, so that the antagonist, and a pharmaceutical composition comprising it, enters the gastrointestinal tract, or may be used buccal or sublingual administration by which the compound enters the blood stream directly from the mouth. Parenteral administration refers to routes of administration other than enteral, transdermal, inhalation, and is typically by injection or infusion. Parenteral administration includes intravenous injection or infusion, intramuscular and/or subcutaneous.

The term "drug" or "pharmaceutical composition", as used herein, refers to any substance used for prevention, diagnosis, alleviation, treatment or cure of disease in man and animals. In the context of the present invention, the disease is cancer, preferably gastric carcinoma, gastric adenocarcinoma, more preferably, colon cancer, most preferably adenocarcinoma of the colon, carcinoma of the pancreas, most preferably adenocarcinoma of the pancreas, breast cancer, most preferably adenocarcinoma breast and/or breast carcinoma, ovarian carcinoma, most preferably adenocarcinoma of the ovary and/or ovarian carcinoma, endometrial carcinoma, choriocarcinoma, cervix carcinoma, lung carcinoma, more preferably lung adenocarcinoma, lung carcinoma non-small cell and/or lung carcinoma, small cell carcinoma of the thyroid, more preferably human papillary thyroid carcinoma metastasizing and/or follicular thyroid carcinoma, bladder carcinoma, more preferably carcinoma of urinary bladder and/or transitional cell carcinoma urinary bladder carcinoma, prostate carcinoma CNS glial, sarcoma, more preferably fibrosarcoma, malignant fibrous histiocytoma, Edwing sarcoma, human endometrial stromal sarcoma, osteosarcoma and/or rhabdomyosarcoma, melanoma, embryonal carcinomas, more preferably neuroblastoma, neuroblastoma bone marrow, and/or retinoblastoma and haematological cancers, more preferably leukemia cell T/NK, lymphoblastic B leukemia, lymphoblastic T leukemia, lymphoblastic leukemia B, Burkitt lymphoma, Hodgkin lymphoma, T lymphoma and/or multiple myeloma.

As used herein, the term "active ingredient", "active substance", "pharmaceutically active substance", "active ingredient" or "active pharmaceutical ingredient" means any component that potentially provides pharmacological activity, or different effect in the diagnosis, cure, mitigation, treatment, or prevention of disease, or to affect the structure or function of the body of man or other animals. The term includes those components that promote a chemical change in the drug development and are present therein in a modified form intended to provide specific activity or effect.

Furthermore, the agents that alter the peritumoral environment, preferably non-peptidic NK1 receptor antagonists of the invention, may be administered alone or in a composition with carriers and/or excipients. A person skilled in the art will adapt the composition depending upon the particular form of administration. In the case of administering a purified antibody, oral administration is the preferred method and is preferably achieved through solid dosage forms, including capsules, tablets, pills, powders and granules, among others, or liquid dosage forms. The preparations of agents that alter the peritumoral environment for parenteral administration preferably include sterile aqueous or non-aqueous sterile, suspensions or emulsions, among others. The term "pharmaceutically acceptable carrier" refers to a vehicle that must be approved by a regulatory agency of the federal or a state government or listed in the U.S. Pharmacopoeia or the European Pharmacopoeia or other generally recognized pharmacopeia for use in animals, and more specifically in humans. Similarly, the suitable pharmaceutically acceptable excipients will vary depending on the particular dosage form selected. In addition, suitable pharmaceutically acceptable excipients may be chosen for a particular function that can be in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate transport of the compound or compounds of the invention once administered to the patient from one organ, or part of the body to another organ or body part. Certain pharmaceutically acceptable excipients may be chosen for their ability to enhance patient acceptance. Suitable pharmaceutically acceptable excipients include the following types of excipients, without excluding others known in the prior art: diluents, fillers, binders, disintegrants, lubricants, glidants, granulating agents, coating agents, moisturizing agents, solvents, co- Solvents, suspending agents, emulsifiers, sweeteners, flavorings, taste masking agents, coloring agents, anti-caking agents, wetting agents, chelating agents, plasticizers, viscosity increasing agents, antioxidants, preservatives, stabilizers, surfactants and buffering. The skilled artisan will appreciate that certain pharmaceutically acceptable excipients may perform more than one function and can perform alternative functions depending on the amount of excipient that is present in the formulation and what other ingredients are present in the formulation. Specialists have the knowledge and skill in the art that allow them to select suitable pharmaceutically acceptable excipients in appropriate amounts for use in the invention. Furthermore, there are several resources available to the specialist that describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The dosage of the active ingredient, in this case, agent that alters the peritumoral environment, may be selected depending on the desired therapeutic effect, the route of administration and the duration of treatment. Administration dosage and frequency will depend on the size, age and general health condition of the individual, taking into account the possibility of side effects. The administration also depends on the simultaneous treatment with other drugs and the individual's tolerance to the drug administered. Skilled practitioners may set the proper dose using standard procedures. The dose should be the effective amount of the active modulator peritumoral environment, preferably non-peptide antagonist of NK1, in the sense that the treatment is at least the same or better effect than current therapies for these patients.

The composition may comprise at least one agent that alters the peritumoral environment used as a single agent in the treatment of cancer, or combinations thereof with other therapeutic agents depending on the condition, preferably selected from agents capable of inducing apoptosis in tumor cells and/or chemotherapy agents and/or radiation agents.

All technical and scientific terms used herein have the same meaning commonly understood by a person skilled in the field of the invention, unless otherwise defined. Methods and materials similar or equivalent to those described herein can be used in the practice of the present invention. Throughout the description and claims the word "comprise" and its variants are not limitative and therefore are not intended to exclude other technical features, additives, components or steps. By contrast, the word "consist" and its variants do describe limited content, referring only to the technical features, additives, components, or steps that accompany it. For those skilled practitioners, other objects, advantages and features of the invention will become apparent from the specification and practice of the invention.

### Detailed description of the invention

One objective of the present invention refers to the use of at least one modifying agent selected from peritumoral environment:
(i) an agent capable of inhibiting neoangiogenesis, by inhibiting the proliferation of vascular cell lineage and/or
(ii) an agent capable of inhibiting the synthesis, by cells of the fibroblastic lineage of markers selected from either of: TGF-α, TGF-β 1, TGF-β 2, TGF-β 3, SPARC, MMP-3, MMP-7, MMP-9, MMP-11, MMP-13 and MMP-14 and/or
(iii) an agent capable of inhibiting the synthesis by cells of the immune lineage and/or inflammatory markers selected from any of the following: TGF-β, NF-kB, EGF, MMP-9, VEGF and TNF-α,
or combinations thereof, for the manufacture of a medicament or pharmaceutical composition useful in treating cancer, or either to at least one agent that alters the peritumoral environment, as previously defined, for use in cancer treatment. As evidenced agents that alter the peritumoral environment are capable of reducing the size of tumors in addition to inhibiting their growth and spread.

In a preferred embodiment, the use of agents that alter the peritumoral environment or agent of the invention is characterized in that the endothelial lineage cells are preferably vascular endothelial cells, the cells are preferably fibroblastic cells and fibroblasts the lineage immune and/or inflammatory mononuclear leukocytes are preferably, polymorphonuclear leukocytes and macrophages.

In another preferred embodiment, the use agents that alter the peritumoral environment or agent of the invention is characterized in that the modifying agent is an antagonist peritumoral environment non-peptide NK1. In another preferred embodiment, antagonists non-peptide NK1 receptors are selected from any of the following: Aprepitant, Vestipitant, Casopitant, Vofopitant, Ezlopitant, Lanepitant, LY-686017, L-733,060, L-732,138, L -703,606, WIN 62,577, CP-122721" TAK-637, and R673, CP-100263, WIN 51708, CP-96345, L-760 735, CP-122721, L-758 298, L-741 671, L-742 694, CP-99994, T-2328, being particularly preferred antagonists selected from: Aprepitant, Vestipitant, Casopitant, Vofopitant, Ezlopitant and Lanepitant.

In another preferred embodiment, the use of modifying peritumoral environment agent oritself agent to the invention is characterized in that the medicament or pharmaceutical composition useful in treating cancer comprising at least one other active principle which induces apoptosis in tumor cells. In a preferred embodiment, the principle that induces apoptosis in tumor cells is selected from any of the following: Chlorambucil, Melphalan, Aldesleukin, 6-mercaptopurine, 5-fluorouracil, Ara-c, Bexarotene, Bleomycin, Capecitabine, Carboplatin, Cisplatin, Docetaxel, Doxorubicin, Epirubicin, Fludarabine, Irinotecan, Methotrexate, Mitoxantrone, Oxaliplatin, Paclitaxel, Rituximab, etoposide, teniposide, vincristine, vinblastine, vinorelbine, imatinib, erlotinib, cetuximab, and Trastuzumab.

In another preferred embodiment, the use of modifying peritumoral environment agent or itself agent to the invention is characterized in that it is administered together with another anticancer agent selected from any of the following: agent chemotherapy or radiotherapy agent.

Another of the objects described in the present invention refers to a composition comprising at least one modifying agent selected from peritumoral environment:
(i) an agent capable of inhibiting neoangiogenesis, by inhibiting the proliferation of vascular cell lineage and/or
(ii) an agent capable of inhibiting the synthesis, by cells of the fibroblastic lineage, of markers selected from any of the following: TGF-α, TGF-β 1, TGF-β 2 TGF-β 3, SPARC, MMP-3, MMP-7, MMP-9, MMP-11, MMP-13 and MMP-14 and/or
(iii) an agent capable of inhibiting the synthesis, by cells of the immune lineage and/or inflammatory, of markers selected from any of the following: TGF-β, NF-kB, EGF, MMP-9, VEGF and TNF-α,
or combinations thereof.

In a preferred embodiment, the composition of the invention is characterized in that the endothelial lineage cells are preferably vascular endothelial cells, the cells are preferably fibroblasts fibroblastic cells and lineage immune and/or inflammatory leukocytes are preferentially mononuclear, polymorphonuclear leukocytes and macrophages.

In another preferred embodiment, the composition of the invention is characterized in that the modifying agent is an antagonist peritumoral environment non-peptide NK1 receptors. In another preferred embodiment, antagonists, non-peptide receptor NK1 are selected from any of the following: Aprepitant, Vestipitant, Casopitant, Vofopitant, Ezlopitant, Lanepitant, LY-686017, L-733,060, L-732,138, L -703,606, WIN 62,577, CP-122721, TAK-637, and R673, CP-100263, WIN 51708, CP-96345, L-760735, CP-122721, L-758298, L-741671, L-742694, CP-99994, T-2328, being particularly preferred antagonists selected from: Aprepitant, Vestipitant, Casopitant, Vofopitant, Ezlopitant and Lanepitant.

In another preferred embodiment, the composition of the invention is characterized in that it is a pharmaceutical composition.

In another preferred embodiment, the composition of the invention is characterized by further comprising a pharmaceutically acceptable carrier.

In another preferred embodiment, the composition of the invention is characterized by further comprising at least one active ingredient that induces apoptosis in tumor cells, said active ingredient being selected from any of the following: Chlorambucil, Melphalan, Aldesleukin, 6-mercaptopurine, 5-fluorouracil, Ara-c, Bexarotene, Bleomycin, Capecitabine, Carboplatin, Cisplatin, Docetaxel, Doxorubicin, Epirubicin, Fludarabine, Irinotecan, Methotrexate, Mitoxantrone, Oxaliplatin, Paclitaxel, Rituximab, etoposide, teniposide, vincristine, vinblastine, vinorelbine, Imatinib, Erlotinib, Cetuximab, Trastuzumab.

In another preferred embodiment, the composition of the invention is characterized by being administered separately, together or sequentially, with another anticancer agent selected from any of the following: agent chemotherapy or radiotherapy agent.

Another object described in the present invention relates to a dosage form comprising a composition as previously defined throughout the present invention.

Another object described herein relates to use of the composition or the dosage form of the invention in the manufacture of a medicament, preferably for the treatment of cancer.

Another object referenced by the present invention is a combined preparation comprising:
(a) at least one modifying peritumoral ambient agent as defined along this invention
(b) at least one active substance which induces apoptosis in tumor cells.

In a preferred embodiment, the combined preparation of the invention is characterized in that the modifying agent is an antagonist peritumoral environment non-peptide NK1 receptors.

In another preferred embodiment, the combined preparation of the invention is characterized by non-peptide antagonists NK1 receptors being selected from any of the following: Aprepitant, Vestipitant, Casopitant, Vofopitant, Ezlopitant, Lanepitant, LY-686017, L- 733,060, L-732,138, L-703,606, WIN 62,577, CP-122721" TAK-637, and R673, CP-100263, WIN 51708, CP-96345, L-760 735, CP-122721, L-758 298 , L-741 671, L-742 694, CP-99994, T-2328.

In another preferred embodiment, the combined preparation of the invention is characterized by non-peptide antagonists NK1 receptors being selected from any of the following: Aprepitant, Vestipitant, Casopitant, Vofopitant, Ezlopitant and Lanepitant.

In another preferred embodiment, the combined preparation of the invention is characterized in that the active substance which induces apoptosis in tumor cells is selected from any of the following: Chlorambucil, Melphalan, Aldesleukin, 6-mercaptopurine, 5-fluorouracil, Ara-c, bexarotene, Bleomycin, Capecitabine, Carboplatin, Cisplatin, Docetaxel, Doxorubicin, Epirubicin, Fludarabine, Irinotecan Methotrexate, Mitoxantrone Oxaliplatin, Paclitaxel, Rituximab, vinblastine, etoposide, teniposide, vincristine, vinorelbine, Imatinib, Erlotinib, Cetuximab and Trastuzumab, or combinations thereof.

In another preferred embodiment, the combined preparation of the invention is characterized in that it is administered separately, together or sequentially with another anti-cancer agent selected from any of the following: chemotherapy or radiotherapy agent.

Another object described herein relates to use separate, simultaneous or sequential administration of the active ingredients of the combined preparation as defined herein, in the manufacture of a medicament, preferably for the treatment of cancer.

Another of the objects described in the present invention relates to a method of treatment directed to a patient suffering from cancer, by administering an effective amount or effective amount of at least the composition or dosage form of the combined preparation or peritumoral temperature modifying agent, described herein.

The term "combined preparation" or also called "juxtaposition" herein, means that the components of the combined preparation need not be present as a union, for example in a composition, to be available for use separately or sequentially. Thus, the term "juxtaposed" means that is not necessarily true combination, in view of the physical separation of the components.

In another preferred embodiment of the invention, the use of modifying peritumoral environment agent, itself modifying peritumoral environment agent, composition, dosage form, the combined preparation and method of treatment of the invention are characterized in that they are useful in treating various cancers, such as gastric cancer, preferably gastric adenocarcinoma, colon carcinoma, preferably colon adenocarcinoma, pancreas carcinoma, preferably pancreatic adenocarcinoma, breast carcinoma, preferably breast adenocarcinoma and/or carcinoma breast, ovarian carcinoma, preferably ovarian adenocarcinoma and/or ovarian carcinoma, endometrial carcinoma, choriocarcinoma, cervix carcinoma, lung carcinoma, preferably lung adenocarcinoma, lung carcinoma, non-small cell and/or lung carcinoma small cell carcinoma of the thyroid, preferably human papillary thyroid carcinoma metastasizing and/or follicular thyroid carcinoma, bladder carcinoma, preferably carcinoma of urinary bladder and/or transitional cell carcinoma of urinary bladder carcinoma, prostate carcinoma CNS glial, sarcoma, preferably fibrosarcoma, malignant fibrous histiocytoma, Edwing sarcoma, human endometrial stromal sarcoma, osteosarcoma and/or rhabdomyosarcoma, melanoma, embryonal carcinoma, preferably neuroblastoma, neuroblastoma bone marrow, and/or retinoblastoma and haematological cancers, more preferably leukemia cell T/NK, lymphoblastic B leukemia, lymphoblastic T leukemia, lymphoblastic leukemia B, Burkitt lymphoma, Hodgkin lymphoma, T lymphoma and/or multiple myeloma.

These examples are showed by way of illustration, not intended to be limiting of the present invention, where are apparent the advantages of the invention.

### Example 1. Treatment non-peptide NK1 receptors inhibits proliferation of human endothelial cell lines.

To demonstrate the modification of the microenvironment around the tumor by treatment with non-peptide antagonists of receptors MK1, firstly, an analysis was made of the presence of such NK1 receptors in the human cell line of vascular endothelial C-12210 (incorporated by microvascular endothelial cells from juvenile foreskin; PromoCell GmbH, SickingenstraBe 63/65, D-69126 Heidelberg, Germany), using the Western blot technique. Briefly, extraction of total proteins was performed from samples obtained from cell cultures. Cells were lysed by methods commonly known in the prior art and its concentration was measured using a commercial kit "Protein Assay" Bio-Rad (Bio-Rad Laboratories, SA Madrid), following the relevant manufacturer's instructions. 50 mg of protein were separated by gel electrophoresis in 10% SDS-polyacrylamide gels and electrophoretically transferred to polyvinylidene fluoride membranes (PVDF) from each sample. These membranes were incubated overnight in blocking solution (5% skimmed milk in phosphate-buffered with PBS-0, 1% Tween-PBST), followed by an overnight incubation with the primary antibodies diluted 1/4000 in PBST buffer. The anti-NK1 was used as primary antibody (S8305, Sigma-Aldrich), which recognizes the domain corresponding to the carboxyl terminal region of the NK1 receptor between amino acids 393-407. Next, the membranes were washed with phosphate buffered saline (PBS) in the presence of the detergent Tween-20 (PBST) and incubated with a secondary antibody conjugated to horseradish peroxidase for 2 hours at room temperature (dilution 1: 10000). Membranes were incubated with monoclonal anti-p-tubulin to confirm that was loaded in the same amount of protein. Detection of antibodies was performed with a chemiluminescence reaction (ECL Western blotting detection, Amersham Life Science, UK). The presence of NK1 receptors was also analyzed by immunohistochemistry. A sample of each of the cultures of the cell lines used in the present invention was centrifuged (5 minutes at 1500 rpm) and the pellet was dehydrated by treatment with increasing concentrations of ethanol and finally in xylene. Then, the samples were embedded in paraffin and dehydrated, creating a cell block. Such paraffin blocks were cut on a microtome to a thickness of 5 microns, which were placed on slides suitable for performing immunohistochemistry. Subsequently, samples were dewaxed by immersion in xylene and then were hydrated through a series of solutions containing decreasing concentrations of ethanol, to be finally immersed in water. Then, these samples were subjected to pressure cooker treatment at IOx citrate buffer at pH 6.0, to obtain an increased exposure of antigens. Subsequently, the samples were cooled at room temperature for 10 minutes. Endogenous peroxidase activity was blocked with hydrogen peroxide of 3% for 30 min at room temperature. After washing the samples with 0.05 M Tris buffer, incubated with serum from non-immune pigs 10% for 30 minutes at room temperature. Cell samples were incubated in the presence of anti-NK1 (S8305, Sigma-Aldrich) diluted 1: 1000, overnight at 4 °C to check the expression of NK1 receptors. After this time, samples were washed in 0.05M Tris buffer at room temperature. The next step was the addition of reagents-HRP Envision System (Dako) for 30 min at room temperature. This time expired, the samples were washed again in 0.05 M Tris buffer and immunoreactivity was visualized by light microscopy with a chromogenic solution with 3,3 '-diaminobenzidine (DAB+, Dako, USA). Samples were lightly stained with hematoxylin to differentiate the cell nuclei. As a negative control, samples which were not incubated with the primary antibody anti-NK1, were replaced by non-immune serum. All samples were evaluated.

The results obtained by both techniques revealed the existence of NK1 receptors in the cell line C-12210.

Then, to analyze the effect on the proliferation of cultured cells of the cell line C-12210, treatment was carried out for these crops with increasing concentrations of different non-peptide NK1. Cell proliferation was assessed by tetrazolium compound 3-(4, 5-dimethylthiazol-2-yl) -5-(3-carboxymethoxyphenyl) 2-(4-sulfophenyl)-2H-tetrazolium (MTS) in accordance with instructions provided by the manufacturer (Kit "CellTiter 96 Aqueous One-Solution Cell Proliferation Assay" Promega, USA). The cell number was quantified using a Coulter counter. C-12210 cells were grown in appropriate medium (PromoCell Growth Medium; Promocell GmbH, Germany) according to the manufacturer's instructions in the presence of increasing concentrations of various non-peptide antagonists of NK1 receptors. In tumor cell culture plates included a blank sample (no cells) and a control sample (containing 10⁴ cells/ml). To staining for the proliferation assays, 20 µl MTS was added to each the wells of cell culture plates 90 minutes prior to reading in a spectrophotometer samples multiscanner (TECAN Spectra Classic, Barcelona, Spain) at 492 nm (test wavelength) and 690 nm (reference wavelength). Different doses of each NK1 non-peptide were assayed in duplicate and each experiment was performed in sextuplicate.

Antagonists used for proliferation assays were: L-733,060 ((2S,3S)-3-[(3,5-bis (Trifluoromethyl) phenyl) methoxy]-2-phenylpiperidine hydrochloride), L-732,138 (N-Acetyl-L-tryptophan 3,5-bis (trifluoromethyl)benzyl ester), L-703,606 oxalate salt hydrate (cis-2- (Diphenylmethyl)-N-[(2-iodophenyl) methyl]-I-azabicyclo [2.2. 2] octan-3-amine oxalate salt), aprepitant (or MK 869 or L-754, 030), Vestipitant (or GW597599), Casopitant (or GW679769), CP-100263, WIN 62,577, WIN 51708, CP-96345 and L-760735. As mentioned along the present invention, other non-peptide receptor NK1 not described here may also be used.

Commercial line with reference C-12210 of microvascular endothelial cells were exposed to increasing concentrations of non-peptide receptor antagonists NK1, and inhibited growth was observed thereof, which proved to be time and dose dependent.

Table 1 shows the increased proliferation of the cells C-12210 grown in the presence of increasing concentrations of SP (NK1 receptor agonist). Said agonist has a stimulating effect on the growth of human endothelial cells. The results shown in Table 1 are expressed as percentage of control ± SD.

**Table 1. Analysis of cell culture proliferation of C-12210 in the presence of increasing concentrations of SP.**

| **SP Concentration** | **Exposure time (hours)** | | | |
|---|---|---|---|---|
| | **0** | **24** | **48** | **72** |
| Control (0 nM) | 100 | 100 | 100 | 100 |
| 10 nM | 102.2 ± 1 | 119.1 ± 1.8 | 139.3 ± 3.1 | 134.3 ± 2.8 |
| 100 nM | 109.5 ± 2.9 | 17 ± 2.7 | 21 ± 2.6 | 32.4 ± 3.3 |
| 500 nM | 128.9 ± 2.7 | 137 ± 3.8 | 146 ± 4.7 | 158.5 ± 4.6 |
| 1µM | 175.3 ± 5.2 | 186 ± 6.3 | 194.4 ± 6.5 | 199 ± 5.5 |
| 50µM | 218 ± 3.5 | 242 ± 6.7 | 247 ± 6.5 | 259.4 ± 4.1 |
| 100µM | 236.4 ± 5.6 | 258 ± 4.5 | 259.5 ± 4.6 | 266.9 ± 6.8 |

The capacity of NK1 receptor antagonists to inhibit cell proliferation as demonstrated by the use of the cell line C12210, is showed in Tables 2 to 4. Tables 2 to 4 present the data expressed as percentage of control ± SD, the receiver non-peptide NK1: Aprepitant, Vestipitant and Casopitant at concentrations indicated in the tables for 24, 48 and 72 hours. Different doses were tested in duplicate and each experiment was performed in sextuplicate. The results show that the inhibition of growth of said endothelial cells are time and dose dependent. Controlling proliferation as the cells cultured in the absence of antagonists above.

**Table 2. Analysis of cell culture proliferation of C-12210 in the presence of increasing concentrations of Aprepitant. The table shows the percentage inhibition ± SD of each treatment relative to the control.**

| **Aprepitant concentration** | **Exposure time (hours)** | | | |
|---|---|---|---|---|
| | **0** | **24** | **48** | **72** |
| Control (0 nM) | 100 | 100 | 100 | 100 |
| 10 nM | 10.1 ± 1.2 | 16.3 ± 1.2 | 21.4 ± 2.5 | 32.7 ± 2.6 |
| 100 nM | 18.1 ± 1.3 | 24.5 ± 2.5 | 35.6 ± 2.7 | 42.6 ± 3.4 |
| 500 nM | 24.2 ± 2.2 | 34.6 ± 1.6 | 41.6 ± 3.8 | 52.7 ± 2.5 |
| 1µM | 32.2 ± 2.4 | 45 ± 2.7 | 61.5 ± 3.6 | 73.6 ± 3.7 |
| 50µM | 45.3 ± 3.2 | 52.7 ± 4.6 | 69.4 ± 4.7 | 74.4 ± 4.6 |
| 100µM | 52 .4 ± 3.3 | 64.6 ± 4.7 | 84.7 ± 5.8 | 96.4 ± 5.7 |

**Table 3. Analysis of cell culture proliferation of C-12210 in the presence of increasing concentrations of Vestipitant. The table shows the percentage inhibition ± SD of each treatment relative to the control.**

| **Vestipitant Concentration** | **Exposure time (hours)** | | | |
|---|---|---|---|---|
| | **0** | **24** | **48** | **72** |
| Control (0 nM) | 100 | 100 | 100 | 100 |
| 10 nM | 12.2 ± 1.4 | 18.2 ± 2.3 | 25.4 ± 2.1 | 33.5 ± 3.8 |
| 100 nM | 15.4 ± 2.1 | 29.4 ± 2.6 | 36.6 ± 3.7 | 45.5 ± 4.4 |
| 500 nM | 19.5 ± 2.6 | 39 ± 3.3 | 46.7 ± 3.6 | 57.5 ± 4 |
| 1µM | 29.6 ± 2.6 | 45.5 ± 3.6 | 52.6 ± 4.2 | 63 ± 4.5 |
| 50µM | 34.7 ± 3.2 | 57.7 ± 4.6 | 61.6 ± 5.8 | 71.6 ± 5.3 |
| 100µM | 45.6 ± 3.8 | 68.7 ± 4.6 | 78.5 ± 5.6 | 93.4 ± 6.5 |

**Table 4. Analysis of cell culture proliferation of C-12210 in the presence of increasing concentrations of Casopitant. The table shows the percentage inhibition ± SD of each treatment relative to the control.**

| **Casopitant Concentration** | **Exposure time (hours)** | | | |
|---|---|---|---|---|
| | **0** | **24** | **48** | **72** |
| Control (0 nM) | 100 | 100 | 100 | 100 |
| 10 nM | 9.3 ± 1.1 | 14 ± 2.1 | 23.3 ± 3.2 | 31.4 ± 3.1 |
| 100 nM | 12.3 ± 2.1 | 18.5 ± 2.4 | 27.4 ± 4.5 | 36.4 ± 4.3 |
| 500 nM | 19.5 ± 2.3 | 24.6 ± 3.6 | 36.6 ± 4.9 | 45.5 ± 5 |
| 1µM | 22.4 ± 2.5 | 32.6 ± 4.8 | 42.3 ± 5.2 | 51.6 ± 5.6 |
| 50µM | 28.3 ± 3.5 | 37.5 ± 5.6 | 47.5 ± 6.8 | 58.6 ± 6.2 |
| 100µM | 36.3 ± 3.7 | 43.6 ± 5.7 | 52.4 ± 7.2 | 79.7 ± 8.7 |

Similar results to those shown in Tables 2 to 4 were obtained when used the other non-peptide NK1 receptor: L-733,060, L-732,138, L-703,606, CP-100263, WIN 62,577, WIN 51708, CP-96345 and L-760735.

Therefore, the results shown in this example show that the non-peptide antagonists of the NK1 receptor inhibit the proliferation of endothelial cells. The proliferation of these is a key element in the development of neovascularisation necessary for tumors to receive a supply of blood (and hence oxygen, nutrients those necessary) sufficient to enable it to maintain its growth and progression.

### Example 2. Peritumoral microenvironment modification, specifically fibroblastic cells, by treatment with non-peptide antagonist NK1 receptor.

This example shows the effect of treatment with non-peptide antagonist NK1 receptors in peritumoral microenvironment modification, specifically in human primary fibroblasts (PHF). These PHF were obtained and purified from samples obtained from the dermis of the skin of healthy volunteers as described in De Bari et al (De Bari et al. 2001). Briefly, small pieces of skin dermis were digested in a solution of hyaluronidase (Sigma-Aldrich, USA) at a concentration of 1 mg/ml for 15 minutes at 37 °C and then treated with 6 mg/ml collagenase type IV (Invitrogen) for 2 hours at 37 °C. Then, the cells were washed, resuspended in DMEM culture medium with high glucose (Dulbecco's Modified Eagle's Medium, Invitrogen) supplemented with 1% antibiotic-antimycotic (Invitrogen) and 1% pyruvate Sodium (Invitrogen), and seeded in culture dishes at a concentration of 10,000 cells per square centimeter. When the cells reached confluence, adherent cells were detached using 0.5% sterile trypsin (Invitrogen) and used between passages 3 and 9. For tests shown below, the PHF were plated in 24-well plates at a concentration of 25,000 cells per well.

To verify that the NK1 receptor agonists, such as SP, induce changes in the stromal cells, preferably fibroblasts, similar to those observed in these same cells in the peritumoral area and that these changes are reversed by non-peptide NK1 receptors have been analyzed for the presence of different molecular markers (TGFa, TGF, SPARC and MMPs), associated with tumor progression in cells that form the tumor microenvironment, specifically in cell cultures of primary human fibroblasts (PFH) in the presence of the receptor agonist SP NK1 and in the presence of agonist together with such non-peptide NK1 receptors. To this, were cultured in the presence of PHF SP I µM a concentration (positive control) (S6883, Sigma-Aldrich) and with various non-peptidic antagonists of the receptors at a concentration of NK1 1 µM specifically shows the results obtained with the antagonist Aprepitant, Vestipitant and Casopitant. After 48 hours of culture, cells were harvested and paraffin cell-blocks for immunohistochemical analysis were constructed, as previously explained in Example 1.

In immunohistochemical assays the expression of the following markers were studied: TGFα (SAB4502953), TGF1 (SAB4502954), TGFβ2 (SAB4502956), TGFβ3 (SAB4502957), SPARC (HPA002989), MMP-3 (HPA007875) MMP-7 (SAB4501894), MMP-9 (SAB4501896), MMP-11 (SAB4501898), MMP-13 (SAB4501900) and MMP-14 (SAB4501901) using specific antibodies against them. All antibodies used were rabbit polyclonal antibodies obtained from Sigma-Aldrich and were used at a concentration of 1/1000. All experiments were carried out sixfold.

On each of the six sections cell counts were performed in 20 high power fields (400x) through an Olympus microscope (model CX31) to assess the immunostaining. On each of the fields total cell number and number of cells displaying immunostaining were counted, being then the percentage of cells displaying said immunostaining.

The results obtained and shown in Table 5 firstly show the presence of all markers analyzed in samples from PHF cell cultures treated only with SP, indicating that the receptor agonist NK1 induces, in human primary fibroblasts, immunophenotypic changes similar to those which occur in the peritumoral area. In contrast, no immunostaining was observed, no signs of expression, in the cell cultures treated jointly with the SP and every non-peptide NK1 receptor (Table 5). This shows that the use of nonpeptide antagonists NK1 receptor is capable of reversing the expression of markers associated with tumor development and progression. In this sense, the use of non-peptide NK1 receptors prevent the survival, development and progression of these tumors.

**Table 5. Analysis of the expression of different markers associated with tumor development and progression in PHF cultured in the presence of nonpeptide antagonists of NK1. The table shows the percentage ± SD of the number of cells which expressed each marker compared to total cells for each sample.**

| **Marker** | **SP** | **SP + Aprepitant** | **SP + Vestipitant** | **SP + Casopitant** |
|---|---|---|---|---|
| TGFα | 16.5±1.2% | 0% | 0% | 0% |
| TGFβ 1 | 18.4±1.3% | 0% | 0% | 0% |
| TGFβ 2 | 24.6±1.4% | 0% | 0% | 0% |
| TGFβ 3 | 23.1±1.7% | 0% | 0% | 0% |
| SPARC | 16±1.9% | 0% | 0% | 0% |
| MMP-3 | 33.9±2.2% | 0% | 0% | 0% |
| MMP-7 | 46.2±2.3% | 0% | 0% | 0% |
| MMP-9 | 35.5±2.5% | 0% | 0% | 0% |
| MMP-11 | 7.8±2.9% | 0% | 0% | 0% |
| MMP-13 | 17.1±2.6% | 0% | 0% | 0% |
| MMP-14 | 47.3±2.5% | 0% | 0% | 0% |

Similar results to those shown in Table 5 were obtained when it were used other non-peptide NK1 receptor: L-733, 060, L-732, 138, L-703, 606, CP-100263, WIN 62,577, WIN 51708, CP-96345 and L-760735.

### Example 3. Modification of peritumoral microenvironment, specifically of the cells involved in inflammatory/immune processes, by the treatment with non-peptide NK1 receptor.

Other cells that make up the microenvironment around the tumor are the cells involved in inflammatory and /or immune processes. In this sense, the next step was to analyze the effect exerted by the treatment with non-peptide NK1 antagonists in these cells, specifically polymorphonuclear and mononuclear cells of the blood. Briefly, heparinized blood from healthy donors was centrifuged so as to separate the various components thereof. Red blood cells remained at the bottom of the tube. Above them was a small white layer composed of the white blood cells and above, on top of the tube, the blood plasma. The layer of white cells was distributed in a culture plate of 24 wells by adding in each the same concentration of blood plasma from the same donor, supplemented with 1% antibiotic-antimycotic (Invitrogen). In each well, approximately 1000 cells (PHF) skin from the dermis were further added, obtained as previously described in Example 2. Then, the SP was added to these cultures in a concentration of 1 µM or SP at the same concentration together with non-peptide NK1 receptors, Aprepitant, Vestipitant and Casopitant, all at a concentration of 1 µM. After 48 hours of incubation, cells were collected and embedded in paraffin for expression analysis by immunohistochemical techniques (as described in Example 1).

The expression of TGF molecular markers (anti-TGF 2, SAB4502956) and NF-kB (anti-NF-kB, SAB4501992) using specific antibodies against them were analyzed. All antibodies used were rabbit polyclonal antibodies obtained from Sigma Aldrich and were used at a concentration of 1/1000. All experiments were carried out sixfold.

The results shown in Tables 6 and 7 demonstrate the presence of TGF-β and NF-kB in mononuclear cells (Table 6) and polymorphonuclear cells (Table 7) of the samples from cultures treated only with the SP. In contrast, the treatment with SP in conjunction with non-peptide NK1 receptors, no expression of molecular markers analyzed (no staining) samples in cultures of mononuclear cells and polymorphonuclear leukocytes. These results demonstrate that treatment with a NK1 agonist receptor, the SP, induces the expression of molecular markers TGF-β and NF-kB, which are mediators of tumor development and progression. In contrast, the use of non-peptidic antagonists of the NK1 receptors is able to reverse the expression of these markers, showing the ability of such antagonists to inhibit the genesis of these mediators and, therefore, prevent the development of tumor microenvironment allowing survival, increased size and progression of tumors. In this sense, the use of non-peptide NK1 receptors inhibits the survival, development and progression of tumors.

**Table 6. Analysis of the expression of different molecular markers associated with tumor development and progression in mononuclear cells in the presence of non-peptide antagonists of NK1 receptors. The table shows the percentage ± SD of the number of cells which expressed each marker compared to total cells for each sample.**

| **Marker** | **SP** | **SP + Aprepitant** | **SP + Vestipitant** | **SP + Casopitant** |
|---|---|---|---|---|
| TGF-β | 16.4 ±1.2% | 0% | 0% | 0% |
| NF-kB | 25.7 ±1.4% | 0% | 0% | 0% |

**Table 7. Analysis of the expression of different markers associated with tumor development and progression polymorphonuclear cells in the presence of non-peptide antagonists of NK1 receptors. The table shows the percentage ± SD of the number of cells which expressed each marker compared to total cells for each sample.**

| **Marker** | **SP** | **SP + Aprepitant** | **SP + Vestipitant** | **SP + Casopitant** |
|---|---|---|---|---|
| TGF-β | 36.8 ±2.1% | 0% | 0% | 0% |
| N F-kB | 17.5 ±1.6% | 0% | 0% | 0% |

Similar results to those shown in Tables 6 and 7 were obtained when used other non-peptide NK1 receptor: L-733,060, L-732,138, L-703,606, CP-100263, WIN 62,577, WIN 51708, CP- 96345 and L-760735.

### Example 4. Modification of peritumoral microenvironment, specifically modifying the macrophage lineage cells, by the treatment with non-peptide antagonists of NK1 receptors.

Further cells that make up the microenvironment are peritumoral macrophage lineage cells that are of great importance in the development of the microenvironment around the tumor, by interacting with tumor cells to stimulate their growth and spread by secretion into the medium of different markers that promote the expansion and growth of tumors. In this sense the next step was to analyze the effect exerted by the treatment with non-peptide NK1 antagonists in human primary macrophages (PHM) obtained and purified from pleural effusion samples obtained from men with chronic age range between 45 and 55 years. Briefly, the pleural fluid from chronic (rich in macrophages) was centrifuged. Macrophages were seeded in culture dishes at a concentration of approximately 1000 cells per square centimetre, using as the culture medium itself pleural fluid (to obtain an experimental model similar to human), supplemented with 1% antibiotic-antimycotic (Invitrogen) for exposure to NK1 receptor agonist (SP) and exposure to NK1 receptor antagonists.

To verify that the agonists of NK1 receptors, such as SP, induced immunophenotypic changes in macrophages, similar to those observed in these same cells in the peritumoral area and that these changes are reversed by treatment with non-peptide antagonists of the receptors NK1, was analyzed for the presence or expression by immunohistochemistry (see Example 1) markers, EGF, MMP-9, VEGF and IL-8, using antibodies specific thereto, in cultures of PHM in the presence of SP (1µM) (S6883, Sigma-Aldrich) and in the presence of SP and each of the NK1 receptor antagonist, Aprepitant, Vestipitant and Casopitant, all at a concentration of 1 µM. After 48 hours of culture, cells were harvested and cellular paraffin blocks were constructed for immunohistochemical analysis (see Example 1). To analyze the expression of markers EGF, MMP-9, VEGF and IL-8 antibodies were used as follows: EGF (rabbit monoclonal antibody, anti-EGF; 07-1432. Merck-Millipore), MMP-9 (polyclonal rabbit anti-MMP-9: SAB4501896, Sigma-Aldrich), VEGF (mouse monoclonal anti-VEGF, GF25-100UG, Merck-Millipore) and TNF-α (mouse monoclonal anti-TNF-α, number MAB 1021 catalogue, obtained from Merck-Millipore). All of these antibodies were used at a concentration of 1/1000. System for labelling with a secondary antibody specific for the primary antibodies obtained from rabbit or mouse was used, as was necessary in each case, both the Envision (Dako). All experiments were carried out sixfold.

Table 8 shows the results, revealing that the PHM express all markers analyzed in the presence of SP (NK1 receptor agonist), which demonstrates that said agonist is capable of inducing in similar immunophenotypic changes PHM to those found in peritumoral areas, typical of so-called "cancer-associated macrophages" (Hagemann T et al, 2009; Condeelis J et al. 2006). In contrast, no immunostaining was observed, no signs of expression for cell cultures treated jointly with the SP and each of the non-peptide antagonists of NK1 receptors. In this sense, the use of non-peptide NK1 prevent the development and progression of tumors via inhibition of secretion by HPM substances promoting the growth and progression of these, and inducing, thereby, reducing the size of such tumors and reducing their invasiveness.

**Table 8. Analysis of the expression of different molecular markers associated with tumor development and progression in macrophages cultured in the presence of SP alone or with non-peptide antagonists of NK1 receptors. The table shows the percentage ± SD of the number of cells expressing each marker compared to total cells for each sample.**

| **Marker** | **SP** | **SP + Aprepitant** | **SP + Vestipitant** | **SP + Casopitant** |
|---|---|---|---|---|
| EGF | 27.4±1.1% | 0% | 0% | 0% |
| MMP-9 | 24.3±1.4% | 0% | 0% | 0% |
| VEGF | 29.6±2.1% | 0% | 0% | 0% |

Similar results were obtained in the case of TNF-α, respect to its expression to those obtained using the other markers analyzed: EGF, MMP-9 and VEGF, i.e., the treatment with the non-peptide receptor NK1 inhibited the TNF-α marker expression. Also the presence of IL-8 were determined by Western blotting, as explained in Example 1, using anti-leucine-8 primary antibody (AB1427, Merck-Millipore). The results showed that HPM cultured in the presence of SP showed expression of this marker, however, when non-peptide antagonists NK1 receptors were added, such expression disappeared completely, as was the case with the results shown above. Similar results to those shown in Table 8 were obtained when we used other non-peptide receptor NK1: L-733,060, L-732,138, L-703,606, CP-100263, WIN 62,577, WIN 51708, CP-96345 and L-760735.

### Example 5. Non-peptide antagonists of NK1 receptor inhibit secretion by fibroblasts, of substances that promote the progression of cancer.

The interaction between tumor cells and stromal cells, preferably fibroblasts, promotes secretion from fibroblasts of different molecules, which in turn stimulate the proliferation and survival of tumor cells.

To check that the interaction of tumor cells with stromal cells - fibroblasts-induced changes similar to those observed in these same cells in the peritumoral area, these changes are exacerbated by exposure to receptor agonist and which are inhibited NK1 in the presence of non-peptide antagonists of this receptor, underwent various kinds of tumor cells from tumor lines commercial co-cultures with fibroblasts (obtained as described in Example 2), to co-cultures in the presence of NK1 receptor agonists (SP) and to co-cultures in the presence of such agonist and various non-peptide receptor NK1 antagonists. The different tumor cell lines used, specifying in each case the type of tumor that corresponds, the company that supplies catalogue and code are shown in Table 9.

**Table 9. Tumor cell lines used in the present invention**

| | **Cellular line** | **Company** |
|---|---|---|
| Human gastric carcinoma | | |
| Human gastric adenocarcinoma | 23132/87 | DSMZ |
| Human colon carcinoma | | |
| Human colon adenocarcinoma | SW-403 | DSMZ |
| Human pancreatic carcinoma | | |
| Human pancreatic adenocarcinoma | CAPAN-1 | DSMZ |
| Human pancreatic adenocarcinoma | PA-TU 8902 | DSMZ |
| Human breast carcinoma | | |
| Human breast adenocarcinoma | MCF-7 | DSMZ |
| Human breast carcinoma | MT-3 | DSMZ |
| Human breast carcinoma | MDA-MB-468 | DSMZ |
| Ovarian carcinoma | | |
| Human ovarian adenocarcinoma | EFO-27 | DSMZ |
| Human ovarian carcinoma | COLO-704 | DSMZ |
| Human endometrial carcinoma | | |
| Human endometrial carcinoma | AN3-CA | DSMZ |
| Human choriocarcinoma | | |
| Human choriocarcinoma | AC-1 M32 | DSMZ |
| Human uterine cervix carcinoma | | |
| Human uterine cervix carcinoma | KB | DSMZ |
| Human lung carcinoma | | |
| Human lung adenocarcinoma | DV-90 | DSMZ |
| Squamous non-small cell lung human | HCC-44 | DSMZ |
| Squamous non-small cell lung human | COR-L23 | ECACC |
| Carcinoma of human small cell lung | H-209 | DSMZ |
| Carcinoma of human small cell lung | H-1963 | DSMZ |
| Human lung carcinoma | A-427 | DSMZ |
| Human thyroid carcinoma | | |
| Human thyroid carcinoma | 8505C | DSMZ |
| Human papillary thyroid carcinoma metastasizing | B-CPAP | DSMZ |
| Human folicular thyroid carcinoma | TT2609-C02 | DSMZ |
| Carcinoma of human urinary bladder | | |
| Carcinoma of human urinary bladder | 5637 | DSMZ |
| Transitional cell carcinoma of human urinary bladder | RT-4 | DSMZ |
| Human prostate carcinoma | | |
| Human prostate carcinoma | 22RV1 | DSMZ |
| Glial tumors of the Central Nervous System | | |
| Human glioma | GAMG | DSMZ |
| Human Sarcomas | | |
| Human fibrosarcoma | HT-1080 | DSMZ |
| Human malignant fibrous histiocytoma | U-2197 | DSMZ |
| Sarcoma human Edwing | MHH-ES-1 | DSMZ |
| Human endometrial stromal sarcoma | ESS-1 | DSMZ |
| Human osteoarcoma | CAL-72 | DSMZ |
| Human Rhabdomyosarcoma | A-204 | DSMZ |
| Human melanoma | | |
| Human melanoma | MEL HO | DSMZ |
| Human melanoma lymph node metastasis | COLO 858 | ICLC |
| Embryonal tumors | | |
| Human Neuroblastoma | KELLY | DSMZ |
| Human neuroblastoma bone marrow | SKN-BE 2 | ICLC |
| Human retinoblastoma | WERI-Rb-1 | DSMZ |
| Hematologic cancer | | |
| Human T-cell leukemia/NK | YT | DSMZ |
| B lymphoblastic leukemia | SD1 | DSMZ |
| Human T-lymphoblastic leukemia | BE-13 | DSMZ |
| Human B lymphoma | BC-1 | DSMZ |
| Human Burkitt lymphoma | CA-46 | DSMZ |
| Human Hodgkin lymphoma | KM-H2 | DSMZ |
| Human T-cell lymphoma | DERL-7 | DSMZ |
| Human Multiple Myeloma | COLO-677 | DSMZ |

Before conducting the experiments, it was found, by Western blot, that all commercial tumor cell lines expressing the NK1 receptor.

The cultures were performed on fresh blood plasma of an healthy donor, obtained by extraction of blood and the same light centrifugation, supplemented with 1% antibiotic-antimycotic (Invitrogen) to obtain an experimental model similar to human physiology. All cultures were maintained 48 hours, following which the same cells were included in paraffin blocks for subsequent analysis of the expression of different markers by immunohistochemistry (see Example 1). The primary antibodies used to perform immunohistochemical assays and their concentration are detailed in Example 2. All experiments were carried out sixfold. Analyzed markers in fibroblast cells (stromal cells) were: TGF-α, TGF-β 1, TGF-β 2, TGF-β 3, SPARC, MMP-3, MMP-7, MMP-9, MMP-11, MMP-13 and MMP-14.

By way of example, in Tables 10 to 29, show the results of the analysis of expression of marker aforementioned in PHF co-cultured with different tumor cell lines (Table 9). In these tables can be seen as the co-culture of stromal cells (fibroblasts PHF) with different tumor cells (and therefore the interaction thereof) increases the percentage of fibroblasts with marker expression analyzed for the observed expression of the same in cultures of these cells alone. This expression, in turn, is increased by exposure to NK1 receptor agonist (SP) (1µM) and is inhibited by exposure to NK1 receptor antagonists, Aprepitant, Vestipitant and Casopitant to 1 µM concentration for each. These tables show the average percentage ± SD presenting cells with immunostaining (or secrete) for each marker in the presence of SP alone or in combination with different non-peptide antagonists of NK1 receptors. Similar results to those shown in Tables 10 to 29 were obtained when we used other non-peptide NK1 receptor: L-733,060, L-732,138, L-703,606, CP-100263, WIN 62,577, WIN 51708, CP-96345 and L-760735.

**Table 10. Analysis of the expression of different molecular markers associated with tumor development and progression in fibroblasts (PHF) co-cultured with tumor cells of human gastric adenocarcinoma (reference 23132/87) (co-culture) in the presence of SP alone or together with nonpeptide NK1. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 32.4±1.1% | 0 | 0 | 0 |
| TGF-β 1 | 0 | 13.4±1.1% | 33.5±1.1% | 0 | 0 | 0 |
| TGF-β 2 | 0 | 20.5±1.2% | 40.5±1.1% | 0 | 0 | 0 |
| TGF-β 3 | 0 | 13.1±1.6% | 41±1.56% | 0 | 0 | 0 |
| SPARC | 0 | 17±1.8% | 47±1.2% | 0 | 0 | 0 |
| MMP-3 | 0 | 23.4±1.2% | 43.4±1.7% | 0 | 0 | 0 |
| MMP-7 | 0 | 46.2±2.3% | 56.1±2.8% | 0 | 0 | 0 |
| MMP-9 | 0 | 33.3±1.5% | 52.3±1.4% | 0 | 0 | 0 |
| MMP-11 | 0 | 7.6±2.4% | 27.4±2.8% | 0 | 0 | 0 |
| MMP-13 | 0 | 16±2.4% | 45±3.5% | 0 | 0 | 0 |
| MMP-14 | 0 | 29.3±2.2% | 49.2±4.5% | 0 | 0 | 0 |

**Table 11. Analysis of the expression of different molecular markers associated with tumor development and progression in fibroblasts (PHF) co-cultured with tumor cells of human colon adenocarcinoma (SW-403 reference) (co-culture) in the presence of SP alone or together with nonpeptide NK1 receptors. The table shows the percentage ± SD of the number of cells expressing each marker compared to total cells for each sample.**

| **Marker** | **PH F** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.5±1% | 0 | 0 | 0 |
| TGF-β 1 | 0 | 13.4±1.1% | 34.3±1.2% | 0 | 0 | 0 |
| TGF-β 2 | 0 | 20.5±1.2% | 42.5±1.5% | 0 | 0 | 0 |
| TGF-β 3 | 0 | 13.1±1.6% | 44±1.9% | 0 | 0 | 0 |
| SPARC | 0 | 17±1.8% | 48±1.6% | 0 | 0 | 0 |
| MMP-3 | 0 | 23.4±1.2% | 46.4±1.9% | 0 | 0 | 0 |
| MMP-7 | 0 | 46.2±2.3% | 53.1±2.2% | 0 | 0 | 0 |
| MMP-9 | 0 | 33.3±1.5% | 55.7±1.6% | 0 | 0 | 0 |
| MMP-11 | 0 | 7.6±2.4% | 26.4±2.6% | 0 | 0 | 0 |
| MMP-13 | 0 | 16±2.4% | 46.5±3.7% | 0 | 0 | 0 |
| MMP-14 | 0 | 29.3±2.2% | 51.3±3.6% | 0 | 0 | 0 |

**Table 12. Analysis of the expression of different molecular markers associated with tumor development and progression in fibroblasts (PHF) co-cultured with tumor cells of human pancreatic adenocarcinoma (reference CAPAN-1) (co-culture) in the presence of SP alone or together non-peptide antagonist of NK1 receptors. Table shows the percentage ± SD of the number of cells expressing each marker compared to all cells of each sample.**

| **Marker** | **PH F** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±2.1% | 0 | 0 | 0 |
| TGF-β 1 | 0 | 13.4±1.1% | 37.6±1.4% | 0 | 0 | 0 |
| TGF-β 2 | 0 | 20.5±1.2% | 42.4±6.1% | 0 | 0 | 0 |
| TGF-β 3 | 0 | 13.1±1.6% | 43±2.6% | 0 | 0 | 0 |
| SPARC | 0 | 17±1.8% | 49±1.4% | 0 | 0 | 0 |
| MMP-3 | 0 | 23.4±1.2% | 44.4±1.6% | 0 | 0 | 0 |
| MMP-7 | 0 | 46.2±2.3% | 58.2±2.4% | 0 | 0 | 0 |
| MMP-9 | 0 | 33.3±1.5% | 56.4±1.5% | 0 | 0 | 0 |
| MMP-11 | 0 | 7.6±2.4% | 25.3±1.5% | 0 | 0 | 0 |
| MMP-13 | 0 | 16±2.4% | 55±3.6% | 0 | 0 | 0 |
| MMP-14 | 0 | 29.3±2.2% | 47.2±4.7% | 0 | 0 | 0 |

Similar results to those shown in Table 12 were obtained when cells were co-cultured with PHF tumor cell line of human pancreatic adenocarcinoma (PA-TU-8902).

**Table 13. Analysis of the expression of different molecular markers associated with tumor development and progression in fibroblasts (PHF) co-cultured with tumor cells of human breast carcinoma (MCF-7 reference) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.6±1.1% | 0 | 0 | 0 |
| TGF-β 1 | 0 | 13.4±1.1% | 43.2±1% | 0 | 0 | 0 |
| TGF-β 2 | 0 | 20.5±1.2% | 42.6±2.1% | 0 | 0 | 0 |
| TGF-β 3 | 0 | 13.1±1.6% | 46±1.7% | 0 | 0 | 0 |
| SPARC | 0 | 17±1.8% | 49±1.5% | 0 | 0 | 0 |
| MMP-3 | 0 | 23.4±1.2% | 44.5±1.8% | 0 | 0 | 0 |
| MMP-7 | 0 | 46.2±2.3% | 57.1±2.7% | 0 | 0 | 0 |
| MMP-9 | 0 | 33.3±1.5% | 55.3±1.7% | 0 | 0 | 0 |
| MMP-11 | 0 | 7.6±2.4% | 25.4±2.7% | 0 | 0 | 0 |
| MMP-13 | 0 | 16±2.4% | 46±3.6% | 0 | 0 | 0 |
| MMP-14 | 0 | 29.3±2.2% | 48.2±3.5% | 0 | 0 | 0 |

Similar results to those shown in Table 13 were obtained when co-cultured cells PHF with tumor cell lines of breast carcinoma human MT-3 or MDA-MB-468.

**Table 14. Analysis of the expression of different molecular markers associated with tumor development and progression in fibroblasts (PHF) co-cultured with tumor cells, ovarian carcinoma (reference EFO-27) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 37.2±2.1% | 0 | 0 | 0 |
| TGF-β 1 | 0 | 13.4±1.1% | 35.5±2.1% | 0 | 0 | 0 |
| TGF-β 2 | 0 | 20.5±1.2% | 45.4±1.2% | 0 | 0 | 0 |
| TGF-β 3 | 0 | 13.1±1.6% | 42±1.6% | 0 | 0 | 0 |
| SPARC | 0 | 17±1.8% | 46±2.2% | 0 | 0 | 0 |
| MMP-3 | 0 | 23.4±1.2% | 44.4±1.5% | 0 | 0 | 0 |
| MMP-7 | 0 | 46.2±2.3% | 55.1±2.6% | 0 | 0 | 0 |
| MMP-9 | 0 | 33.3±1.5% | 56.6±1.7% | 0 | 0 | 0 |
| MMP-11 | 0 | 7.6±2.4% | 22.4±2.6% | 0 | 0 | 0 |
| MMP-13 | 0 | 16±2.4% | 55±2.5% | 0 | 0 | 0 |
| MMP-14 | 0 | 29.3±2.2% | 46.2±2.5% | 0 | 0 | 0 |

Similar results to those shown in Table 14 were obtained when co-cultured with cells PHF tumor cell line of human ovarian carcinoma COLO-704.

**Table 15. Analysis of the expression of different molecular markers associated with tumor development and progression in fibroblasts (PHF) co-cultured with tumor cells of human endometrial carcinoma (reference AN3-CA) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±2% | 0 | 0 | 0 |
| TGF-β 1 | 0 | 13.4±1.1% | 33.3±3.1% | 0 | 0 | 0 |
| TGF-β 2 | 0 | 20.5±1.2% | 43.5±1.1% | 0 | 0 | 0 |
| TGF-β 3 | 0 | 13.1±1.6% | 44±1.56% | 0 | 0 | 0 |
| SPARC | 0 | 17±1.8% | 49±1.5% | 0 | 0 | 0 |
| MMP-3 | 0 | 23.4±1.2% | 44.2±2.7% | 0 | 0 | 0 |
| MMP-7 | 0 | 46.2±2.3% | 54.4±2.1% | 0 | 0 | 0 |
| MMP-9 | 0 | 33.3±1.5% | 54.3±1.6% | 0 | 0 | 0 |
| MMP-11 | 0 | 7.6±2.4% | 29.4±1.8% | 0 | 0 | 0 |
| MMP-13 | 0 | 16±2.4% | 49±3.4% | 0 | 0 | 0 |
| MMP-14 | 0 | 29.3±2.2% | 45.2±3.5% | 0 | 0 | 0 |

**Table 16. Analysis of the expression of different molecular markers associated with tumor development and progression in fibroblasts (PHF) co-cultured with human choriocarcinoma tumor cells (reference AC-1 M32) (co-culture) in the presence of SP antagonists alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 41.4±1.1% | 0 | 0 | 0 |
| TGF-β 1 | 0 | 13.4±1.1% | 36.5±2.1% | 0 | 0 | 0 |
| TGF-β 2 | 0 | 20.5±1.2% | 53.5±3.1% | 0 | 0 | 0 |
| TGF-β 3 | 0 | 13.1±1.6% | 46±3% | 0 | 0 | 0 |
| SPARC | 0 | 17±1.8% | 46±3.1% | 0 | 0 | 0 |
| MMP-3 | 0 | 23.4±1.2% | 45.4±2.7% | 0 | 0 | 0 |
| MMP-7 | 0 | 46.2±2.3% | 57.1±3.8% | 0 | 0 | 0 |
| MMP-9 | 0 | 33.3±1.5% | 56.3±2.4% | 0 | 0 | 0 |
| MMP-11 | 0 | 7.6±2.4% | 28.4±2.8% | 0 | 0 | 0 |
| MMP-13 | 0 | 16±2.4% | 47±3.7% | 0 | 0 | 0 |
| MMP-14 | 0 | 29.3±2.2% | 51.2±4.5% | 0 | 0 | 0 |

**Table 17. Analysis of the expression of different molecular markers associated with tumor development and progression in fibroblasts (PHF) co-cultured with tumor cells of human cervix carcinoma (reference KB) (co-culture) in the presence of SP alone or together with nonpeptide NK1 receptors. The table shows the percentage ± SD of the number of cells expressing each marker compared to total cells for each sample.**

| **Marker** | **PH F** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 33.4±1.1% | 0 | 0 | 0 |
| TGF-β 1 | 0 | 13.4±1.1% | 34.5±1.1% | 0 | 0 | 0 |
| TGF-β 2 | 0 | 20.5±1.2% | 45.6±1.1% | 0 | 0 | 0 |
| TGF-β 3 | 0 | 13.1±1.6% | 44.5±3.1% | 0 | 0 | 0 |
| SPARC | 0 | 17±1.8% | 46.9±1.6% | 0 | 0 | 0 |
| MMP-3 | 0 | 23.4±1.2% | 42.6±1.8% | 0 | 0 | 0 |
| MMP-7 | 0 | 46.2±2.3% | 57.5±5.8% | 0 | 0 | 0 |
| MMP-9 | 0 | 33.3±1.5% | 57.3±6.4% | 0 | 0 | 0 |
| MMP-11 | 0 | 7.6±2.4% | 36.4±6.8% | 0 | 0 | 0 |
| MMP-13 | 0 | 16±2.4% | 45.4±3.7% | 0 | 0 | 0 |
| MMP-14 | 0 | 29.3±2.2% | 51.6±3.5% | 0 | 0 | 0 |

**Table 18. Analysis of the expression of different molecular markers associated with tumor development and progression in fibroblasts (PHF) co-cultured with tumor cells of lung carcinoma (A-427 reference) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.3±2.1% | 0 | 0 | 0 |
| TGF-β 1 | 0 | 13.4±1.1% | 33.4±2.1% | 0 | 0 | 0 |
| TGF-β 2 | 0 | 20.5±1.2% | 40.9±2.1% | 0 | 0 | 0 |
| TGF-β 3 | 0 | 13.1±1.6% | 51.4±1.6% | 0 | 0 | 0 |
| SPARC | 0 | 17±1.8% | 47±1.2% | 0 | 0 | 0 |
| MMP-3 | 0 | 23.4±1.2% | 45.5±1.6% | 0 | 0 | 0 |
| MMP-7 | 0 | 46.2±2.3% | 54.3±2.2% | 0 | 0 | 0 |
| MMP-9 | 0 | 33.3±1.5% | 55.5±2.4% | 0 | 0 | 0 |
| MMP-11 | 0 | 7.6±2.4% | 25.5±1.8% | 0 | 0 | 0 |
| MMP-13 | 0 | 16±2.4% | 44.3±2.5% | 0 | 0 | 0 |
| MMP-14 | 0 | 29.3±2.2% | 45.1±3.5% | 0 | 0 | 0 |

Similar results to those shown in Table 18 were obtained when co-cultured cells PHF with tumor cell lines of human lung adenocarcinoma (DV-90), lung carcinoma, non-small cell human (HCC44 and COR-L23) and carcinoma of human small cell lung (H-209 and H-1963).

**Table 19. Analysis of the expression of different molecular markers associated with tumor development and progression in fibroblasts (PHF) co-cultured with tumor cells, thyroid carcinoma (reference A-427) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±1.2% | 0 | 0 | 0 |
| TGF-β1 | 0 | 13.4±1.1% | 33.7±1.3% | 0 | 0 | 0 |
| TGF-β2 | 0 | 20.5±1.2% | 42.6±1.1% | 0 | 0 | 0 |
| TGF-β3 | 0 | 13.1±1.6% | 42.1±1.5% | 0 | 0 | 0 |
| SPARC | 0 | 17±1.8% | 48.1±1.3% | 0 | 0 | 0 |
| MMP-3 | 0 | 23.4±1.2% | 43.1±1.7% | 0 | 0 | 0 |
| MMP-7 | 0 | 46.2±2.3% | 56.4±3.8% | 0 | 0 | 0 |
| MMP-9 | 0 | 33.3±1.5% | 51.4±3.4% | 0 | 0 | 0 |
| MMP-11 | 0 | 7.6±2.4% | 29.4±3.8% | 0 | 0 | 0 |
| MMP-13 | 0 | 16±2.4% | 4.25±3.6% | 0 | 0 | 0 |
| MMP-14 | 0 | 29.3±2.2% | 49.1±3.6% | 0 | 0 | 0 |

Similar results to those shown in Table 19 were obtained when co-cultured cells PHF with tumor cell lines of mestastasis human papillary thyroid carcinoma (B-CPAP) or human follicular thyroid carcinoma (TT2609-C02).

**Table 20. Analysis of the expression of different molecular markers associated with tumor development and progression in fibroblasts (PHF) co-cultivated with tumor cell carcinoma of urinary bladder (reference 5637) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±1.1% | 0 | 0 | 0 |
| TGF-β 1 | 0 | 13.4±1.1% | 43.6±1.1% | 0 | 0 | 0 |
| TGF-β 2 | 0 | 20.5±1.2% | 44.3±1.1% | 0 | 0 | 0 |
| TGF-β 3 | 0 | 13.1±1.6% | 42.3±1.2% | 0 | 0 | 0 |
| SPARC | 0 | 17±1.8% | 47.4±2.2% | 0 | 0 | 0 |
| MMP-3 | 0 | 23.4±1.2% | 44.5±2.7% | 0 | 0 | 0 |
| MMP-7 | 0 | 46.2±2.3% | 55.4±3.8% | 0 | 0 | 0 |
| MMP-9 | 0 | 33.3±1.5% | 53.3±4.4% | 0 | 0 | 0 |
| MMP-11 | 0 | 7.6±2.4% | 26.5±1.8% | 0 | 0 | 0 |
| MMP-13 | 0 | 16±2.4% | 44.5±2.5% | 0 | 0 | 0 |
| MMP-14 | 0 | 29.3±2.2% | 47.1±3.5% | 0 | 0 | 0 |

Similar results to those shown in Table 20 were obtained when co-cultured with cells PHF tumor cell line of transitional cell carcinoma of human urinary bladder (RT-4).

**Table 21. Analysis of the expression of different molecular markers associated with tumor development and progression in fibroblasts (PHF) co-cultured with tumor cells in human prostate carcinoma (reference 22Rv1) (co-culture) in the presence of SP antagonists alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 41.4±2.1% | 0 | 0 | 0 |
| TGF-β 1 | 0 | 13.4±1.1% | 34.5±3.1% | 0 | 0 | 0 |
| TGF-β 2 | 0 | 20.5±1.2% | 42.5±2.1% | 0 | 0 | 0 |
| TGF-β 3 | 0 | 13.1±1.6% | 4.11±3.6% | 0 | 0 | 0 |
| SPARC | 0 | 17±1.8% | 48.1±3.2% | 0 | 0 | 0 |
| MMP-3 | 0 | 23.4±1.2% | 43.3±2.7% | 0 | 0 | 0 |
| MMP-7 | 0 | 46.2±2.3% | 54.3±4.8% | 0 | 0 | 0 |
| MMP-9 | 0 | 33.3±1.5% | 51.4±2.4% | 0 | 0 | 0 |
| MMP-11 | 0 | 7.6±2.4% | 28.5±3.8% | 0 | 0 | 0 |
| MMP-13 | 0 | 16±2.4% | 46±2.5% | 0 | 0 | 0 |
| MMP-14 | 0 | 29.3±2.2% | 49±3.5% | 0 | 0 | 0 |

**Table 22. Analysis of the expression of different molecular markers associated with tumor development and progression in fibroblasts (PHF) co-cultured with human glioma tumor cells (reference GAMG) (co-culture) in the presence of SP alone or with nonpeptide NK1 receptor. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.5±3.1% | 0 | 0 | 0 |
| TGF-β 1 | 0 | 13.4±1.1% | 33.6±3.1% | 0 | 0 | 0 |
| TGF-β 2 | 0 | 20.5±1.2% | 40.7±2.1% | 0 | 0 | 0 |
| TGF-β 3 | 0 | 13.1±1.6% | 51±1.6% | 0 | 0 | 0 |
| SPARC | 0 | 17±1.8% | 51.2±3.3% | 0 | 0 | 0 |
| MMP-3 | 0 | 23.4±1.2% | 43.4±2.8% | 0 | 0 | 0 |
| MMP-7 | 0 | 46.2±2.3% | 57.1±3.8% | 0 | 0 | 0 |
| MMP-9 | 0 | 33.3±1.5% | 52.3±2.4% | 0 | 0 | 0 |
| MMP-11 | 0 | 7.6±2.4% | 27.5±3.8% | 0 | 0 | 0 |
| MMP-13 | 0 | 16±2.4% | 45.4±3.6% | 0 | 0 | 0 |
| MMP-14 | 0 | 29.3±2.2% | 49.3±3.5% | 0 | 0 | 0 |

**Table 23. Analysis of the expression of different molecular markers associated with tumor development and progression in fibroblasts (PHF) co-cultured with human fibrosarcoma tumor cells (HT-1080 reference) (co-culture) in the presence of SP antagonists alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.5±3.1% | 0 | 0 | 0 |
| TGF-β 1 | 0 | 13.4±1.1% | 33.6±3.1% | 0 | 0 | 0 |
| TGF-β 2 | 0 | 20.5±1.2% | 40.7±2.1% | 0 | 0 | 0 |
| TGF-β 3 | 0 | 13.1±1.6% | 51±1.6% | 0 | 0 | 0 |
| SPARC | 0 | 17±1.8% | 51.2±3.3% | 0 | 0 | 0 |
| MMP-3 | 0 | 23.4±1.2% | 43.4±2.8% | 0 | 0 | 0 |
| MMP-7 | 0 | 46.2±2.3% | 57.1±3.8% | 0 | 0 | 0 |
| MMP-9 | 0 | 33.3±1.5% | 52.3±2.4% | 0 | 0 | 0 |
| MMP-11 | 0 | 7.6±2.4% | 27.5±3.8% | 0 | 0 | 0 |
| MMP-13 | 0 | 16±2.4% | 45.4±3.6% | 0 | 0 | 0 |
| MMP-14 | 0 | 29.3±2.2% | 49.3±3.5% | 0 | 0 | 0 |

**Table 24. Analysis of the expression of different molecular markers associated with tumor development and progression in fibroblasts (PHF) co-cultured with tumor cells of human Edwing sarcoma (reference MHH-ES-1) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 36.4±3.1% | 0 | 0 | 0 |
| TGF-β 1 | 0 | 13.4±1.1% | 35.5±2.1% | 0 | 0 | 0 |
| TGF-β 2 | 0 | 20.5±1.2% | 42.6±5.1% | 0 | 0 | 0 |
| TGF-β 3 | 0 | 13.1±1.6% | 42.4±1.6% | 0 | 0 | 0 |
| SPARC | 0 | 17±1.8% | 46.5±3.2% | 0 | 0 | 0 |
| MMP-3 | 0 | 23.4±1.2% | 43.5±3.7% | 0 | 0 | 0 |
| MMP-7 | 0 | 46.2±2.3% | 56.7±4.8% | 0 | 0 | 0 |
| MMP-9 | 0 | 33.3±1.5% | 54.4±2.4% | 0 | 0 | 0 |
| MMP-11 | 0 | 7.6±2.4% | 28.4±3.8% | 0 | 0 | 0 |
| MMP-13 | 0 | 16±2.4% | 46.6±3.6% | 0 | 0 | 0 |
| MMP-14 | 0 | 29.3±2.2% | 49.6±4.2% | 0 | 0 | 0 |

Similar results to those shown in Tables 23 and 24 were obtained when co-cultured cells PHF with tumor cell lines of human malignant fibrous histiocytoma (U-2197) or human endometrial stromal sarcoma (ESS-1) or Human osteosarcoma (CAL-72) or human rhabdomyosarcoma (A-204).

**Table 25. Analysis of the expression of different molecular markers associated with tumor development and progression in fibroblasts (PHF) co-cultured with human melanoma tumor cells (MEL-HO1 reference) (co-culture) in the presence of SP antagonists alone or together with nonpeptide NK1. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±3.2% | 0 | 0 | 0 |
| TGF-β 1 | 0 | 13.4±1.1% | 42.5±2.1% | 0 | 0 | 0 |
| TGF-β 2 | 0 | 20.5±1.2% | 44.5±1.9% | 0 | 0 | 0 |
| TGF-β 3 | 0 | 13.1±1.6% | 43.5±2.6% | 0 | 0 | 0 |
| SPARC | 0 | 17±1.8% | 44.5±1.4% | 0 | 0 | 0 |
| MMP-3 | 0 | 23.4±1.2% | 44.5±4.7% | 0 | 0 | 0 |
| MMP-7 | 0 | 46.2±2.3% | 55.5±3.8% | 0 | 0 | 0 |
| MMP-9 | 0 | 33.3±1.5% | 53.3±2.4% | 0 | 0 | 0 |
| MMP-11 | 0 | 7.6±2.4% | 24.4±3.8% | 0 | 0 | 0 |
| MMP-13 | 0 | 16±2.4% | 46.1±3.5% | 0 | 0 | 0 |
| MMP-14 | 0 | 29.3±2.2% | 46.2±3.5% | 0 | 0 | 0 |

Similar results to those shown in Table 25 were obtained when PHF cocultured with cells from the cell line of human melanoma tumor, lymph node metastasis (COLO 858).

**Table 26. Analysis of the expression of different molecular markers associated with tumor development and progression in fibroblasts (PHF) co-cultured with human neuroblastoma tumor cells (reference KELLY) (co-culture) in the presence of SP alone or with nonpeptide NK1 receptor. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 42.4±3.1% | 0 | 0 | 0 |
| TGF-β 1 | 0 | 13.4±1.1% | 36.5±2.1% | 0 | 0 | 0 |
| TGF-β 2 | 0 | 20.5±1.2% | 42.2±2.2% | 0 | 0 | 0 |
| TGF-β 3 | 0 | 13.1±1.6% | 43.4±2.3% | 0 | 0 | 0 |
| SPARC | 0 | 17±1.8% | 46.5±4.2% | 0 | 0 | 0 |
| MMP-3 | 0 | 23.4±1.2% | 45.4±4.7% | 0 | 0 | 0 |
| MMP-7 | 0 | 46.2±2.3% | 57.4±3.8% | 0 | 0 | 0 |
| MMP-9 | 0 | 33.3±1.5% | 51.2±2.4% | 0 | 0 | 0 |
| MMP-11 | 0 | 7.6±2.4% | 29.4±4.8% | 0 | 0 | 0 |
| MMP-13 | 0 | 16±2.4% | 44.3±4.5% | 0 | 0 | 0 |
| MMP-14 | 0 | 29.3±2.2% | 51.2±4.2% | 0 | 0 | 0 |

Similar results to those shown in Table 26 were obtained when co-cultured with cells PHF tumor cell lines of human neuroblastoma bone marrow (SKN-BE 2) or human retinoblastoma (WERI-RB-1).

**Table 27. Analysis of the expression of different molecular markers associated with tumor development and progression in fibroblasts (PHF) co-cultivated with tumor cell lymphoblastic leukemia B (reference SDI) (co-culture) in the presence of SP antagonists alone or with no NK1 peptide receptor. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 41.4±3.1% | 0 | 0 | 0 |
| TGF-β 1 | 0 | 13.4±1.1% | 42.5±4.2% | 0 | 0 | 0 |
| TGF-β 2 | 0 | 20.5±1.2% | 50.5±1.1% | 0 | 0 | 0 |
| TGF-β 3 | 0 | 13.1±1.6% | 51.4±5.5% | 0 | 0 | 0 |
| SPARC | 0 | 17±1.8% | 56±6.2% | 0 | 0 | 0 |
| MMP-3 | 0 | 23.4±1.2% | 53.4±5.7% | 0 | 0 | 0 |
| MMP-7 | 0 | 46.2±2.3% | 59.1±4.8% | 0 | 0 | 0 |
| MMP-9 | 0 | 33.3±1.5% | 59.3±3.4% | 0 | 0 | 0 |
| MMP-11 | 0 | 7.6±2.4% | 47.4±4.8% | 0 | 0 | 0 |
| MMP-13 | 0 | 16±2.4% | 49.5±6.5% | 0 | 0 | 0 |
| MMP-14 | 0 | 29.3±2.2% | 53.9±5.5% | 0 | 0 | 0 |

**Table 28. Analysis of the expression of different molecular markers associated with tumor development and progression in fibroblasts (PHF) co-cultured with tumor cells of human Burkitt lymphoma (reference CA-46) (co-culture) in the presence of SP alone or together with nonpeptide NK1 receptors. The table shows the percentage ± SD of the number of cells expressing each marker compared to all cells of each specimen.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 61.4±5.1% | 0 | 0 | 0 |
| TGF-β 1 | 0 | 13.4±1.1% | 63.4±4.1% | 0 | 0 | 0 |
| TGF-β 2 | 0 | 20.5±1.2% | 60.5±5.1% | 0 | 0 | 0 |
| TGF-β 3 | 0 | 13.1±1.6% | 71±4.56% | 0 | 0 | 0 |
| SPARC | 0 | 17±1.8% | 77±4.2% | 0 | 0 | 0 |
| MMP-3 | 0 | 23.4±1.2% | 63.4±4.3% | 0 | 0 | 0 |
| MMP-7 | 0 | 46.2±2.3% | 66.1±3.8% | 0 | 0 | 0 |
| MMP-9 | 0 | 33.3±1.5% | 72.3±4.4% | 0 | 0 | 0 |
| MMP-11 | 0 | 7.6±2.4% | 67.4±5.8% | 0 | 0 | 0 |
| MMP-13 | 0 | 16±2.4% | 55±6.4% | 0 | 0 | 0 |
| MMP-14 | 0 | 29.3±2.2% | 59.1±6.3% | 0 | 0 | 0 |

**Table 29. Analysis of the expression of different molecular markers associated with tumor development and progression in fibroblasts (PHF) cells co-cultured with human T lymphoma tumor (Reference DERL-7) (co-culture) in the presence of SP alone or together with nonpeptide NK1 receptors. The table shows the percentage ± SD of the number of cells expressing each marker compared to total cells for each sample.**

| **Marker** | **PHF** | **Co-cultured** | **Co-cultured +SP** | **Co-cultured +SP +Aprepitant** | **Co-cultured +SP +Vestipitant** | **Co-cultured +SP +Casopitant** |
|---|---|---|---|---|---|---|
| TGF-α | 0 | 12.3±1.1% | 62.5±3.1% | 0 | 0 | 0 |
| TGF-β 1 | 0 | 13.4±1.1% | 64.5±4.3% | 0 | 0 | 0 |
| TGF-β 2 | 0 | 20.5±1.2% | 70.6±4.1% | 0 | 0 | 0 |
| TGF-β 3 | 0 | 13.1±1.6% | 61±5.6% | 0 | 0 | 0 |
| SPARC | 0 | 17±1.8% | 67±4.2% | 0 | 0 | 0 |
| MMP-3 | 0 | 23.4±1.2% | 63.4±3.7% | 0 | 0 | 0 |
| MMP-7 | 0 | 46.2±2.3% | 66.1±4.8% | 0 | 0 | 0 |
| MMP-9 | 0 | 33.3±1.5% | 62.3±3.4% | 0 | 0 | 0 |
| MMP-11 | 0 | 7.6±2.4% | 67.8±4.8% | 0 | 0 | 0 |
| MMP-13 | 0 | 16±2.4% | 61±5.3% | 0 | 0 | 0 |
| MMP-14 | 0 | 29.3±2.2% | 58.4±3.5% | 0 | 0 | 0 |

Similar results to those shown in Tables 27 to 29 were obtained when co-cultured with cells PHF tumor cell lines, various hematological cancers, such as human T-cell leukemia / NK (YT) or human T lymphoblastic leukemia (BE-13) or human B lymphoma (BC-1) or human Hodgkin's lymphoma (KM-H2) or human multiple myeloma (COLO-677).

Therefore, the results shown in this example reveal that the interaction between tumor cells and stromal cells, fibroblasts, induce changes and modifications in the physiology of these cells itself corresponding to said cells in the context associated with cancer, such as TGF-α, TGF-β 1, TGF-β 2, TGF-β 3, SPARC, MMP-3, MMP-7, MMP-9, MMP-11, MMP-13 and MMP-14. The addiction to co-culture NK1 receptor agonist (SP) induces an increased secretion of these substances and this secretion is reversed by treatment with non-peptide antagonists of this receptor. The expression of these substances is a key event in tumor progression and is, in turn, a key event in the survival and maintenance of tumors. Using NK1 receptor antagonists have, therefore, beneficial effects in the treatment of cancer.

### Example 6. Non-peptide NK1 receptor antagonists inhibit, by fibroblasts, the secretion of substances that promote the progression of cancer in vivo.

*In vitro* tests conducted in Example 5 are now carried out in an experimental model in mice *in vivo.* To check that the interaction of tumor cells with stromal cells- fibroblasts-induce changes in these stromal cells similar to those observed in peritumoral areas, tumor cells were implanted into mice and were subsequently treated with non-peptide antagonists of the NK1 receptor. Subsequently, the expression in fibroblasts tumoral and peritumoral area of molecular markers associated with progression and maintenance of tumors: TGF-α, TGF-β 1, TGF-β 2, TGF- β 3, MMP-7, MMP-11, MMP-14 were analyzed by immunohistochemistry.

The authorization for this animal experiment was obtained from the ethics committee of the Hospital Juan Ramón Jiménez de Huelva. Immunocompromised female mice 5-6 weeks of age, nu/nu Balb/c nude mice, supplied by Harlan Ibérica Barcelona (Spain) were used. They were kept at 24°C and sterile conditions with food and water "ad libitum". Were injected with 2 x 10⁷ cells tumors (corresponding, in each case to each of the tumor types as specified in Table 9) in 200 of PBS subcutaneously µl. Tumor size was measured and mice were assessed for their health and weight daily. Mice were randomized into two groups when the tumors reached a volume of 75 mm³. One group was treated with non-peptide receptor antagonists NK1 and the other with placebo 200µ1. Table 30 is a list of non-peptide receptor antagonists NK1 used, the route of administration and dose. They were treated 10 animals per group, for 7 days. All mice were sacrificed at the end of the experiment. Tumors were excised, besides taking a sample of subcutaneous tissue away from the tumor stroma. Tumors and samples of non-neoplastic stromal areas remote from the tumor were halved, formalin fixed (4%) and then were included in paraffin blocks for analysis by immunohistochemistry.

The primary antibodies used for immunohistochemistry were TGF-α (SAB4502953), TGF-β 1 (SAB4502954), TGF-β 2 (SAB4502956), TGF-β 3 (SAB4502957), MMP-7 (SAB4501894), MMP- 11 (SAB4501898), MMP-14 (SAB4501901).

**Table 30. Non-peptide receptor NK1 antagonists used in the animal model.**

| **Antagonist** | **Dose** | **Route of administration** |
|---|---|---|
| L-733,060 | 30 mg/kg/day | oral |
| L-732,138 | 10 mg / kg / day | intraperitoneal |
| L-733,606 | 10 mg / kg / day | intraperitoneal |
| Aprepitant | 30 mg/kg/day | oral |
| Vestipitant | 30 mg/kg/day | oral |
| Casopitant | 30 mg/kg/day | oral |
| CP-100263 | 10 mg / kg / day | intraperitoneal |
| WIN 62,577 | 10 mg / kg / day | intraperitoneal |
| L-760735 | 30mg/kg/day | oral |

Table 31 shows the mean volume ± SD of tumors from the control mice (untreated) or treated with non-peptide antagonists of the NK-1 receptor. This table also indicates the cell line used for the formation of tumors, specifying in each case the type of tumor that corresponds, the company that supplies and catalogue code.

**Table 31. Tumor type caused in vivo mouse model. Mean tumor volume in the control mice (untreated) or in mice after treatment with non-peptide receptor antagonists NK1.**

| **Cell line** | **Control** | **Aprepitant** | **Vestipitant** | **Casopitant** |
|---|---|---|---|---|
| Human gastric adenocarcinoma 23132/87 | 230±9 | 77±6 | 63±8 | 83±4 |
| Human colon carcinoma SW-403 | 240±12 | 79±4 | 78±2 | 78±7 |
| Human pancretic adenocarcinoma CAPAN-1 | 210±8 | 86±8 | 85±8 | 73±8 |
| Human pancretic adenocarcinoma PA-TU 8902 | 230±10 | 102±7 | 91±6 | 75±6 |
| Human breast adenocarcinoma humano MCF-7 | 240±11 | 95±6 | 78±7 | 86±8 |
| Human breast carcinoma MT-3 | 221±9 | 109±2 | 78±8 | 87±12 |
| Human breast carcinoma MDA-MB-468 | 241±8 | 99±7 | 84±7 | 77±9 |
| Human ovarian adenocarcinoma EFO-27 | 210±9 | 98±7 | 63±7 | 88±6 |
| Human ovarian carcinoma COLO-704 | 240±18 | 101±7 | 78±8 | 97±6 |
| Human endometrial carcinoma AN3-CA | 235±12 | 90±4 | 89±1 | 82±3 |
| Human choriocarcinoma, AC-1M32 | 243±18 | 112±4 | 88±8 | 84±1 |
| Human uterine cervix carcinoma KB | 251±11 | 103±9 | 89±1 | 112±1 |
| Human lung carcinoma DV-90 | 236±10 | 97±3 | 136±10 | 99±5 |
| Carcinoma of human non-small cell lung HCC-44 | 254±11 | 105±7 | 100±4 | 101±8 |
| Carcinoma of human non-small cell lung COR-L23 | 261±12 | 102±5 | 111±3 | 119±5 |
| Carcinoma of human small cell lung H-209 | 243±9 | 104±6 | 113±5 | 114±7 |
| Carcinoma of human non-small cell lung H-1963 | 210±6 | 98±5 | 101±6 | 97±3 |
| Human lung carcinoma A-427 | 230±10 | 104±4 | 98±10 | 102±8 |
| Human thyroid carcinoma 8505C | 259±9 | 105±3 | 98±9 | 99±9 |
| Human papillary thyroid carcinoma metastasizing B-CPAP | 252±12 | 102±4 | 102±2 | 106±4 |
| Human folicular thyroid carcinoma TT2609-C02 | 321±23 | 105±7 | 100±4 | 112±5 |
| Carcinoma of human urinary bladder 5637 | 235±12 | 102±4 | 112±3 | 100±2 |
| Transitional Carcinoma of human urinary bladder RT-4 | 253±11 | 102±6 | 120±3 | 101±3 |
| Human prostate carcinoma 22RV1 | 325±12 | 103±5 | 98±2 | 98±4 |
| Human glioma GAMG | 199±12 | 108±8 | 101±4 | 102±4 |
| Human Fibrosarcoma HT-1080 | 312±9 | 100±4 | 112±4 | 89±4 |
| Human malignant fibrous histiocytoma U-2197 | 214±8 | 102±6 | 95±8 | 107±2 |
| Sarcoma human Edwing MHH-ES-1 | 245±12 | 103±5 | 115±3 | 97±9 |
| Human endometrial stromal sarcoma ESS-1 | 212±10 | 107±4 | 99±3 | 101±5 |
| Human osteosarcoma CAL-72 | 245±9 | 112±4 | 104±9 | 106±7 |
| Human Rhabdomyosarcoma A-204 | 267±9 | 102±8 | 97±9 | 98±5 |
| Human melanoma MEL HO | 312±23 | 104±5 | 102±4 | 102±9 |
| Melanoma humano, metástasis en ganglio linfático COLO 858 | 211±10 | 109±4 | 110±4 | 99±5 |
| Human neuroblastoma KELLY | 234±19 | 105±6 | 121±4 | 112±5 |
| Human neuroblastoma bone marrow SKN-BE 2 | 288±7 | 97±9 | 101±7 | 103±4 |
| Human retinoblastoma WERI-Rb-1 | 274±8 | 99±4 | 112±8 | 101±5 |
| Human T-cell leukemia/NK YT | 305±11 | 106±6 | 101±3 | 102±7 |
| B lymphoblastic leukemia (peripheral human B lymphoblastoid cells) SD1 | 233±8 | 103±8 | 112±8 | 109±8 |
| Human lymphoblastic leukemia T BE-13 | 223±14 | 102±6 | 111±4 | 101±4 |
| Human B lymphoma BC-1 | 209±9 | 108±5 | 101±9 | 83±11 |
| Human Burkitt lymphoma CA-46 | 294±19 | 109±7 | 100±19 | 94±9 |
| Human Hodgkin lymphoma KM-H2 | 310±8 | 101±5 | 99±8 | 86±7 |
| Human T-lymphoma DERL human Hodgkin 7Linfoma KM-H2 | 250±9 | 105±6 | 90±9 | 97±6 |
| Human multiple myeloma COLO-677 | 224±21 | 102±6 | 101±4 | 104±6 |

The expression on fibroblast cells obtained in the animal model was analyzed by immunohistochemistry, of the above markers: TGF-α, TGF-β 1, TGF-β 2, TGF-β 3, MMP-7, MMP-1 1, MMP-14MP-14. Tables 32 to 35 depict the type of label used and the average percentage of fibroblasts present in non-tumor areas, tumor and peritumoral (± SD) which were expressing the marker shown in each of the tumor types developed from of the different cell lines used. This process was realized in mice treated with non-peptide antagonists of NK1 receptor and in untreated animals (control). For example, Tables 32 to 35 demonstrate the results obtained with four tumor lines as described in Table 9.

**Table 32. Analysis of the expression of different molecular markers associated with tumor development and progression in fibroblasts isolated from the tumor or peritumoral area of tumors produced in a mouse model by injecting tumor cells of the lung carcinoma non small cell human (HCC-44), in mice treated or not (control group) with different NK1 receptor antagonists. Table show the percentage ± SD of the number of fibroblasts expressing each marker compared to total cells for each sample.**

| **Marker** | **Control group** | **Aprepitant group** | **Vestipitant group** | **Casopitant group** |
|---|---|---|---|---|
| TGF-α | 32.4±1.1% | 0 | 0 | 0 |
| TGF-β 1 | 33.5±1.1% | 0 | 0 | 0 |
| TGF-β 2 | 41.3±1.5% | 0 | 0 | 0 |
| TGF-β 3 | 33.2±1.9% | 0 | 0 | 0 |
| MMP-7 | 46.5±2.3% | 0 | 0 | 0 |
| MMP-11 | 27.6±3.3% | 0 | 0 | 0 |
| MMP-14 | 25.4±3.1% | 0 | 0 | 0 |

**Table 33. Analysis of the expression of different molecular markers associated with tumor development and progression in fibroblasts isolated from the tumor or peritumoral area of tumors produced in a mouse model by injecting cells from the human glioma tumor line (GAMG) in mice treated or not (control group) with different NK1 receptor antagonists. The table shows the percentage ± SD of the number of fibroblasts expressing each marker to total cells in each sample.**

| **Marker** | **Control group** | **Aprepitant group** | **Vestipitant group** | **Casopitant group** |
|---|---|---|---|---|
| TGF-α | 32.4±1.1% | 0 | 0 | 0 |
| TGF-β 1 | 33.5±1.1% | 0 | 0 | 0 |
| TGF-β 2 | 41.3±1.5% | 0 | 0 | 0 |
| TGF-β 3 | 33.2±1.9% | 0 | 0 | 0 |
| MMP-7 | 46.5±2.3% | 0 | 0 | 0 |
| MMP-11 | 27.6±3.3% | 0 | 0 | 0 |
| MMP-14 | 25.4±3.1% | 0 | 0 | 0 |

**Table 34. Analysis of the expression of different molecular markers associated with tumor development and progression in fibroblasts isolated from the tumor or peritumoral area of tumors produced in a mouse model by injecting cells from the human rhabdomyosarcoma tumor line (A-204) in mice treated or not (control group) with different NK1 receptor antagonists. The table shows the percentage ± SD of the number of fibroblasts expressing each marker to total cells in each sample.**

| **Marker** | **Control group** | **Aprepitant group** | **Vestipitant group** | **Casopitant group** |
|---|---|---|---|---|
| TGF-α | 32.4±1.1% | 0 | 0 | 0 |
| TGF-β 1 | 33.5±1.1% | 0 | 0 | 0 |
| TGF-β 2 | 41.3±1.5% | 0 | 0 | 0 |
| TGF-β 3 | 33.2±1.9% | 0 | 0 | 0 |
| MMP-7 | 46.5±2.3% | 0 | 0 | 0 |
| MMP-11 | 27.6±3.3% | 0 | 0 | 0 |
| MMP-14 | 25.4±3.1% | 0 | 0 | 0 |

**Table 35. Analysis of the expression of different molecular markers associated with tumor development and progression in fibroblasts isolated from the tumor or peritumoral area of tumors produced in a mouse model by injecting tumor cell line of human melanoma (MEL HO) in mice treated or not (control group) with different NK1 receptor antagonists. The table shows the percentage ± SD of the number of fibroblasts expressing each marker to total cells in each sample.**

| **Marker** | **Control group** | **Aprepitant group** | **Vestipitant group** | **Casopitant group** |
|---|---|---|---|---|
| TGF-α | 32.4±1.1% | 0 | 0 | 0 |
| TGF-β1 | 33.5±1.1% | 0 | 0 | 0 |
| TGF-β2 | 41.3±1.5% | 0 | 0 | 0 |
| TGF-β3 | 33.2±1.9% | 0 | 0 | 0 |
| MMP-7 | 46.5±2.3% | 0 | 0 | 0 |
| MMP-11 | 27.6±3.3% | 0 | 0 | 0 |
| MMP-14 | 25.4±3.1% | 0 | 0 | 0 |

As illustrated in Tables 32 to 35, fibroblasts of tumor and peritumoral areas express tumor markers, while no expression is seen in the areas thereof not tumor areas, these are demonstrating that the interaction "*in vivo*" of fibroblasts with the tumor cells induces the expression and secretion of these molecules. It is also demonstrated that treatment with non-peptide NK1 receptors inhibits the expression of these molecules. Therefore, in mammals the interaction between the fibroblasts and the tumor cells induces the expression in fibroblasts of the aforementioned molecules of importance in the microenvironment in the persistence and progression of tumors. This expression is cancelled by exposure to non-peptide antagonists of the NK1 receptor.

In stromal cell samples, fibroblasts, tumor remote areas of all the cases treated with the various NK1 receptor antagonists and control groups (untreated) the fibroblasts percentage expression of all studied was immunohistochemical markers 0 %. Similar results to those shown in Tables 33 to 35 were obtained when we used other nonpeptide NK1 receptor: L-733,060, L-732,138, L-703, 606, CP-100263, WIN 62.577, WIN 51708, CP- 96345 and L-760735.

### Example 7. The interaction of primary tumor cells, obtained directly from human with human vascular endothelial cells, promotes the survival of both types of cells and treatment with non-peptide NK1 receptors antagonists inhibits said survival.

To check that the interaction of primary tumor cells extracted directly from human with human vascular endothelial cells, promotes the survival of both cell types, co-cultures were performed microvascular endothelial cells from juvenile foreskins (PromoCell GmbH, Sickingenstraβe 63/65 , D-69126 Heidelberg, Germany; reference C-12210) with tumor cells obtained directly from primary human tumors. Individual tumors and patient characteristics are shown in Table 36.

**Table 36. Characteristics of patients from whom samples were taken for cell culture**

| **Type of tumor** | **Gender** | **Age** | **Histological type** |
|---|---|---|---|
| Carcinoma of stomach. | Male | 56 | Adenocarcinoma |
| Carcinoma of Colon. | Male | 61 | Adenocarcinoma |
| Carcinoma of Pancreas. | Female | 52 | Adenocarcinoma |
| Renal carcinoma | Male | 62 | Clear cell carcinoma |
| Carcinoma of breast. | Female | 48 | Ductal adenocarcinoma. |
| Carcinoma of ovarian. | Female | 59 | Adenocarcinoma |
| Carcinoma of endometrial. | Female | 63 | Adenocarcinoma |
| Carcinoma of cervix. | Female | 36 | Squamous cell carcinoma |
| Carcinoma of lung | Male | 57 | Small cell carcinoma |
| Carcinoma of lung | Male | 65 | Non Small Cell Carcinoma |
| Carcinoma of thyroid. | Female | 42 | Follicular Carcinoma |
| Carcinoma of thyroid. | Female | 37 | Papillary Carcinoma |
| Carcinoma of prostate | Male | 71 | Adenocarcinoma (Gleasson 3+4) |
| Glioma | Male | 57 | Oligoastrocytoma |
| Fibrosarcoma | Male | 61 | Fiborarcoma Histologic Grade 2 |
| Malignant fibrous histiocytom | Male | 72 | Malignant fibrous histiocytoma (Grade 3). |
| Sarcoma of Ewing. | Male | 31 | Ewing Sarcoma |
| Melanoma | Mujer | 61 | Nodular melanoma. |
| Neuroblastoma | Male | 12 | Neuroblastoma |
| Leukemia B | Female | 21 | Chronic leukemia B |
| Leukemia T | Male | 32 | Chronic leukemia T |
| Non-Hodgkin's Lymphoma B | Male | 52 | Angioimmunoblastic T-cell lymphoma |
| Non-Hodgkin's Lymphoma T | Male | 34 | Cell Lymphoma Diffuse Large B |
| Hodgkin's Lymphoma | Male | 41 | Hodgkin Lymphoma |
| Myeloma | Male | 63 | Multiple Myeloma |

The same method described in Example 2 was used to obtain tumor cells from primary human tumors Once isolated, tumor cells were seeded in 24-well plates at a concentration of 25,000 cells per well. A total of 6 wells containing tumor cells were used as control for survival. Another 6 wells containing tumor cells were cultured with addition of endothelial cells. Another 6 wells containing tumor cells were cultured in the presence of endothelial cells and NK1 receptor agonist (SP). Other groups of six wells containing tumor cells were cultured in the presence of endothelial cells, NK1 receptor agonist (SP) and each of the non-peptide receptor antagonists NK1, Aprepitant, Vestipitant and Casopitant.

Firstly, it was found that both tumor cells and endothelial cells express NK1 receptor by Western blotting as described in Example 1. Subsequently, cells were embedded in paraffin for analysis by immunohistochemistry. In order to identify the different cell types and quantity of them [They were labelled with primary antibodies specific for human endothelial cells (anti-CD31), carcinoma cells (Anti-Cytokeratins Spread Spectrum), glioma (Anti- glial fibrillary acidic protein), fibrosarcoma and malignant fibrous histiocytoma (Anti-Smooth Muscle Actin), Ewing sarcoma (Anti-CD99), Melanoma (Anti-HMB45), leukemias and lymphomas (Anti-Common Antigen Leucitario) and myeloma (anti CD 138). All antibodies are distributed by Dako and were used at the same concentration which is supplied (supplied pre-diluted - "ready to use").

Tables 37 to 41 detail the percentage inhibition/survival in reference to control for co-cultures of tumor cells-endothelial cells (Table 37), tumor cells-endothelial cells with NK1 receptor agonist exposure (SP) (Table 38), tumor cells-endothelial cells with NK1 receptor antagonist exposure (SP) and each of NK1 receptor antagonists: Aprepitant, Vestipitant and Casopitant (Tables 39-41). We obtained the same results when used other nonpeptide receptor NK1: L-733,060, L-732,138, L-703,606, CP-100263, WIN 62,577, WIN 51708, CP-96345 and L-760735.

**Table 37. Percent inhibition/survival in reference to the control of tumor cell co-cultures-endothelial cells.**

| **Type of tumor** | **Endothel ial cells (control)** | **Tumor cells (control)** | **Endothelial cells co-cultured with tumor cells** | **Tumor cells co-cultured with endothelial cells.** |
|---|---|---|---|---|
| Carcinoma of stomach. | 100 | 100 | 150±3 | 160±5 |
| Carcinoma of Colon. | 100 | 100 | 142±4 | 153±4 |
| Carcinoma of Pancreas. | 100 | 100 | 187±2 | 152±3 |
| Renal carcinoma | 100 | 100 | 182±5 | 160±5 |
| Carcinoma of breast. | 100 | 100 | 181±3 | 153±4 |
| Carcinoma of ovarian. | 100 | 100 | 182±5 | 154±3 |
| Carcinoma of endometrial. | 100 | 100 | 186±4 | 152±4 |
| Carcinoma of cervix. | 100 | 100 | 191±6 | 160±3 |
| Carcinoma of lung | 100 | 100 | 193±3 | 165±4 |
| Carcinoma of lung | 100 | 100 | 184±2 | 165±3 |
| Carcinoma of thyroid. | 100 | 100 | 180±3 | 150±5 |
| Carcinoma of thyroid. | 100 | 100 | 185±3 | 150±5 |
| Carcinoma of prostate | 100 | 100 | 180±3 | 169±1 |
| Glioma | 100 | 100 | 188±7 | 157±9 |
| Fibrosarcoma | 100 | 100 | 189±4 | 167±3 |
| Malignant fibrous histiocytom | 100 | 100 | 196±4 | 145±4 |
| Sarcoma of Ewing. | 100 | 100 | 187±5 | 157±4 |
| Melanoma | 100 | 100 | 187±5 | 157±3 |
| Neuroblastoma | 100 | 100 | 188±4 | 141±6 |
| Leukemia B | 100 | 100 | 179±5 | 157±7 |
| Leukemia T | 100 | 100 | 179±5 | 148±7 |
| Non-Hodgkin's Lymphoma B | 100 | 100 | 135±8 | 140±6 |
| Non-Hodgkin's Lymphoma T | 100 | 100 | 150±3 | 150±5 |
| Hodgkin's Lymphoma | 100 | 100 | 163±3 | 140±5 |
| Myeloma | 100 | 100 | 165±3 | 150±5 |

**Table 38. Percent inhibition/survival in reference to control for co-cultures of tumor cells-endothelial cells in the presence of SP (1 µM).**

| **Type of tumor** | **Endotheli al cells (control)** | **Tumor cells (control)** | **Endothelial cells co-cultured with tumor cells+SP** | **Tumor cells co-cultured with endothelial cells+SP** |
|---|---|---|---|---|
| Carcinoma of stomach. | 100 | 100 | 251±3 | 260±4 |
| Carcinoma of Colon. | 100 | 100 | 243±4 | 253±4 |
| Carcinoma of Pancreas. | 100 | 100 | 287±3 | 252±4 |
| Renal carcinoma | 100 | 100 | 282±4 | 260±4 |
| Carcinoma of breast. | 100 | 100 | 281±4 | 253±5 |
| Carcinoma of ovarian. | 100 | 100 | 282±4 | 254±4 |
| Carcinoma of endometrial. | 100 | 100 | 283±5 | 252±3 |
| Carcinoma of cervix. | 100 | 100 | 292±4 | 260±4 |
| Carcinoma of lung | 100 | 100 | 294±2 | 264±3 |
| Carcinoma of lung | 100 | 100 | 274±5 | 245±6 |
| Carcinoma of thyroid. | 100 | 100 | 260±5 | 240±6 |
| Carcinoma of thyroid. | 100 | 100 | 265±2 | 230±4 |
| Carcinoma of prostate | 100 | 100 | 270±4 | 269±1 |
| Glioma | 100 | 100 | 238±6 | 247±5 |
| Fibrosarcoma | 100 | 100 | 249±5 | 257±3 |
| Malignant fibrous histiocytoma | 100 | 100 | 246±4 | 245±3 |
| Sarcoma of Ewing. | 100 | 100 | 234±5 | 236±4 |
| Melanoma | 100 | 100 | 267±4 | 245±2 |
| Neuroblastoma | 100 | 100 | 258±3 | 256±5 |
| Leukemia B | 100 | 100 | 278±4 | 265±6 |
| Leukemia T | 100 | 100 | 276±4 | 256±6 |
| Non-Hodgkin's Lymphoma B | 100 | 100 | 254±7 | 267±5 |
| Non-Hodgkin's Lymphoma T | 100 | 100 | 256±4 | 276±4 |
| Hodgkin's Lymphoma | 100 | 100 | 248±5 | 265±4 |
| Myeloma | 100 | 100 | 267±2 | 232±3 |

**Table 39. Percent inhibition/survival in reference to control for co-cultures of tumor cells-endothelial cells in the presence of SP (1 µM) and non-peptide NK1 receptor antagonist, Aprepitant (1µM).**

| **Type of tumor** | **Endothelial cells (control)** | **Tumor cells (control)** | **Endothelial cells co-cultured with tumor cells+SP+ Aprepitant** | **Tumor cells co-cultured with endothelial cells+SP+ Aprepitant** |
|---|---|---|---|---|
| Carcinoma of stomach. | 100 | 100 | 65±2 | 54±5 |
| Carcinoma of Colon. | 100 | 100 | 57±3 | 61±3 |
| Carcinoma of Pancreas. | 100 | 100 | 52±4 | 46±6 |
| Renal carcinoma | 100 | 100 | 56±2 | 53±3 |
| Carcinoma of breast. | 100 | 100 | 56±6 | 56±3 |
| Carcinoma of ovarian. | 100 | 100 | 49±5 | 54±4 |
| Carcinoma of endometrial. | 100 | 100 | 57±4 | 49±3 |
| Carcinoma of cervix. | 100 | 100 | 45±3 | 46±3 |
| Carcinoma of lung | 100 | 100 | 46±7 | 63±3 |
| Carcinoma of lung | 100 | 100 | 43±4 | 65±4 |
| Carcinoma of thyroid. | 100 | 100 | 45±3 | 46±6 |
| Carcinoma of thyroid. | 100 | 100 | 56±6 | 56±4 |
| Carcinoma of prostate | 100 | 100 | 54±4 | 47±5 |
| Glioma | 100 | 100 | 56±5 | 49±4 |
| Fibrosarcoma | 100 | 100 | 45±6 | 55±6 |
| Malignant fibrous histiocytoma | 100 | 100 | 56±6 | 44±5 |
| Sarcoma of Ewing. | 100 | 100 | 45±5 | 45±7 |
| Melanoma | 100 | 100 | 56±6 | 46±5 |
| Neuroblastoma | 100 | 100 | 49±4 | 55±6 |
| Leukemia B | 100 | 100 | 46±5 | 54±6 |
| Leukemia T | 100 | 100 | 56±6 | 56±5 |
| Non-Hodgkin's Lymphoma B | 100 | 100 | 67±5 | 49±6 |
| Non-Hodgkin's Lymphoma T | 100 | 100 | 49±4 | 83±8 |
| Hodgkin's Lymphoma | 100 | 100 | 48±4 | 74±6 |
| Myeloma | 100 | 100 | 54±8 | 64±3 |

**Table 40. Percent inhibition/survival in reference to control, for co-cultures of tumor cells-endothelial cells in the presence of SP (1 µM) and non-peptide NK1 receptor antagonist, Vestipitant (1µM).**

| **Type of tumor** | **Endothelial cells (control)** | **Tumor cells (control)** | **Endothelial cells co-cultured with tumor cells+SP+ Vestipitant** | **Tumor cells co-cultured with endothelial cells+SP+ Vestipitant** |
|---|---|---|---|---|
| Carcinoma of stomach. | 100 | 100 | 62±3 | 56±4 |
| Carcinoma of Colon. | 100 | 100 | 54±3 | 65±6 |
| Carcinoma of Pancreas. | 100 | 100 | 55±3 | 47±5 |
| Renal carcinoma | 100 | 100 | 54±3 | 54±4 |
| Carcinoma of breast. | 100 | 100 | 55±4 | 55±5 |
| Carcinoma of ovarian. | 100 | 100 | 44±4 | 57±6 |
| Carcinoma of endometrial. | 100 | 100 | 56±5 | 47±6 |
| Carcinoma of cervix. | 100 | 100 | 47±4 | 47±4 |
| Carcinoma of lung | 100 | 100 | 45±6 | 67±5 |
| Carcinoma of lung | 100 | 100 | 45±3 | 64±6 |
| Carcinoma of thyroid. | 100 | 100 | 46±4 | 47±5 |
| Carcinoma of thyroid. | 100 | 100 | 52±5 | 55±6 |
| Carcinoma of prostate | 100 | 100 | 51±6 | 48±6 |
| Glioma | 100 | 100 | 54±3 | 47±5 |
| Fibrosarcoma | 100 | 100 | 46±7 | 56±7 |
| Malignant fibrous histiocytoma | 100 | 100 | 53±4 | 46±7 |
| Sarcoma of Ewing. | 100 | 100 | 46±4 | 47±6 |
| Melanoma | 100 | 100 | 55±5 | 44±4 |
| Neuroblastoma | 100 | 100 | 46±3 | 56±4 |
| Leukemia B | 100 | 100 | 47±4 | 53±5 |
| Leukemia T | 100 | 100 | 57±4 | 56±4 |
| Non-Hodgkin's Lymphoma B | 100 | 100 | 65±4 | 43±5 |
| Non-Hodgkin's Lymphoma T | 100 | 100 | 48±6 | 81±5 |
| Hodgkin's Lymphoma | 100 | 100 | 47±5 | 76±5 |
| Myeloma | 100 | 100 | 56±7 | 66±3 |

**Table 41. Percent inhibition/survival in reference to control for co-cultures of tumor cells-endothelial cells in the presence of SP (1 µM) and non-peptide NK1 receptor antagonist, Casopitant (1 µM).**

| **Type of tumor** | **Endothelial cells (control)** | **Tumor cells (control)** | **Endothelial cells co-cultured with tumor cells+SP+ Casopitant** | **Tumor cells co-cultured with endothelial cells+SP+ Casopitant** |
|---|---|---|---|---|
| Carcinoma of stomach. | 100 | 100 | 61±2 | 55±4 |
| Carcinoma of Colon. | 100 | 100 | 53±4 | 66±6 |
| Carcinoma of Pancreas. | 100 | 100 | 54±5 | 48±5 |
| Renal carcinoma | 100 | 100 | 55±2 | 57±4 |
| Carcinoma of breast. | 100 | 100 | 54±5 | 56±5 |
| Carcinoma of ovarian. | 100 | 100 | 46±4 | 58±6 |
| Carcinoma of endometrial. | 100 | 100 | 57±6 | 44±6 |
| Carcinoma of cervix. | 100 | 100 | 51±5 | 46±4 |
| Carcinoma of lung | 100 | 100 | 47±5 | 68±4 |
| Carcinoma of lung | 100 | 100 | 51±2 | 67±5 |
| Carcinoma of thyroid. | 100 | 100 | 47±3 | 46±4 |
| Carcinoma of thyroid. | 100 | 100 | 56±4 | 58±5 |
| Carcinoma of prostate | 100 | 100 | 57±5 | 49±3 |
| Glioma | 100 | 100 | 56±4 | 51±3 |
| Fibrosarcoma | 100 | 100 | 47±8 | 53±4 |
| Malignant fibrous histiocytoma | 100 | 100 | 56±5 | 47±3 |
| Sarcoma of Ewing. | 100 | 100 | 49±5 | 49±3 |
| Melanoma | 100 | 100 | 56±4 | 48±2 |
| Neuroblastoma | 100 | 100 | 47±2 | 57±3 |
| Leukemia B | 100 | 100 | 46±3 | 54±5 |
| Leukemia T | 100 | 100 | 56±7 | 54±6 |
| Non-Hodgkin's Lymphoma B | 100 | 100 | 64±5 | 49±7 |
| Non-Hodgkin's Lymphoma T | 100 | 100 | 47±8 | 86±4 |
| Hodgkin's Lymphoma | 100 | 100 | 45±4 | 77±4 |
| Myeloma | 100 | 100 | 55±5 | 67±3 |

Tables 38 to 41 show that the interaction between human primary tumor cells obtained directly from human tumors and human vascular endothelial cells promotes the survival of the same; on the other hand, the NK1 receptor antagonists nullify it. Since angiogenesis is essential for the development and maintenance of tumors, the use of non-peptide NK1 antagonists can inhibit angiogenesis avoiding such extension and tumor growth.

### Example 8. The interaction of primary tumor cells, obtained directly from human stromal cell-to-human fibroblasts, obtained from the same patient, promotes the survival of both types of cells and treatment with non-peptide NK1 receptor antagonist inhibits said survival.

To check that the interaction of the human primary tumor cells with stromal cells, fibroblasts, human, stimulates the survival of both cell types were carried stromal cell co-cultures, with human fibroblasts or tumor cells obtained directly from primary human tumors. Individual tumors and patient characteristics are shown in Table 36.

The same method described in Example 7 was used to obtain tumor cells from primary human tumors. Similarly, stromal cells were obtained - fibroblasts, which were cultured, from skin samples obtained from the same patient in the area of surgical incision that was made during the procedure performed to remove their tumor. Similarly to that conducted in Example 7, a total of 6 wells containing tumor cells were used as control. Survival of these other six wells containing tumor cells were cultured with the addition of the stromal cells, fibroblasts and another 6 wells containing tumor cells were cultured in the presence of stromal cells, fibroblasts, and NK1 receptor agonist (SP), another group of six wells containing tumor cells cultured in the presence of stromal cells, fibroblasts, human NK1 receptor agonist (SP) and each of the non-peptide receptor antagonists NK1 analyzed: Aprepitant, and Casopitant Vestipitant.

As in Example 7, it was found that both fibroblast and tumor cells were expressing NK1 receptor by Western blotting and using the antibodies described in Example 7.

Tables 42 to 46 detail the percentage inhibition/survival in reference to control for co-cultures of tumor cells, stromal fibroblast cells (Table 42), tumor cells, stromal cells with a fibroblast receptor agonist exposure NK1 (SP ) (Table 43), tumor cells, stromal fibroblast cells with receptor antagonist exposure NK1 (SP) and each of NK1 receptor antagonists: Aprepitant, Vestipitant and Casopitant (Tables 44-46). We obtained the same results when other non-peptide receptor antagonists NK1 were used: L-733, 060, L-732, 138, L-703.606, CP-100263, WIN 62,577, WIN 51708, CP-96345 and L-760 735. The results shown in these tables demonstrate the interaction of primary human tumor cells and stromal cells, fibroblasts, human, stimulates the survival of the same and that NK1 receptor agonist (SP) enhances this phenomenon, but the non-peptide receptor antagonists NK1 inhibit such proliferation.

**Table 42. Percent inhibition/survival in reference to the control of co-cultures of tumor cells-fibroblast stromal cells.**

| **Type of tumor** | **Fibroblast cells (control)** | **Tumor cells (control)** | **Fibroblast cells co-cultured with tumor cells** | **Tumor cells co-cultured with fibroblast cells** |
|---|---|---|---|---|
| Carcinoma of stomach. | 100 | 100 | 115±4 | 145±4 |
| Carcinoma of Colon. | 100 | 100 | 114±3 | 144±5 |
| Carcinoma of Pancreas. | 100 | 100 | 117±3 | 132±2 |
| Renal carcinoma | 100 | 100 | 132±3 | 143±4 |
| Carcinoma of breast. | 100 | 100 | 121±4 | 144±3 |
| Carcinoma of ovarian. | 100 | 100 | 122±3 | 152±4 |
| Carcinoma of endometrial. | 100 | 100 | 126±2 | 143±5 |
| Carcinoma of cervix. | 100 | 100 | 124±5 | 145±4 |
| Carcinoma of lung | 100 | 100 | 124±4 | 146±5 |
| Carcinoma of lung | 100 | 100 | 126±5 | 142±5 |
| Carcinoma of thyroid. | 100 | 100 | 125±4 | 143±4 |
| Carcinoma of thyroid. | 100 | 100 | 127±4 | 142±6 |
| Carcinoma of prostate | 100 | 100 | 126±5 | 141±5 |
| Glioma | 100 | 100 | 124±4 | 151±4 |
| Malignant fibrous histiocytom | 100 | 100 | 125±6 | 152±5 |
| Sarcoma of Ewing. | 100 | 100 | 134±5 | 153±4 |
| Melanoma | 100 | 100 | 134±3 | 155±6 |
| Neuroblastoma | 100 | 100 | 124±6 | 154±7 |
| Leukemia B | 100 | 100 | 123±4 | 145±5 |
| Leukemia T | 100 | 100 | 135±6 | 156±7 |
| Non-Hodgkin's Lymphoma B | 100 | 100 | 126±3 | 147±4 |
| Non-Hodgkin's Lymphoma T | 100 | 100 | 123±5 | 151±3 |
| Myeloma | 100 | 100 | 124±5 | 152±5 |

**Table 43. Percent inhibition/survival in reference to the control of co-cultures of tumor cells-fibroblast stromal cells in the presence of SP (1 µM).**

| **Type of tumor** | **Fibroblast cells (control)** | **Tumor cells (control)** | **Fibroblast cells co-cultured with tumor cells +SP** | **Tumor cells co-cultured with fibroblast cells + SP** |
|---|---|---|---|---|
| Carcinoma of stomach. | 100 | 100 | 117±3 | 181±4 |
| Carcinoma of Colon. | 100 | 100 | 114±3 | 198±5 |
| Carcinoma of Pancreas. | 100 | 100 | 117±3 | 189±2 |
| Renal carcinoma | 100 | 100 | 132±3 | 188±4 |
| Carcinoma of breast. | 100 | 100 | 121±4 | 193±3 |
| Carcinoma of ovarian. | 100 | 100 | 122±3 | 197±4 |
| Carcinoma of endometrial. | 100 | 100 | 126±2 | 193±5 |
| Carcinoma of cervix. | 100 | 100 | 124±5 | 196±4 |
| Carcinoma of lung | 100 | 100 | 124±4 | 193±5 |
| Carcinoma of lung | 100 | 100 | 126±5 | 194±5 |
| Carcinoma of thyroid. | 100 | 100 | 125±4 | 196±4 |
| Carcinoma of thyroid. | 100 | 100 | 127±4 | 197±6 |
| Carcinoma of prostate | 100 | 100 | 126±5 | 198±5 |
| Glioma | 100 | 100 | 124±4 | 197±4 |
| Malignant fibrous histiocytoma | 100 | 100 | 125±6 | 196±5 |
| Sarcoma of Ewing. | 100 | 100 | 134±5 | 199±4 |
| Melanoma | 100 | 100 | 134±3 | 189±6 |
| Neuroblastoma | 100 | 100 | 124±6 | 184±7 |
| Leukemia B | 100 | 100 | 123±4 | 198±5 |
| Leukemia T | 100 | 100 | 135±6 | 189±7 |
| Non-Hodgkin's Lymphoma B | 100 | 100 | 126±3 | 190±4 |
| Non-Hodgkin's Lymphoma T | 100 | 100 | 123±5 | 197±3 |
| Myeloma | 100 | 100 | 124±5 | 195±5 |

**Table 44. Percent inhibition/survival in reference to the control of co-cultures of tumor cells-fibroblast stromal cells in the presence of SP (1 µM) and non-peptide antagonist of the NK1 receptor, Aprepitant (1µM).**

| **Type of tumor** | **Endothelial cells (control)** | **Tumor cells (control)** | **Endothelial cells co-cultured with tumor cells + SP+Aprepitant** | **Tumor cells co-cultured with endothelial cells +SP+ Aprepitant** |
|---|---|---|---|---|
| Carcinoma of stomach. | 100 | 100 | 85±1 | 44±3 |
| Carcinoma of Colon. | 100 | 100 | 87±2 | 41±2 |
| Carcinoma of Pancreas. | 100 | 100 | 85±3 | 36±5 |
| Renal carcinoma | 100 | 100 | 86±4 | 43±2 |
| Carcinoma of breast. | 100 | 100 | 86±5 | 49±2 |
| Carcinoma of ovarian. | 100 | 100 | 89±4 | 45±6 |
| Carcinoma of endometrial. | 100 | 100 | 87±3 | 44±6 |
| Carcinoma of cervix. | 100 | 100 | 85±5 | 47±2 |
| Carcinoma of lung | 100 | 100 | 86±8 | 45±5 |
| Carcinoma of lung | 100 | 100 | 83±6 | 44±4 |
| Carcinoma of thyroid. | 100 | 100 | 87±3 | 45±3 |
| Carcinoma of thyroid. | 100 | 100 | 84±5 | 51±5 |
| Carcinoma of prostate | 100 | 100 | 85±6 | 49±4 |
| Glioma | 100 | 100 | 86±6 | 49±4 |
| Malignant fibrous histiocytoma | 100 | 100 | 87±6 | 44±5 |
| Sarcoma of Ewing. | 100 | 100 | 86±5 | 45±7 |
| Melanoma | 100 | 100 | 88±6 | 57±3 |
| Neuroblastoma | 100 | 100 | 87±4 | 58±4 |
| Leukemia B | 100 | 100 | 86±6 | 57±6 |
| Leukemia T | 100 | 100 | 88±4 | 51±7 |
| Non-Hodgkin's Lymphoma B | 100 | 100 | 89±5 | 71±6 |
| Non-Hodgkin's Lymphoma T | 100 | 100 | 86±5 | 79±5 |
| Myeloma | 100 | 100 | 88±6 | 84±6 |

**Table 45. Percent inhibition/survival in reference to the control of co-cultures of tumor cells-fibroblast stromal cells in the presence of SP (1 µM) and non-peptide antagonist of the NK1 receptor, Vestipitant (1µM).**

| **Type of tumor** | **Endothelial cells (control)** | **Tumor cells (control)** | **Endothelial cells co-cultured with tumor cells +SP+ Vestipitant** | **Tumor cells co-cultured with endothelial cells +SP+ Vestipitant** |
|---|---|---|---|---|
| Carcinoma of stomach. | 100 | 100 | 85±2 | 54±3 |
| Carcinoma of Colon. | 100 | 100 | 87±3 | 62±2 |
| Carcinoma of Pancreas. | 100 | 100 | 88±5 | 44±6 |
| Renal carcinoma | 100 | 100 | 88±6 | 53±5 |
| Carcinoma of breast. | 100 | 100 | 89±3 | 54±6 |
| Carcinoma of ovarian. | 100 | 100 | 84±5 | 55±5 |
| Carcinoma of endometrial. | 100 | 100 | 86±6 | 46±4 |
| Carcinoma of cervix. | 100 | 100 | 87±5 | 46±7 |
| Carcinoma of lung | 100 | 100 | 85±7 | 69±4 |
| Carcinoma of lung | 100 | 100 | 85±6 | 68±5 |
| Carcinoma of thyroid. | 100 | 100 | 86±5 | 49±4 |
| Carcinoma of thyroid. | 100 | 100 | 82±6 | 51±5 |
| Carcinoma of prostate | 100 | 100 | 81±5 | 49±5 |
| Glioma | 100 | 100 | 84±6 | 46±4 |
| Malignant fibrous histiocytoma | 100 | 100 | 86±5 | 45±5 |
| Sarcoma of Ewing. | 100 | 100 | 85±5 | 46±6 |
| Melanoma | 100 | 100 | 86±3 | 55±6 |
| Neuroblastoma | 100 | 100 | 87±4 | 54±4 |
| Leukemia B | 100 | 100 | 87±5 | 55±6 |
| Leukemia T | 100 | 100 | 86±6 | 42±3 |
| Non-Hodgkin's Lymphoma B | 100 | 100 | 91±5 | 46±4 |
| Non-Hodgkin's Lymphoma T | 100 | 100 | 92±4 | 76±4 |
| Myeloma | 100 | 100 | 91±6 | 65±3 |

**Table 46. Percent inhibition/survival in reference to the control of co-cultures of tumor cells-fibroblast stromal cells in the presence of SP (1 µM) and non-peptide antagonist of the NK1 receptor, Casopitant (1µM).**

| **Type of tumor** | **Endothelial cells (control)** | **Tumor cells (control)** | **Endothelial cells co-cultured with tumor cells + SP+Casopitant** | **Tumor cells co-cultured with endothelial cells+SP+ Casopitant** |
|---|---|---|---|---|
| Carcinoma of stomach. | 100 | 100 | 91±3 | 51±3 |
| Carcinoma of Colon. | 100 | 100 | 83±4 | 62±3 |
| Carcinoma of Pancreas. | 100 | 100 | 85±3 | 49±4 |
| Renal carcinoma | 100 | 100 | 86±4 | 56±5 |
| Carcinoma of breast. | 100 | 100 | 84±5 | 57±7 |
| Carcinoma of ovarian. | 100 | 100 | 91±4 | 56±8 |
| Carcinoma of endometrial. | 100 | 100 | 94±5 | 48±7 |
| Carcinoma of cervix. | 100 | 100 | 83±4 | 47±5 |
| Carcinoma of lung | 100 | 100 | 78±4 | 67±5 |
| Carcinoma of lung | 100 | 100 | 76±3 | 66±4 |
| Carcinoma of thyroid. | 100 | 100 | 75±4 | 41±3 |
| Carcinoma of thyroid. | 100 | 100 | 75±5 | 56±4 |
| Carcinoma of prostate | 100 | 100 | 57±7 | 47±2 |
| Glioma | 100 | 100 | 51±6 | 50±2 |
| Malignant fibrous histiocytoma | 100 | 100 | 52±4 | 47±2 |
| Sarcoma of Ewing. | 100 | 100 | 52±3 | 46±3 |
| Melanoma | 100 | 100 | 53±1 | 56±2 |
| Neuroblastoma | 100 | 100 | 52±2 | 53±4 |
| Leukemia B | 100 | 100 | 51±5 | 52±5 |
| Leukemia T | 100 | 100 | 54±4 | 48±6 |
| Non-Hodgkin's Lymphoma B | 100 | 100 | 61±7 | 44±3 |
| Non-Hodgkin's Lymphoma T | 100 | 100 | 60±3 | 75±3 |
| Myeloma | 100 | 100 | 57±4 | 65±4 |

### Example 9. The interaction of primary tumor cells, obtained directly from human cells with immune/inflammatory (mono-and polymorphonuclear leukocytes) obtained from the same patient, promote the survival of both types of cells and treatment with non-peptide receptor NK1 inhibits said survival.

To check that the interaction between the human primary tumor cells with human immune/inflammatory (polymorphonuclear and mononuclear leukocytes) cells, promotes the survival of both types of cells, cultures were carried out with sets of immune/inflammatory cells (mono and polymorphonuclear leukocytes) with tumor cells obtained directly from human primary tumors of patients. Individual tumors and patient characteristics are shown in Table 36.

The same method described in Example 7 was used to obtain tumor cells from primary human tumors. The leukocytes were obtained from centrifugation of blood from the same patient. The leukocytes were cultured in fresh plasma from the same patient, in order to build a model similar to human physiology. Similarly to what was done in the above example, a total of six wells were used to cultivate the inflammatory cells (mono and polymorphonuclear leukocytes) as a control, a total of 6 wells containing tumor cells were used as control survival of these tumor cells, another 6 wells containing tumor cells were cultured with addition of immune cells/inflammatory (mono and polymorphonuclear leukocytes), another 6 wells containing tumor cells were cultured in the presence of immune cells/inflammatory cells (mono and polymorphonuclear leukocytes) and the NK1 receptor agonist (SP), other groups of six wells containing tumor cells were cultured in the presence of immune cells/inflammatory (mono and polymorphonuclear leukocytes), NK1 receptor agonist (SP) and each one of the non-peptide antagonists of the NK1 receptor analyzed: Aprepitant, Vestipitant and Casopitant.

[As in Example 7, it was found that both the immune cells/inflammatory (mono and polymorphonuclear leukocytes), such as tumor cells expressed NK1 receptor by Western blotting and using the antibodies described in Example 7].

Tables 47 to 51 detail the percentage inhibition/survival in reference to control for co-cultures of tumor cells, immune system cells - mono or polymorphonuclear leukocyte(Table 47), tumor cells, immune system cells - mono or polymorphonuclear leukocyte (Table 48), tumor cells, immune system cells - mono or polymorphonuclear leukocyte and each of the antagonists NK1 receptor: Aprepitant, Vestipitant and Casopitant (Tables 49-51). We obtained the same results when other non-peptide NK1 receptor was used: L-733,060, L-732,138, L-703,606, CP-100263, WIN 62,577, WIN 51708, CP-96345 and L-760 735. The results shown in these tables demonstrate the interaction of primary human tumor cells and immune system cells mono or polymorphonuclear leucocytes, promotes the survival of the same and that the NK1 receptor agonist (SP) enhances this phenomenon, but that non-peptide NK1 receptor antagonists inhibit such proliferation.

**Table 47. Percent inhibition/survival in reference to the control of co-cultures of tumor cells- immune/inflammatory cells (leukocytes)**

| **Type of tumor** | **Leukocytes (control)** | **Tumor cells (control)** | **Leukocytes co-cultured with tumor cells** | **Tumor cells co-cultured with leukocytes** |
|---|---|---|---|---|
| Carcinoma of stomach. | 100 | 100 | 125±4 | 185±5 |
| Carcinoma of Colon. | 100 | 100 | 124±2 | 184±6 |
| Carcinoma of Pancreas. | 100 | 100 | 116±4 | 192±3 |
| Renal carcinoma | 100 | 100 | 122±2 | 193±5 |
| Carcinoma of breast. | 100 | 100 | 123±3 | 194±4 |
| Carcinoma of ovarian. | 100 | 100 | 123±4 | 192±5 |
| Carcinoma of endometrial. | 100 | 100 | 124±4 | 193±6 |
| Carcinoma of cervix. | 100 | 100 | 125±4 | 195±7 |
| Carcinoma of lung | 100 | 100 | 125±3 | 196±6 |
| Carcinoma of lung | 100 | 100 | 124±5 | 192±7 |
| Carcinoma of thyroid. | 100 | 100 | 126±3 | 193±5 |
| Carcinoma of thyroid. | 100 | 100 | 125±5 | 192±7 |
| Carcinoma of prostate | 100 | 100 | 124±6 | 191±6 |
| Glioma | 100 | 100 | 125±5 | 188±6 |
| Sarcoma of Ewing. | 100 | 100 | 126±7 | 189±4 |
| Melanoma | 100 | 100 | 135±6 | 189±6 |
| Neuroblastoma | 100 | 100 | 136±7 | 189±5 |
| Myeloma | 100 | 100 | 125±6 | 189±6 |
| Carcinoma of breast. | 100 | 100 | 123±3 | 194±4 |

**Table 48. Percent inhibition/survival in reference to the control of co-cultures of tumor cells- immune/inflammatory cells (leukocytes) in the presence of SP (1 µM).**

| **Type of tumor** | **Leukocytes (control)** | **Tumor cells (control)** | **Leukocytes co-cultured with tumor cells + SP** | **Tumor cells co-cultured with leukocytes+SP** |
|---|---|---|---|---|
| Carcinoma of stomach. | 100 | 100 | 118±3 | 186±4 |
| Carcinoma of Colon. | 100 | 100 | 117±3 | 187±5 |
| Carcinoma of Pancreas. | 100 | 100 | 116±5 | 188±2 |
| Renal carcinoma | 100 | 100 | 122±4 | 189±4 |
| Carcinoma of breast. | 100 | 100 | 130±5 | 192±3 |
| Carcinoma of ovarian. | 100 | 100 | 131±3 | 195±4 |
| Carcinoma of endometrial. | 100 | 100 | 130±3 | 195±6 |
| Carcinoma of cervix. | 100 | 100 | 127±5 | 195±4 |
| Carcinoma of lung | 100 | 100 | 124±4 | 197±7 |
| Carcinoma of lung | 100 | 100 | 127±4 | 191±6 |
| Carcinoma of thyroid. | 100 | 100 | 119±4 | 191±4 |
| Carcinoma of thyroid. | 100 | 100 | 119±5 | 192±5 |
| Carcinoma of prostate | 100 | 100 | 121±4 | 192±5 |
| Glioma | 100 | 100 | 125±6 | 195±3 |
| Malignant fibrous histiocytoma | 100 | 100 | 121±4 | 197±6 |
| Sarcoma of Ewing. | 100 | 100 | 130±4 | 196±5 |
| Melanoma | 100 | 100 | 131±5 | 192±4 |
| Neuroblastoma | 100 | 100 | 127±5 | 189±5 |
| Leukemia B | 100 | 100 | 127±3 | 197±6 |
| Leukemia T | 100 | 100 | 130±5 | 188±5 |
| Non-Hodgkin's Lymphoma B | 100 | 100 | 125±4 | 198±9 |
| Non-Hodgkin's Lymphoma T | 100 | 100 | 127±6 | 187±5 |
| Myeloma | 100 | 100 | 125±4 | 196±6 |

**Table 49. Percent inhibition/survival in reference to the control of co-cultures of tumor cells- immune/inflammatory cells (leukocytes) in the presence of SP (1 µM) and non-peptide antagonist of the NK1 receptor, Aprepitant 1 µM).**

| **Type of tumor** | **Leukocytes (control)** | **Tumor cells (control)** | **Leukocytes co-cultured with tumor cells+SP+ Aprepitant** | **Tumor cells co-cultured with leukocytes+ SP+Aprepitant** |
|---|---|---|---|---|
| Carcinoma of stomach. | 100 | 100 | 89±6 | 34±5 |
| Carcinoma of Colon. | 100 | 100 | 89±6 | 31±6 |
| Carcinoma of Pancreas | 100 | 100 | 89±6 | 35±6 |
| Renal carcinoma | 100 | 100 | 91±5 | 41±7 |
| Carcinoma of breast. | 100 | 100 | 92±5 | 47±2 |
| Carcinoma of ovarian. | 100 | 100 | 90±4 | 40±7 |
| Carcinoma of endometrial. | 100 | 100 | 93±3 | 42±5 |
| Carcinoma of cervix. | 100 | 100 | 94±4 | 43±6 |
| Carcinoma of lung | 100 | 100 | 95±6 | 45±7 |
| Carcinoma of lung | 100 | 100 | 86±6 | 42±6 |
| Carcinoma of thyroid. | 100 | 100 | 85±3 | 46±7 |
| Carcinoma of thyroid. | 100 | 100 | 87±5 | 52±6 |
| Carcinoma of prostate | 100 | 100 | 80±5 | 41±6 |
| Glioma | 100 | 100 | 78±6 | 45±5 |
| Sarcoma of Ewing. | 100 | 100 | 75±5 | 40±6 |
| Melanoma | 100 | 100 | 80±4 | 43±5 |
| Neuroblastoma | 100 | 100 | 81±5 | 51±5 |
| Myeloma | 100 | 100 | 87±5 | 64±7 |

**Table 50. Percent inhibition/survival in reference to the control of co-cultures of tumor cells- immune/inflammatory cells (leukocytes) in the presence of SP (1 µM) and non-peptide antagonist of the NK1 receptor, Vestipitant (1µM).**

| **Type of tumor** | **Leukocytes (control)** | **Tumor cells (control)** | **Leukocytes co-cultured with tumor cells+SP+ Vestipitant** | **Tumor cells co-cultured with leukocytes+ SP+Vestipitant** |
|---|---|---|---|---|
| Carcinoma of stomach. | 100 | 100 | 84±7 | 51±7 |
| Carcinoma of Colon. | 100 | 100 | 85±6 | 60±5 |
| Carcinoma of Pancreas. | 100 | 100 | 85±6 | 46±6 |
| Renal carcinoma | 100 | 100 | 86±5 | 58±4 |
| Carcinoma of breast. | 100 | 100 | 87±6 | 56±7 |
| Carcinoma of ovarian. | 100 | 100 | 88±7 | 58±6 |
| Carcinoma of endometrial. | 100 | 100 | 83±5 | 48±7 |
| Carcinoma of cervix. | 100 | 100 | 85±6 | 49±5 |
| Carcinoma of lung | 100 | 100 | 87±6 | 61±3 |
| Carcinoma of lung | 100 | 100 | 89±7 | 68±6 |
| Carcinoma of thyroid. | 100 | 100 | 89±8 | 51±7 |
| Carcinoma of thyroid. | 100 | 100 | 89±7 | 50±6 |
| Carcinoma of prostate | 100 | 100 | 89±6 | 51±6 |
| Glioma | 100 | 100 | 88±4 | 52±7 |
| Sarcoma of Ewing. | 100 | 100 | 89±7 | 53±6 |
| Melanoma | 100 | 100 | 89±6 | 52±6 |
| Neuroblastoma | 100 | 100 | 88±7 | 53±7 |
| Myeloma | 100 | 100 | 90±5 | 58±8 |

**Table 51. Percent inhibition/survival in reference to the control of co-cultures of tumor cells- immune/inflammatory cells (leukocytes) in the presence of SP (1 µM) and non-peptide antagonist of the NK1 receptor, Casopitant (1µM).**

| **Type of tumor** | **Leukocytes (control)** | **Tumor cells (control)** | **Leukocytes co-cultured with tumor cells+SP+ Casopitant** | **Tumor cells co-cultured with leukocytes+ SP+Casopitant** |
|---|---|---|---|---|
| Carcinoma of stomach. | 100 | 100 | 92±5 | 50±5 |
| Carcinoma of Colon. | 100 | 100 | 91±6 | 60±6 |
| Carcinoma of Pancreas. | 100 | 100 | 91±5 | 51±7 |
| Renal carcinoma | 100 | 100 | 89±6 | 51±6 |
| Carcinoma of breast. | 100 | 100 | 89±7 | 52±8 |
| Carcinoma of ovarian. | 100 | 100 | 90±8 | 54±7 |
| Carcinoma of endometrial. | 100 | 100 | 91±7 | 52±8 |
| Carcinoma of cervix. | 100 | 100 | 89±8 | 53±6 |
| Carcinoma of lung | 100 | 100 | 90±6 | 52±6 |
| Carcinoma of lung | 100 | 100 | 92±7 | 51±7 |
| Carcinoma of thyroid. | 100 | 100 | 93±6 | 61±6 |
| Carcinoma of thyroid. | 100 | 100 | 94±8 | 60±7 |
| Carcinoma of prostate | 100 | 100 | 91±6 | 58±6 |
| Glioma | 100 | 100 | 92±7 | 57±5 |
| Sarcoma of Ewing. | 100 | 100 | 93±5 | 49±5 |
| Melanoma | 100 | 100 | 91±7 | 49±4 |
| Neuroblastoma | 100 | 100 | 92±7 | 51±6 |
| Myeloma | 100 | 100 | 92±7 | 60±5 |

### Example 10. The interaction of primary tumor cells, obtained directly from human cells with immune/inflammatory (macrophages), obtained from the same patient, promote the survival of both types of cells and treatment with non-peptide NK1 receptors antagonists inhibits said survival.

To check that the interaction of the human primary tumor cells with cells of the immune/inflammatory (macrophages) cells promotes the survival of both cell types, co-cultures were performed on fresh blood plasma, immune cell / inflammatory diseases (macrophages) with tumor cells derived from primary tumors obtained from the same patient. All samples were obtained from the same patient, to build an experimental model similar to human physiology. Individual tumors and patient characteristics are shown in Table 36. The samples for the isolation of macrophages were taken from pleural or peritoneal fluid.

The same method described in Example 7 was used to obtain tumor cells from primary human tumors .Similarly to what was done in the above example, a total of six wells were used to cultivate the inflammatory cells (macrophages) as a control, a total of 6 wells containing tumor cells were used as control survival of these tumor cells, another 6 wells containing tumor cells were cultured with addition of immune cells/inflammatory (macrophages), another 6 wells containing tumor cells were cultured in the presence of immune cells/inflammatory (macrophages) and the NK1 receptor agonist (SP), other groups of six wells containing tumor cells were cultured in the presence of immune cells/inflammatory (mono and polymorphonuclear leukocytes), NK1 receptor agonist (SP) and each of the non-peptide antagonists of the NK1 receptor analyzed: Aprepitant, Vestipitant and Casopitant.

As in Example 7, it was found that both the immune cells/inflammatory (macrophage), such as tumor cells which expressed NK1 receptor by Western blotting and using the antibodies specific to immune cells/inflammatory (macrophages) (Anti-CD68) carcinoma cells (Anti-cytokeratin spectrum), Melanoma (Anti-HMB45). All antibodies are distributed by Dako and used at the concentration at which they are supplied (supplied pre-diluted -"ready to use").

Tables 52 to 56 detail the percentage inhibition/survival in reference to control for co-cultures of tumor cells, immune system cells - inflammatory (macrophages)-(Table 52), tumor cells- immune system cells - inflammatory (macrophages)- with exposure NK1 receptor agonist (SP) (Table 53), tumor cells- immune system cells - inflammatory (macrophage) -, with exposure to the NK1 receptor antagonist (SP) and each of the antagonists NK1 receptor: Aprepitant, Vestipitant and Casopitant (Tables 54-56). We obtained the same results when other nonpeptide NK1 receptor was used: L-733, 060, L-732, 138, L-703.606, CP-100263, WIN 62,577, WIN 51708, CP-96345 and L-760 735. The results shown in these tables demonstrate the interaction of primary human tumor cells and immune system cells - inflammatory (macrophages), promotes the survival of the same and that the NK1 receptor agonist (SP) power said phenomenon, but that nonpeptide NK1 receptors inhibit such proliferation.

**Table 52. Percent inhibition/survival in reference to the control of co-cultures of tumor cells- immune/inflammatory cells (macrophages).**

| **Type of tumor** | **Macrophages (control)** | **Tumor cells (control)** | **Macrophages co-cultured with tumor cells** | **Tumor cells co-cultured with macrophages** |
|---|---|---|---|---|
| Carcinoma of stomach | 100 | 100 | 128±6 | 189±8 |
| Carcinoma of Colon. | 100 | 100 | 127±7 | 188±7 |
| Carcinoma of breast | 100 | 100 | 129±5 | 191±8 |
| Carcinoma of ovarian. | 100 | 100 | 125±5 | 190±8 |
| Carcinoma of endometrial | 100 | 100 | 128±7 | 197±7 |
| Carcinoma of lung | 100 | 100 | 124±6 | 194±8 |
| Carcinoma of lung | 100 | 100 | 128±7 | 192±8 |
| Melanoma | 100 | 100 | 137±5 | 184±9 |

**Table 53. Percent inhibition/survival in reference to the control of co-cultures of tumor cells- immune/inflammatory cells in the presence of SP (1 µM).**

| **Type of tumor** | **Macrophages (control)** | **Tumor cells (control)** | **Macrophages co-cultured with tumor cells+SP** | **Tumor cells co-cultured with macrophages +SP** |
|---|---|---|---|---|
| Carcinoma of stomach | 100 | 100 | 121±7 | 191±7 |
| Carcinoma of Colon. | 100 | 100 | 124±6 | 186±6 |
| Carcinoma of breast | 100 | 100 | 127±6 | 193±5 |
| Carcinoma of ovarian. | 100 | 100 | 130±6 | 198±7 |
| Carcinoma of endometrial | 100 | 100 | 128±7 | 190±7 |
| Carcinoma of cervix | 100 | 100 | 131±7 | 193±8 |
| Carcinoma of lung | 100 | 100 | 129±5 | 192±6 |
| Carcinoma of lung | 100 | 100 | 128±6 | 196±7 |
| Melanoma | 100 | 100 | 134±6 | 195±7 |

**Table 54. Percent inhibition/survival in reference to the control of co-cultures of tumor cells- immune/inflammatory cells in the presence of SP (1 µM) and non-peptide antagonist of the NK1 receptor, Aprepitant (1 µM).**

| **Type of tumor** | **Macrophages (control)** | **Tumor cells (control)** | **Macrophages co-cultured with tumor cells+SP+ Aprepiant** | **Tumor cells co-cultured with macrophages+ SP+Aprepiant** |
|---|---|---|---|---|
| Carcinoma of stomach. | 100 | 100 | 91±5 | 39±6 |
| Carcinoma of Colon. | 100 | 100 | 89±5 | 37±7 |
| Carcinoma of Pancreas. | 100 | 100 | 88±7 | 38±7 |
| Renal carcinoma | 100 | 100 | 90±6 | 40±6 |
| Carcinoma of breast. | 100 | 100 | 90±6 | 40±5 |
| Carcinoma of ovarian. | 100 | 100 | 94±5 | 42±6 |
| Carcinoma of endometrial. | 100 | 100 | 96±6 | 48±7 |
| Carcinoma of cervix. | 100 | 100 | 95±5 | 45±6 |
| Carcinoma of lung | 100 | 100 | 96±5 | 47±6 |
| Carcinoma of lung | 100 | 100 | 89±7 | 48±7 |
| Carcinoma of thyroid. | 100 | 100 | 89±5 | 47±6 |
| Carcinoma of thyroid. | 100 | 100 | 91±6 | 50±7 |
| Carcinoma of prostate | 100 | 100 | 89±5 | 51±7 |
| Glioma | 100 | 100 | 88±5 | 52±6 |
| Sarcoma of Ewing. | 100 | 100 | 81±6 | 51±7 |
| Melanoma | 100 | 100 | 86±6 | 52±6 |
| Neuroblastoma | 100 | 100 | 87±7 | 56±6 |
| Myeloma | 100 | 100 | 86±6 | 54±7 |

**Table 55. Percent inhibition/survival in reference to the control of co-cultures of tumor cells- immune/inflammatory cells in the presence of SP (1 µM) and non-peptide antagonist of the NK1 receptor, Vestipitant (1µM).**

| **Type of tumor** | **Macrophages (control)** | **Tumor cells (control)** | **Macrophages co-cultured with tumor cells+SP+ Vestipitant** | **Tumor cells co-cultured with macrophages+ SP+Vestipitant** |
|---|---|---|---|---|
| Carcinoma of stomach. | 100 | 100 | 86±6 | 49±6 |
| Carcinoma of Colon. | 100 | 100 | 87±7 | 54±4 |
| Carcinoma of breast | 100 | 100 | 85±6 | 48±5 |
| Carcinoma of ovarian. | 100 | 100 | 86±5 | 51±7 |
| Carcinoma of endometrial. | 100 | 100 | 87±8 | 49±5 |
| Carcinoma of lung | 100 | 100 | 91±7 | 56±6 |
| Carcinoma of lung | 100 | 100 | 92±6 | 57±7 |
| Melanoma | 100 | 100 | 90±7 | 58±7 |

**Table 56. Percent inhibition/survival in reference to the control of co-cultures of tumor cells- immune/inflammatory cells in the presence of SP (1 µM) and non-peptide antagonist of the NK1 receptor, Casopitant (1µM).**

| **Type of tumor** | **Macrophages (control)** | **Tumor cells (control)** | **Macrophages co-cultured with tumor cells+SP+ Casopitant** | **Tumor cells co-cultured with macrophages+ SP+Casopitant** |
|---|---|---|---|---|
| Carcinoma of stomach | 100 | 100 | 95±6 | 55±7 |
| Carcinoma of Colon. | 100 | 100 | 94±5 | 58±5 |
| Carcinoma of breast | 100 | 100 | 91±6 | 59±6 |
| Carcinoma of ovarian. | 100 | 100 | 92±5 | 57±6 |
| Carcinoma of endometrial. | 100 | 100 | 90±6 | 58±7 |
| Carcinoma of lung | 100 | 100 | 90±7 | 56±7 |
| Carcinoma of lung | 100 | 100 | 89±6 | 58±8 |
| Melanoma | 100 | 100 | 88±8 | 51±6 |

### Example 11. The interaction of human tumor cells with the stromal cells, human fibroblasts, stimulates the secretion of substances of importance for survival and tumor progression and the treatment with non-peptide NK1 receptors antagonist inhibit said secretion.

To check that the interaction of the human primary tumor cells with stromal cells, [human fibroblasts,] stimulates the secretion of substances of importance for survival and tumor progression, co-cultures were performed using stromal cells with human fibroblast- tumor cells obtained directly from primary human tumors. Individual tumors and patient characteristics are shown in Table 36.

The same method described in Example 7 was used to obtain tumor cells from primary human tumors. A control group containing exclusively fibroblasts in culture was used as expression control of the substances therein, another group containing tumor cells cultured with the addition of the stromal cells-fibroblast, a group of other tumor cells were cultured in the presence of stromal cells, fibroblasts, and the NK1 receptor agonist (SP) and other groups containing tumor cells were cultured in the presence of stromal cells - fibroblasts or human NK1 receptor agonist (SP) and each of the nonpeptide NK1 receptor: Aprepitant, Vestipitant and Casopitant.

Previously, it was found that the tumor cells and stromal cells, human fibroblasts, expressed NK1 receptor by the technique of Western blotting. Later, paraffin blocks were prepared, for subsequent immunohistochemical assays. On this occasion, in order to identify, using immunohistochemical techniques, the secretion of substances of importance for the survival and progression of tumors, the following antibodies were used: TGF-α (SAB4502953), TGF-β 1 (SAB4502954), TGF-β 2 (SAB4502956), TGF-β 3 (SAB4502957), SPARC (HPA002989), MMP-3 (HPA007875), MMP-7 (SAB4501894), MMP-9 (SAB4501896), MMP-11 (SAB4501898) MMP-13 (SAB4501900) and MMP-14 (SAB4501901) using specific antibodies against them. All antibodies used were rabbit polyclonal antibodies obtained from Sigma-Aldrich and were used at a concentration of 1/1000 to achieve these immunohistochemical studies. All experiments were carried out sixfold.

Tables 57 to 61 details the percentage of cells with an expression for each of the markers (substances important for the tumor microenvironment) for co-cultures of tumor cells-stromal cells, fibroblast, tumor cells- fibroblast stromal cells with NK1 receptor agonist (SP) exposure, tumor and fibroblast stromal cells, - with NK1 receptor agonist (SP) exposure and each of NK1 receptor antagonists: Aprepitant, Vestipitant and Casopitant. In the tables 57 to 61, five examples of co-cultures fibroblast stromal cells are shown with different tumor cells taken from tumors of the type: colon cancer, pancreatic cancer, lung cancer, glioma and myeloma. We obtained the same results when used other non-peptide NK1 receptor: L-733,060, L-732,138, L-703,606, CP-100263, WIN 62,577, WIN 51708, CP-96345 and L-760735.

The results shown in these tables demonstrate the interaction of primary human tumor cells and stromal cells, human fibroblasts, stimulates the expression of substances in the tumor microenvironment importance for survival and tumor progression and NK1 receptor agonist (SP) enhances this phenomenon, whereas treatment with the non-peptide NK1 receptors inhibit such expression.

**Table 57. Percentage of fibroblast cells which expressed the analyzed markers in co-culture with cells isolated from colon carcinoma and in the presence of SP (1 µM) and various non-peptide antagonists of the NK1 receptor to 1µM concentration.**

| **Marker** | **Fibroblast** | **Primary Tumor cells co-cultured + fibroblast** | **Primary Tumor cells co-cultured + fibroblast + SP** | **Primary Tumor cells co-cultured + fibroblast + SP + Aprepitant** | **Primary Tumor cells co-cultured + fibroblast + SP + Vestipitant** | **Primary Tumor cells co-cultured + fibroblast + SP + Casopitant** |
|---|---|---|---|---|---|---|
| TGFα | 0 | 14.2±3.2% | 45.5±4.2% | 0 | 0 | 0 |
| TGFβ 1 | 0 | 15.4±4.3% | 35.3±2.3% | 0 | 0 | 0 |
| TGFβ 2 | 0 | 23.6±5.3% | 44.5±3.6% | 0 | 0 | 0 |
| TGFβ 3 | 0 | 15.7±3.4% | 45±3.9% | 0 | 0 | 0 |
| SPARC | 0 | 16.6±4.5% | 47±2.7% | 0 | 0 | 0 |
| MMP-3 | 0 | 27.5±4.5% | 47.4±2.4% | 0 | 0 | 0 |
| MMP-7 | 0 | 48.6±3.4% | 55.1±3.6% | 0 | 0 | 0 |
| MMP-9 | 0 | 35.5±5.6% | 54.7±4.5% | 0 | 0 | 0 |
| MMP-11 | 0 | 7.6±5.5% | 28.4±4.4% | 0 | 0 | 0 |
| MMP-13 | 0 | 18±3.6% | 43.4±4.6% | 0 | 0 | 0 |
| MMP-14 | 0 | 26.3±4.4% | 48.6±5.6% | 0 | 0 | 0 |

**Table 58. Percentage of fibroblast cells which expressed the analyzed markers in co-culture with cells isolated from pancreatic carcinoma and in the presence of SP (1 µM) and various non-peptide antagonists of the NK1 receptor to 1µM concentration.**

| **Marker** | **Fibroblast** | **Primary Tumor cells co-cultured + fibroblast** | **Primary Tumor cells co-cultured + fibroblast + SP** | **Primary Tumor cells co-cultured + fibroblast + SP + Aprepitant** | **Primary Tumor cells co-cultured + fibroblast + SP + Vestipitant** | **Primary Tumor cells co-cultured + fibroblast + SP + Casopitant** |
|---|---|---|---|---|---|---|
| TGFα | 0 | 16.5±3.2% | 44.2±4.5% | 0 | 0 | 0 |
| TGFβ 1 | 0 | 18.6±4.2% | 356±5.4% | 0 | 0 | 0 |
| TGFβ 2 | 0 | 21.3±3.1% | 43.6±7.4% | 0 | 0 | 0 |
| TGFβ 3 | 0 | 14.3±5.4% | 45.4±5.6% | 0 | 0 | 0 |
| SPARC | 0 | 18.1±6.7% | 43.7±4.5% | 0 | 0 | 0 |
| MMP-3 | 0 | 24.5±4.4% | 47.5±6.1% | 0 | 0 | 0 |
| MMP-7 | 0 | 45.6±4.4% | 56.6±5.1% | 0 | 0 | 0 |
| MMP-9 | 0 | 37.6±3.6% | 54.1±5.2% | 0 | 0 | 0 |
| MMP-11 | 0 | 8±4.7% | 27.1±6.2% | 0 | 0 | 0 |
| MMP-13 | 0 | 16.6±5.1% | 57.2±4.7% | 0 | 0 | 0 |
| MMP-14 | 0 | 19.3±3.1% | 46.2±5.1% | 0 | 0 | 0 |

**Table 59. Percentage of fibroblast cells which expressed the analyzed markers in co-culture with cells isolated from lung cancer and in the presence of SP (1 µM) and various non-peptide antagonists of the NK1 receptor to 1µM concentration.**

| **Marker** | **Fibroblast** | **Primary Tumor cells co-cultured + fibroblast** | **Primary Tumor cells co-cultured + fibroblast + SP** | **Primary Tumor cells co-cultured + fibroblast + SP + Aprepitant** | **Primary Tumor cells co-cultured + fibroblast + SP + Vestipitant** | **Primary Tumor cells co-cultured + fibroblast + SP + Casopitant** |
|---|---|---|---|---|---|---|
| TGFα | 0 | 15.4±2.2% | 37.5±2.3% | 0 | 0 | 0 |
| TGFβ 1 | 0 | 14.5±3.2% | 37.3±3.3% | 0 | 0 | 0 |
| TGFβ 2 | 0 | 21.4±3.3% | 52.3±2.8% | 0 | 0 | 0 |
| TGFβ 3 | 0 | 14.6±3.5% | 46.1±4.3% | 0 | 0 | 0 |
| SPARC | 0 | 18.7±3.6% | 47.3±4.2% | 0 | 0 | 0 |
| MMP-3 | 0 | 22.5±4.1% | 51.5±5.8% | 0 | 0 | 0 |
| MMP-7 | 0 | 47.3±4.5% | 54.4±6.3% | 0 | 0 | 0 |
| MMP-9 | 0 | 34.4±4.7% | 56.5±4.6% | 0 | 0 | 0 |
| MMP-11 | 0 | 10.5±4.2% | 38.6±3.4% | 0 | 0 | 0 |
| MMP-13 | 0 | 15.8±3.3% | 46.3±3.4% | 0 | 0 | 0 |
| MMP-14 | 0 | 31.5±5.1% | 47.6±5.6% | 0 | 0 | 0 |

**Table 60. Percentage of fibroblast cells which expressed the analyzed markers in co-culture with cells isolated from glioma cells and in the presence of SP (1 µM) and various non-peptide antagonists of the NK1 receptor to 1µM concentration.**

| **Marker** | **Fibroblast** | **Primary Tumor cells co-cultured + fibroblast** | **Primary Tumor cells co-cultured + fibroblast + SP** | **Primary Tumor cells co-cultured + fibroblast + SP + Aprepitant** | **Primary Tumor cells co-cultured + fibroblast + SP + Vestipitant** | **Primary Tumor cells co-cultured + fibroblast + SP + Casopitant** |
|---|---|---|---|---|---|---|
| TGFα | 0 | 15.5±2.3% | 38.4±2.3% | 0 | 0 | 0 |
| TGFβ 1 | 0 | 16.5±3.3% | 35.4±2.6% | 0 | 0 | 0 |
| TGFβ 2 | 0 | 23.4±2.4% | 42.4±4.2% | 0 | 0 | 0 |
| TGFβ 3 | 0 | 19.3±3.1% | 52.1±3.7% | 0 | 0 | 0 |
| SPARC | 0 | 18.2±2.7% | 56.2±4.2% | 0 | 0 | 0 |
| MMP-3 | 0 | 25.3±3.5% | 48.4±5.1% | 0 | 0 | 0 |
| MMP-7 | 0 | 47.3±4.6% | 59.4±5.7% | 0 | 0 | 0 |
| MMP-9 | 0 | 34.4±4.4% | 55.5±4.5% | 0 | 0 | 0 |
| MMP-11 | 0 | 7.1±1.7% | 28.9±4.7% | 0 | 0 | 0 |
| MMP-13 | 0 | 19.2±2.5% | 48.0±5.6% | 0 | 0 | 0 |
| MMP-14 | 0 | 21.2±3.5% | 46.3±3.6% | 0 | 0 | 0 |

**Table 61. Percentage of fibroblast cells which expressed the analyzed markers in co-culture with cells isolated from multiple myeloma and in the presence of SP (1 µM) and various non-peptide antagonists of the NK1 receptor to 1µM concentration.**

| **Marker** | **Fibroblast** | **Primary Tumor cells co-cultured + fibroblast** | **Primary Tumor cells co-cultured + fibroblast + SP** | **Primary Tumor cells co-cultured + fibroblast + SP + Aprepitant** | **Primary Tumor cells co-cultured + fibroblast + SP + Vestipitant** | **Primary Tumor cells co-cultured + fibroblast + SP + Casopitant** |
|---|---|---|---|---|---|---|
| TGFα | 0 | 14.4±1.1% | 49.3±3.3% | 0 | 0 | 0 |
| TGFβ 1 | 0 | 14.5±1.1% | 45.6±4.2% | 0 | 0 | 0 |
| TGFβ 2 | 0 | 23.4±1.2% | 52.3±3.2% | 0 | 0 | 0 |
| TGFβ 3 | 0 | 15.4±1.6% | 47.4±3.5% | 0 | 0 | 0 |
| SPARC | 0 | 16.1±1.8% | 46.3±3.2% | 0 | 0 | 0 |
| MMP-3 | 0 | 22.5±1.2% | 46.6±5.7% | 0 | 0 | 0 |
| MMP-7 | 0 | 45.4±2.3% | 57.6±4.1% | 0 | 0 | 0 |
| MMP-9 | 0 | 34.5±1.5% | 57.3±4.5% | 0 | 0 | 0 |
| MMP-11 | 0 | 12.7±2.4% | 29.4±3.1% | 0 | 0 | 0 |
| MMP-13 | 0 | 18.4±2.4% | 47.5±4.3% | 0 | 0 | 0 |
| MMP-14 | 0 | 22.4±2.2% | 47.2±3.4% | 0 | 0 | 0 |

### Example 12. The interaction of human tumor cells directly obtained from the tumor sample from a patient with immune cells/inflammatory (mono and polymorphonuclear leukocytes) obtained from the same patient, stimulates the secretion of substances of importance for survival and progression of tumors and treatment with non-peptide antagonists NK1 receptors inhibits said secretion.

To check that the interaction of the human primary tumor cells with cells of the immune/inflammatory (mono and polymorphonuclear leukocytes) cells, stimulates the secretion of substances of importance for survival and tumor progression, cultures were carried out using sets of immune cells / inflammatory (mono and polymorphonuclear leukocytes) with tumor cells obtained directly from primary human tumors of the same patients. Individual tumors and patient characteristics are expressed in Table 36.

The same method described in Example 7 and 9 was used to obtain tumor cells from primary human tumors. Similarly cultured immune cells/inflammatory (mono and polymorphonuclear leukocytes) were used as control and secondly, cultured tumor cells isolated from patients in the presence of immune cells/inflammatory (mono and polymorphonuclear leukocytes). On the other hand the tumor cells were cultured in the presence of immune cells/inflammatory (mono and polymorphonuclear leukocytes) and the NK1 receptor agonist (SP). Finally, the cells were cultured in the presence of immune/inflammatory (mono and polymorphonuclear leukocytes) tumor cells, NK1 receptor agonist (SP) and each of the non-peptide receptor antagonists Aprepitant, Vestipitant and Casopitant.

As a preliminary step, it was found that all cells expressed NK1 receptor, by Western blotting. Then, paraffin blocks were prepared with the contents of each of the groups mentioned previously for expression studies using immunohistochemistry. On this occasion, in order to identify the secretion of substances with importance for the survival and progression of tumor the following markers were analyzed: TGF-β 2 (SAB4502956) and NF-kB (SAB4501992) using specific antibodies against them. All antibodies used were rabbit polyclonal antibodies obtained from Sigma Aldrich and were used at a concentration of 1/1000. Immunohistochemical techniques were performed and evaluated as described in previous examples.

Tables 62 to 66 detail the percentage of cells with an expression for each of the markers (a substance important for the tumor microenvironment) for co-cultures of tumor cells-leukocyte cell, tumor cells-leukocyte cell with NK1 receptor agonist exposure (SP), tumor cells and leukocyte with NK1 receptor agonist (SP) exposure and each of the NK1 receptor antagonists: Aprepitant, Vestipitant and Casopitant. In the tables 62 to 66, five examples of co-cultures leukocyte cell are shown, together with various tumor cells taken from tumors of the type: colon cancer, pancreatic cancer, lung cancer, glioma and melanoma.

We obtained the same results when other non-peptide NK1 receptors were used: L-733,060, L-732,138, L-703,606, CP-100263, WIN 62,577, WIN 51708, CP-96345 and L-760735.

The results shown in these tables demonstrate the interaction of primary human tumor cells and immune system cells, human mono and polymorphonuclear leukocytes, stimulates the expression of substances in the tumor microenvironment importance to the survival and tumor progression and NK1 receptor agonist (SP) enhances this phenomenon, whereas treatment with the non-peptide NK1 receptors inhibit such expression.

**Table 62. Percentage of leukocyte cells (poly and morfonuclear) which expressed the analyzed markers in co-culture with cells isolated from colon carcinoma and in the presence of SP (1 µM) and various non-peptide antagonists of the NK1 receptor to 1µM concentration.**

| **Marker** | **Leukocytes** | **Primary Tumor cells co-cultured + Leukocytes** | **Primary Tumor cells co-cultured + Leukocyte s + SP** | **Primary Tumor cells co-cultured + Leukocyte s+ SP + Aprepitant** | **Primary Tumor cells co-cultured + Leukocyte s + SP + Vestipitant** | **Primary Tumor cells co-cultured + Leukocyte s + SP+ Casopitant** |
|---|---|---|---|---|---|---|
| Mononuclear leukocytes cells | | | | | | |
| TGF-β | 0 | 16.6±3.1 % | 36.5±3.5 % | 0 | 0 | 0 |
| N F-kB | 0 | 21.5±4.1 % | 47.5±4.6 % | 0 | 0 | 0 |

| Polymorphonuclear leukocytes cells | | | | | | |
|---|---|---|---|---|---|---|
| TGF-β | 0 | 16.6±3.2 % | 45.8±3.5 % | 0 | 0 | 0 |
| NF-kB | 0 | 21.3±4.2 % | 36.6±4.7 % | 0 | 0 | 0 |

**Table 63. Percentage of leukocyte cells (poly and morfonuclear) which expressed the analyzed markers in co-culture with cells isolated from pancreatic carcinoma and in the presence of SP (1 µM) and various non-peptide antagonists of the NK1 receptor to 1µM concentration.**

| **Marker** | **Leukocytes** | **Primary Tumor cells co-cultured + Leukocyte s** | **Primary Tumor cells co-cultured + Leukocyte s + SP** | **Primary Tumor cells co-cultured + Leukocyte s+ SP + Aprepitant** | **Primary Tumor cells co-cultured + Leukocyte s + SP + Vestipitant** | **Primary Tumor cells co-cultured + Leukocyte s + SP+ Casopitant** |
|---|---|---|---|---|---|---|
| Mononuclear leukocytes cells | | | | | | |
| TGF-β | 0 | 16.5±3.1 % | 39.5±4.5 % | 0 | 0 | 0 |
| NF-kB | 0 | 20.4±3.2 % | 375±3.6% | 0 | 0 | 0 |

| Polymorphonuclear leukocytes cells | | | | | | |
|---|---|---|---|---|---|---|
| TGF-β | 0 | 15.5±3.5 % | 36.5±3.1 % | 0 | 0 | 0 |
| NF-kB | 0 | 24.6±4.6 % | 35.4±4.2 % | 0 | 0 | 0 |

**Table 64. Percentage of leukocyte cells (poly and morfonuclear) which expressed the analyzed markers in co-culture with cells isolated from lung carcinoma and in the presence of SP (1 µM) and various non-peptide antagonists of the NK1 receptor to 1µM concentration.**

| **Marker** | **Leukocytes** | **Primary Tumor cells co-cultured + Leukocytes** | **Primary Tumor cells co-cultured + Leukocyte s + SP** | **Primary Tumor cells co-cultured + Leukocytes+ SP + Aprepitant** | **Primary Tumor cells co-cultured + Leukocytes + SP + Vestipitant** | **Primary Tumor cells co-cultured + Leukocytes + SP + Casopitant** |
|---|---|---|---|---|---|---|
| Mononuclear leukocytes cells | | | | | | |
| TGF-β | 0 | 15.6±3.1 % | 42.7±3.3 % | 0 | 0 | 0 |
| N F-kB | 0 | 21.5±4.3 % | 41.6±4.2 % | 0 | 0 | 0 |

| Polymorphonuclear leukocytes cells | | | | | | |
|---|---|---|---|---|---|---|
| TGF-β | 0 | 14.5±3.1 % | 38.7±3.3 % | 0 | 0 | 0 |
| N F-kB | 0 | 19.5±4.3 % | 35.5±4.2 % | 0 | 0 | 0 |

**Table 65. Percentage of leukocyte cells (poly and morfonuclear) which expressing the analyzed markers in co-culture with cells isolated from glioma cells and in the presence of SP (1 µM) and various non-peptide antagonists of the NK1 receptor to 1µM concentration.**

| **Marker** | **Leukocytes** | **Primary Tumor cells co-cultured + Leukocytes** | **Primary Tumor cells co-cultured + Leukocyte s + SP** | **Primary Tumor cells co-cultured + Leukocyte s+ SP + Aprepitant** | **Primary Tumor cells co-cultured + Leukocyte s + SP + Vestipitant** | **Primary Tumor cells co-cultured + Leukocytes + SP+ Casopitant** |
|---|---|---|---|---|---|---|
| Mononuclear leukocytes cells | | | | | | |
| TGF-β | 0 | 21.5±3.1% | 41.7±3.4 % | 0 | 0 | 0 |
| N F-kB | 0 | 26.5±4.3% | 41.6±4.1 % | 0 | 0 | 0 |

| Polymorphonuclear leukocytes cells | | | | | | |
|---|---|---|---|---|---|---|
| TGF-β | 0 | 29.3±3.6% | 39.7±3.3 % | 0 | 0 | 0 |
| N F-kB | 0 | 19.5±3.8% | 39.4±4.2 % | 0 | 0 | 0 |

**Table 66. Percentage of leukocyte cells (poly and morfonuclear) which expressed the analyzed markers in co-culture with cells isolated from melanoma and in the presence of SP (1 µM) and various non-peptide antagonists of the NK1 receptor to 1µM concentration.**

| **Marker** | **Leukocyte s** | **Primary Tumor cells co-cultured + Leukocytes** | **Primary Tumor cells co-cultured + Leukocyte s+SP** | **Primary Tumor cells co-cultured + Leukocyte s+ SP + Aprepitant** | **Primary Tumor cells co-cultured + Leukocyte s + SP + Vestipitant** | **Primary Tumor cells co-cultured + Leukocyte s + SP+ Casopitant** |
|---|---|---|---|---|---|---|
| Mononuclear leukocytes cells | | | | | | |
| TGF-β | 0 | 19.9±2.2% | 49.6±4.2 % | 0 | 0 | 0 |
| NF-kB | 0 | 24.8±3.2% | 51.7±5.3 % | 0 | 0 | 0 |

| Polymorphonuclear leukocytes cells | | | | | | |
|---|---|---|---|---|---|---|
| TGF-β | 0 | 22.9±4.3% | 54.5±3.5 % | 0 | 0 | 0 |
| NF-kB | 0 | 26.9±3.4% | 56.6±4.6 % | 0 | 0 | 0 |

### Example 13. The interaction of the tumor cells with human immune cells/inflammatory (macrophages), stimulates the expression of substances of great importance in the survival of both types of cells and treatment with non-peptide NK1 receptors antagonist inhibit said secretion.

To check that the interaction of tumor cells obtained directly from primary human immune cell/inflammatory (macrophages) cells, obtained from the patient stimulate secretion of substances by these macrophages recognized as important in survival of both cell types and in the progression of tumor cells, co-cultures were performed on fresh blood plasma, immune cell / inflammatory (macrophages) with tumor cells derived from primary tumors. All obtained from the same patient, to build an experimental model similar to human physiology. Different tumors were obtained for culture cells and the characteristics of these patients and donors are given in Table 36.

The same method, as described in Example 7 and 10, was used to obtain tumor cells from primary human tumors. Macrophages were obtained from washing or pleural or peritoneal from the same patient from which the primary tumor cells were obtained in each case.

Similarly to the method in Example 7 (above), a total of 6 wells containing only macrophages were used as control, another 6 wells containing tumor cells were cultured with the addition of immune cells/inflammatory (macrophages), another 6 wells containing tumor cells were cultured in the presence of immune cells/inflammatory (macrophages) and the NK1 receptor agonist (SP), another group of six wells containing tumor cells were cultured in the presence of immune cells/inflammatory (macrophages), NK1 receptor agonist (SP) and each of the non-peptide antagonists of the NK1 receptor, Aprepitant, Vestipitant and Casopitant.

Previously, it was found that the tumor cells and immune system cells/inflammatory (macrophages) human NK1 receptor, expressed by the technique of Western blotting. then a paraffin block with the content of each of the wells were prepared, as described in Example 1.

In order to identify the different cell types to be able to count data immunohistochemistry with primary antibodies specific to detect substances of importance in survival and tumor progression was performed: EGF (rabbit monoclonal antibody, anti-EGF, 07 - 1432, Merck-M lipore), MMP-9 (rabbit polyclonal anti-MMP-9, SAB4501896, Sigma-Aldrich), VEGF (mouse monoclonal anti-VEGF, GF25-100UG, Merck-Miilipore) and TNF-α (mouse monoclonal anti-TNF-α, Merck- Miilipore) MAB102L).

Tables 67 to 71 detail the percentage of cells with an expression for each of the markers (a substance important for the tumor microenvironment) for co-cultures of macrophage tumor cells, tumor cells with macrophage receptor agonist exposure NK1 (SP), with tumor cells and macrophages exposed to NK1 receptor agonist (SP) and each of the NK1 receptor antagonists: Aprepitant, Vestipitant and Casopitant. In the tables 67 to 71 five examples are shown of leukocyte cells co-cultures together with various tumor cells taken from tumors of the type: colon cancer, melanoma, lung cancer, ovarian cancer and breast cancer. We obtained the same results when used other non-peptide NK1 receptor antagonists: L-733,060, L-732, 138, L-703,606, CP-100263, WIN 62,577, WIN 51708, CP-96345 and L-760735.

The results shown in these tables demonstrate the interaction of primary human tumor cells and cells of the immune/inflammatory human system, macrophage, stimulates the expression of substances in the tumor microenvironment and its importance to the survival and progression of tumors; NK1 receptor agonist (SP) enhances this phenomenon, whereas treatment with the non-peptide NK1 receptors antagonists inhibit such expression.

**Table 67. Percentage of macrophages which expressed the analyzed markers in co-culture with cells isolated from colon carcinoma and in the presence of SP (1 µM) and various non-peptide antagonists of the NK1 receptor to 1µM concentration.**

| **Marker** | **Macrophages** | **Primary Tumor cells co-cultured + Macropha ges** | **Primary Tumor cells co-cultured + Macropha ges + SP** | **Primary Tumor cells co-cultured + Macropha ges + SP + Aprepitant** | **Primary Tumor cells co-cultured + Macropha ges + SP + Vestipitant** | **Primary Tumor cells co-cultured + Macrophages + SP+ Casopitant** |
|---|---|---|---|---|---|---|
| EGF | 0 | 17.6±3.4 % | 41.4±4.2 % | 0 | 0 | 0 |
| MMP-9 | 0 | 21.7±3.4 % | 39.5±3.5 % | 0 | 0 | 0 |
| VEGF | 0 | 19.3±2.9 % | 38.5±2.9 % | 0 | 0 | 0 |

**Table 68. Percentage of macrophages which expressed the analyzed markers in co-culture with cells isolated from breast carcinoma and in the presence of SP (1 µM) and various non-peptide antagonists of the NK1 receptor to 1µM concentration.**

| **Marker** | **Macrophages** | **Primary Tumor cells co-cultured + Macropha ges** | **Primary Tumor cells co-cultured + Macropha ges + SP** | **Primary Tumor cells co-cultured + Macropha ges + SP + Aprepitant** | **Primary Tumor cells co-cultured + Macropha ges + SP + Vestipitant** | **Primary Tumor cells co-cultured + Macrophages + SP+ Casopitant** |
|---|---|---|---|---|---|---|
| EGF | 0 | 19.6±3.2 % | 41.6±4.2 % | 0 | 0 | 0 |
| MMP-9 | 0 | 23.6±3.3 % | 39.5±3.2 % | 0 | 0 | 0 |
| VEGF | 0 | 19.8±4.2 % | 37.4±4.2 % | 0 | 0 | 0 |

**Table 69. Percentage of macrophages which expressed the analyzed markers in co-culture with cells isolated from ovarian carcinoma and in the presence of SP (1 µM) and various non-peptide antagonists of the NK1 receptor to 1µM concentration.**

| **Marker** | **Macrophages** | **Primary Tumor cells co-cultured + Macrophages** | **Primary Tumor cells co-cultured + Macrophages+SP** | **Primary Tumor cells co-cultured + Macropha ges + SP + Aprepitant** | **Primary Tumor cells co-cultured + Macropha ges + SP + Vestipitant** | **Primary Tumor cells co-cultured + Macrophages + SP+ Casopitant** |
|---|---|---|---|---|---|---|
| EGF | 0 | 12.1±2% | 41.5±4.2% | 0 | 0 | 0 |
| MMP-9 | 0 | 19.4±2.5% | 39.5±3.2% | 0 | 0 | 0 |
| VEGF | 0 | 18.4±3.5% | 37.4±4% | 0 | 0 | 0 |

**Table 70. Percentage of macrophages which expressed the analyzed markers in co-culture with cells isolated from lung carcinoma and in the presence of SP (1 µM) and various non-peptide antagonists of the NK1 receptor to 1µM concentration.**

| **Marker** | **Macrophages** | **Primary Tumor cells co-cultured + Macrophages** | **Primary Tumor cells co-cultured + Macrophages + SP** | **Primary Tumor cells co-cultured + Macrophages + SP + Aprepitant** | **Primary Tumor cells co-cultured + Macrophages + SP + Vestipitant** | **Primary Tumor cells co-cultured + Macrophages + SP+ Casopitant** |
|---|---|---|---|---|---|---|
| EGF | 0 | 16.2±3.1% | 38.4±2.9 % | 0 | 0 | 0 |
| MMP-9 | 0 | 22.5±2.6% | 39.4±3.7 % | 0 | 0 | 0 |
| VEGF | 0 | 21.8±4.2% | 37.4±4.2 % | 0 | 0 | 0 |

**Table 71. Percentage of macrophages which expressed the analyzed markers in co-culture with cells isolated from melanoma cells and in the presence of SP (1 µM) and various non-peptide antagonists of the NK1 receptor to 1µM concentration.**

| **Marker** | **Macrophages** | **Primary Tumor cells co-cultured + Macrophages** | **Primary Tumor cells co-cultured + Macrophages + SP** | **Primary Tumor cells co-cultured + Macrophages + SP + Aprepitant** | **Primary Tumor cells co-cultured + Macrophages + SP + Vestipitant** | **Primary Tumor cells co-cultured + Macrophages + SP+ Casopitant** |
|---|---|---|---|---|---|---|
| EGF | 0 | 10.3±1.5 % | 41.9±3.2 % | 0 | 0 | 0 |
| MMP-9 | 0 | 12.4±2.6 % | 39.5±5.3 % | 0 | 0 | 0 |
| VEGF | 0 | 16.6±3.1 % | 35.4±4.4 % | 0 | 0 | 0 |

### Example 14. The treatment of the non-peptide antagonist of the NK1 receptors inhibits proliferation of tumor cells when the receptor acts exclusively by way of the MAP-kinases. The treatment of the non-peptide antagonist of the NK1 receptors inhibits both the proliferation of these cells when cultured together with stromal cells - fibroblasts.

As demonstrated in Example 8, the interaction of the human primary tumor cells with stromal cells, human fibroblasts, stimulates the proliferation of both cell types, demonstrating the importance of the substances secreted by the latter (which constitute the tumor microenvironment) have for the survival and the progression of said tumor cells.

The same method described in Example 7 was used to obtain tumor cells from primary human tumors. Different tumors and patient characteristics are shown in Table 36. Of each of the tumors shown in the Table 36, four showed expression of MAP kinases route after treatment with different non-peptide NK1 antagonists and four others did not express the aforesaid route after treatment with different non-peptide NK1 antagonists. Similarly, cultures from stromal fibroblast cells were created from skin samples obtained from the same patient in the area of the surgical incision that was made during the procedure to remove their tumor. Similarly to the procedure in Example 7, a total of 6 wells containing tumor cells from tumors pathway integrity of MAP Kinases ([ERK with absent] after treatment with nonpeptide NK1 receptors) are used as control survival of tumor cells of such tumors. Another 6 wells were used as control survival of tumor cells from tumors pathway impairment of MAP Kinases (ERK present after treatment with non-peptide NK1 receptors). Another 6 wells containing tumor cells from tumors pathway integrity of MAP Kinases ([ERK with absent] after treatment with nonpeptide receptor NK1) were cultured in the presence of various non-peptide receptor antagonists NK1. Another 6 wells containing tumor cells from tumors pathway impairment of MAP Kinases (ERK presence after treatment with non-peptide NK1 receptor antagonists) were cultured in the presence of various non-peptide receptor antagonists NK1. Another 6 wells containing tumor cells from tumors pathway integrity of MAP Kinases (ERK with absent after treatment with non-peptide NK1 receptor antagonists) were cultured in the presence of stromal cells, fibroblasts, and various non-peptide NK1 receptor antagonists. Another 6 wells containing tumor cells from tumors pathway impairment of MAP Kinases (ERK presence after treatment with non-peptide NK1 receptor antagonists) were cultured in the presence of stromal cells, fibroblasts, and various non-peptide NK1 receptor antagonists.

Previously, it was found that the tumor cells and stromal cells- fibroblasts- expressed NK1 receptor by the technique of Western blotting. Then a paraffin block with the content of each of the wells was prepared, as described in Example 1. To test for proteins belonging to the route of MAP Kinases in different cell cultures was performed by Western blotting, using as primary antibody: Phospho-p44/42 MAPK (Erkl / 2) (Thr202/Tyr204) (D 13.14.4E) XP ^{®} Rabbit mAb (4370, Cell Signaling). On this occasion, in order to identify the different cell types in order to quantify them, immunohistochemistry was performed with labeling with primary antibodies specific for stromal cell human-fibroblast (Anti-smooth muscle actin), carcinoma cells (anti-cytokeratins broad spectrum), Glioma (Anti-glial fibrillary acidic protein), Ewing's sarcoma (Anti-CD99), Melanoma (Anti-HMB45), leukemias and lymphomas (Anti-leukocyte common antigen) and myeloma (anti-CD138). All antibodies are distributed by Dako and used at the concentration at which they are supplied (supplied pre-diluted - "ready to use" -).

In Tables 72 and 73, it can be seen that Aprepitant only inhibits the growth of cells in which the receptor acts NK1 through the MAP Kinases pathway, while cells in which no inhibition of growth occurs, there was no observed modification of this route. However, when tumor cells are co-cultured with stromal fibroblasts cells, the antagonist produces a proliferation of inhibition of tumor cells, whether they originate from tumors or where there is no path integrity of MAP Kinases "downstream" of the receiver. That is, whether or not acting receiver via said signaling pathway in tumor cells.

We obtained the same results when other non-peptide receptor NK1 antagonists were used: Vestipitant, Casopitant, L-733,060, L-732,138, L-703,606, CP-100263, WIN 62,577, WIN 51708, CP-96345 and L- 760735.

**Table 72. Percentage of inhibition/proliferation of tumor cells from primary human tumors in amending ERK (their presence is not detected after treatment with NK1 receptor antagonists - said receptor acts through the MAP Kinase pathway in tumor cells) in culture with this receptor antagonist Aprepitant (1µM) and stromal cell culture with human fibroblast and Aprepitant (1µM).**

| **Type of tumor** | **Tumor cells (control)** | **Tumor cells + Aprepitant.** | **Tumor cells co-cultivated with fibroblast (control)** | **Tumor cells co-cultivated with fibroblast + Aprepitant.** |
|---|---|---|---|---|
| Carcinoma of stomach. | 100 | 50±2 | 100 | 21±3 |
| Carcinoma of Colon. | 100 | 62±4 | 100 | 19±4 |
| Carcinoma of Pancreas. | 100 | 68±5 | 100 | 22±3 |
| Renal carcinoma | 100 | 76±3 | 100 | 23±3 |
| Carcinoma of breast. | 100 | 78±2 | 100 | 19±4 |
| Carcinoma of ovarian. | 100 | 61±2 | 100 | 20±5 |
| Carcinoma of endometrial. | 100 | 68±3 | 100 | 19±8 |
| Carcinoma of cervix. | 100 | 70±3 | 100 | 19±8 |
| Carcinoma of human small cell lung | 100 | 61±3 | 100 | 23±5 |
| Carcinoma of human non-small cell lung | 100 | 69±4 | 100 | 19±7 |
| Carcinoma of thyroid. | 100 | 70±2 | 100 | 19±1 |
| Carcinoma of prostate. | 100 | 68±3 | 100 | 19±7 |
| Glioma | 100 | 69±4 | 100 | 31±4 |
| Sarcoma of Ewing. | 100 | 69±5 | 100 | 19±6 |
| Melanoma | 100 | 68±6 | 100 | 19±7 |
| Neuroblastoma | 100 | 69±6 | 100 | 21±5 |
| Leukemia B | 100 | 70±1 | 100 | 18±4 |
| Leukemia T | 100 | 60±1 | 100 | 23±4 |
| Non-Hodgkin's Lymphoma B | 100 | 61±2 | 100 | 19±6 |
| Non-Hodgkin's Lymphoma T | 100 | 61±2 | 100 | 2±5 |
| Hodgkin's Lymphoma | 100 | 60±3 | 100 | 18±4 |
| Myeloma | 100 | 71±4 | 100 | 19±4 |

**Table 73. Percentage of inhibition/stimulation of the proliferation of tumor cells from primary human tumors in which ERK is unchanged (its presence is detected after treatment with NK1 receptor antagonists - said receptor does not act through the MAP Kinase pathway in tumor cells) in culture with said receptor antagonist Aprepitant (1µM) and in culture with stromal cells, human-fibroblasts, and Aprepitant (1µM).**

| **Type of tumor** | **Tumor cells (control)** | **Tumor cells + Aprepitant.** | **Tumor cells co-cultivated with fibroblast (control)** | **Tumor cells co-cultivated with fibroblast + Aprepitant.** |
|---|---|---|---|---|
| Carcinoma of stomach. | 100 | 100±2 | 100 | 21±3 |
| Carcinoma of Colon. | 100 | 100±4 | 100 | 19±4 |
| Carcinoma of Pancreas. | 100 | 101±5 | 100 | 22±3 |
| Renal carcinoma | 100 | 99±3 | 100 | 23±3 |
| Carcinoma of breast. | 100 | 100±2 | 100 | 19±4 |
| Carcinoma of ovarian. | 100 | 101±2 | 100 | 20±5 |
| Carcinoma of endometrial. | 100 | 98±3 | 100 | 19±8 |
| Carcinoma of cervix. | 100 | 100±3 | 100 | 19±8 |
| Carcinoma of human small cell lung | 100 | 101±3 | 100 | 23±5 |
| Carcinoma of human non-small cell lung | 100 | 99±4 | 100 | 19±7 |
| Carcinoma of thyroid. | 100 | 100±2 | 100 | 19±1 |
| Carcinoma of prostate. | 100 | 98±3 | 100 | 19±7 |
| Glioma | 100 | 99±4 | 100 | 31±4 |
| Sarcoma of Ewing. | 100 | 99±5 | 100 | 19±6 |
| Melanoma | 100 | 98±6 | 100 | 19±7 |
| Neuroblastoma | 100 | 99±6 | 100 | 21±5 |
| Leukemia B | 100 | 100±1 | 100 | 18±4 |
| Leukemia T | 100 | 100±1 | 100 | 23±4 |
| Non-Hodgkin's Lymphoma B | 100 | 101±2 | 100 | 19±6 |
| Non-Hodgkin's Lymphoma T | 100 | 101±2 | 100 | 2±5 |
| Hodgkin's Lymphoma | 100 | 100±3 | 100 | 18±4 |
| Myeloma | 100 | 101±4 | 100 | 19±4 |

The results show that in the cases where there was an inhibition of proliferation, was pathway integrity of MAP Kinases, but showed a lack of expression of ERK. This means that antagonists NK1 receptor inhibit proliferation in this group of tumor cells through the MAP Kinase pathway. However, in cases where there was decreased proliferation after treatment with NK1 receptor antagonists, the presence of ERK expression was confirmed, which means that with treatment with NK1 receptor antagonists, that path does not inhibit the MAP Kinases. Therefore, the inhibition of tumor cell proliferation by said inhibition occurs by antagonists, "downstream" of the MAP Kinase pathway. However, when both types of tumor cells are grown (with integrity of the MAP Kinases, and therefore with no ERK after treatment with NK1 receptor antagonists - and alteration of the MAP Kinase pathway - and therefore presence of ERK after treatment with NK1 receptor antagonists) with fibroblasts derived from the same patient, they observed inhibition of proliferation of tumor cells, regardless of the state of the MAP Kinase pathway. This demonstrates that the non-peptide NK1 receptors antagonists inhibit the proliferation of tumor cells by mechanisms different from those known in the prior art and related to blocking the secretion of substances produced by the interaction of these cells with other cells characteristic of the tumor microenvironment, specifically stromal fibroblast cells.

### Example 15. Treatment with non-peptide NK1 receptors antagonists inhibits proliferation of tumor cells when the receptor acts exclusively through the PI3 Kinase pathway. Treatment with non-peptide NK1 receptor antagonists inhibits both the proliferation of these cells when cultured together with cells of the stromal fibroblasts.

The same method described in Example 7 was used to obtain tumor cells from primary human tumors. Individual tumors and patient characteristics are shown in Table 36. Of each of the tumors shown in the Table 36, four showed expression of PI3 Kinase route after treatment with different non-peptide NK1 antagonists and four others did not express said route after treatment with different non-peptide NK1 antagonists. Similarly, stromal fibroblast cells were obtained, to culture from skin samples obtained from the same patient in the area of surgical incision during the procedure performed to remove their tumor. Similarly to what was conducted in Example 7, a total of 6 wells containing tumor cells from tumors pathway integrity of the PI3 Kinase (AKT was absent after treatment with non-peptide NK1 receptor antagonists) is used as control survival of tumor cells of such tumors. Another 6 wells were used as control survival of tumor cells derived from tumors with altered via the PI3 Kinase (AKT presence after treatment with non-peptide NK1 receptor antagonists). Another 6 wells containing tumor cells from tumors pathway integrity of the PI3 Kinase (AKT was absent after treatment with non-peptide NK1 receptor antagonists) were cultured in the presence of various non-peptide receptor antagonists NK1. Another 6 wells containing tumor cells from tumors with altered via the PI3 Kinase (with presence of AKT after treatment with non-peptide NK1 receptor antagonists) were cultured in the presence of various non-peptide NK1 receptor antagonists. Another 6 wells containing tumor cells from tumors pathway integrity of the PI3 Kinase (AKT was absent after treatment with non-peptide NK1 receptor antagonists) were cultured in the presence of stromal cells, fibroblasts, and various non-peptide NK1 receptor antagonists. Another 6 wells containing tumor cells from tumors with alterations via the PI3 Kinase (with presence of AKT after treatment with non-peptide NK1 receptor antagonists) were cultured in the presence of stromal fibroblast cells and various non-peptide NK1 receptor antagonists.

Previously, it was found that the tumor cells and stromal fibroblast cells expressed NK1 receptor by the technique of Western blotting. Then a paraffin block with the content of each of the wells was prepared, as described in Example 1. AKT in different cell cultures were performed Western blotting (as described in Example 1-Western Blot-section), but using the primary antibody with reference: Akt (pan) (11 E7) Rabbit mAb (4685, Cell Signalling). On this occasion, in order to identify the different cell types in order to quantify them, immunohistochemistry was performed with labeling with primary antibodies specific for stromal cell human-fibroblast (Anti-smooth muscle actin), carcinoma cells ( anti-cytokeratins broad spectrum) Glioma (Anti-glial fibrillary acidic protein), Ewing's sarcoma (Anti-CD99), Melanoma (Anti-HMB45), leukemias and lymphomas (Anti-leukocyte common antigen) and myeloma (anti-CD138 ). All antibodies are distributed by Dako and used at the concentration at which they are supplied (supplied pre-diluted - "ready to use").

In tables 74 and 75, it can be seen that non-peptide antagonist of the NK1 receptor Aprepitant only inhibits the growth of cells in which said receptor acts through the PI3 Kinase pathway, whereas cells that do not produce inhibition there is no observed modification of this route. However, when tumor cells are co-cultured with stromal fibroblast cells, said antagonist produces a proliferation of inhibition of tumor cells, whether they originate from tumors or where there is no path integrity of the PI3 kinases "downstream" of the receiver, that is, whether or not acting receiver via said signaling pathway in tumor cells. We obtained the same results when used other non-peptide NK1 receptor antagonists: Vestipitant, Casopitant, L-733,060, L-732,138, L-703,606, CP-100263, WIN 62,577, WIN 51708, CP-96345 and L-760735.

**Table 74. Percent of inhibition/stimulation of the proliferation of tumor cells from primary human tumors in which AKT amending (their presence is not detected after treatment with NK1 receptor antagonists - said receiver acts through the route of the PI3 Kinases in tumor cells) in culture with said receptor antagonist Aprepitant (1µM) and in culture with stromal cells- human -fibroblasts, and Aprepitant (1µM)**

| **Type of tumor** | **Tumor cells (control)** | **Tumor cells + Aprepitant** | **Tumor cells co-cultivated with fibroblast (control)** | **Tumor cells co-cultivated with fibroblast + Aprepitant** |
|---|---|---|---|---|
| Carcinoma of stomach. | 100 | 51±3 | 100 | 22±3 |
| Carcinoma of Colon. | 100 | 61±3 | 100 | 21±4 |
| Carcinoma of Pancreas. | 100 | 65±4 | 100 | 21±3 |
| Renal carcinoma | 100 | 76±4 | 100 | 20±3 |
| Carcinoma of breast. | 100 | 77±3 | 100 | 21±4 |
| Carcinoma of ovarian. | 100 | 65±4 | 100 | 21±5 |
| Carcinoma of endometrial. | 100 | 66±5 | 100 | 21±8 |
| Carcinoma of cervix. | 100 | 75±3 | 100 | 20±8 |
| Carcinoma of human small cell lung | 100 | 61±4 | 100 | 22±5 |
| Carcinoma of human non-small cell lung | 100 | 66±5 | 100 | 21±7 |
| Carcinoma of thyroid. | 100 | 69±6 | 100 | 20±1 |
| Carcinoma of prostate. | 100 | 69±6 | 100 | 22±7 |
| Glioma | 100 | 68±5 | 100 | 26±4 |
| Sarcoma of Ewing. | 100 | 67±4 | 100 | 25±6 |
| Melanoma | 100 | 67±5 | 100 | 20±7 |
| Neuroblastoma | 100 | 67±5 | 100 | 26±5 |
| Leukemia B | 100 | 69±4 | 100 | 24±4 |
| Leukemia T | 100 | 68±3 | 100 | 25±4 |
| Non-Hodgkin's Lymphoma B | 100 | 62±4 | 100 | 23±6 |
| Non-Hodgkin's Lymphoma T | 100 | 63±3 | 100 | 22±5 |
| Hodgkin's Lymphoma | 100 | 62±4 | 100 | 21±4 |
| Myeloma | 100 | 68±5 | 100 | 20±4 |

**Table 75. Percentage of inhibition/stimulation of the proliferation of tumor cells from primary human tumors in which AKT is not modified (its presence is detected after treatment with NK1 receptor antagonists - said receiver does not act through the path of PI3 Kinases in tumor cells) in culture with said receptor antagonist Aprepitant (1µM) and in culture with stromal cells- human -fibroblasts, and Aprepitant (1µM).**

| **Type of tumor** | **Tumor cells (control)** | **Tumor cells + Aprepitant** | **Tumor cells co-cultivated with fibroblast (control)** | **Tumor cells co-cultivated with fibroblast + Aprepitant** |
|---|---|---|---|---|
| Carcinoma of Colon. | 100 | 100±4 | 100 | 21±5 |
| Carcinoma of Pancreas. | 100 | 101±3 | 100 | 20±4 |
| Renal carcinoma | 100 | 101±4 | 100 | 25±5 |
| Carcinoma of breast. | 100 | 99±5 | 100 | 22±5 |
| Carcinoma of ovarian. | 100 | 102±5 | 100 | 23±4 |
| Carcinoma of endometrial. | 100 | 99±2 | 100 | 18±7 |
| Carcinoma of cervix. | 100 | 101±2 | 100 | 17±7 |
| Carcinoma of human small cell lung | 100 | 102±2 | 100 | 21±6 |
| Carcinoma of human non-small cell lung | 100 | 100±2 | 100 | 18±6 |
| Carcinoma of thyroid. | 100 | 101±3 | 100 | 18±4 |
| Carcinoma of prostate. | 100 | 99±4 | 100 | 21±3 |
| Glioma | 100 | 100±3 | 100 | 25±3 |
| Sarcoma of Ewing. | 100 | 98±4 | 100 | 24±4 |
| Melanoma | 100 | 99±3 | 100 | 26±4 |
| Neuroblastoma | 100 | 100±5 | 100 | 25±4 |
| Leukemia B | 100 | 101±2 | 100 | 22±5 |
| Leukemia T | 100 | 101±2 | 100 | 25±5 |
| Non-Hodgkin's Lymphoma B | 100 | 102±3 | 100 | 22±5 |
| Non-Hodgkin's Lymphoma T | 100 | 102±3 | 100 | 21±6 |
| Hodgkin's Lymphoma | 100 | 99±2 | 100 | 28±5 |
| Myeloma | 100 | 100±1 | 100 | 29±5 |

The results show that in the cases where there was an inhibition of proliferation there was pathway integrity of PI3 Kinase, by determining AKT. In these cases where perceived decreased proliferation is also perceived absence of expression of AKT. This means that NK1 receptor antagonists inhibit proliferation in this group of tumor cells, through the route of the PI3 Kinase. However, in cases where there was decreased proliferation after treatment with NK1 receptor antagonists, the presence of AKT was confirmed, which means that treatment with NK1 receptor antagonists does not inhibit the PI3 Kinase pathway. Therefore, the inhibition of proliferation of tumor cells by the aforementioned antagonists is caused by inhibition, "downstream" of the PI3 Kinase pathway. However, when both types of tumor cells are grown (with integrity PI3-Kinases and thus with no AKT after treatment with NK1 receptor antagonists and altering the path of the PI3 Kinase - and therefore AKT is present after treatment with NK1 receptor antagonist) together with fibroblasts derived from the same patient, they observed an inhibition of proliferation of tumor cells, regardless of the state of the PI3 Kinase pathway. This demonstrates that the non-peptide antagonists inhibit receptor NK1 survival of tumor cells by mechanisms different from those known in the prior art and related to blocking the secretion of substances produced by the interaction of these cells with other cells characteristic of the tumor microenvironment, specifically stromal fibroblast cells.

### Example 16. Treatment with non-peptide NK1 receptors antagonists inhibits proliferation of tumor cells when the receptor acts exclusively via the MAP Kinase pathway. Treatment with non-peptide NK1 receptor antagonists inhibits both the proliferation of these cells when cultured together with cells of inflammation/immunity (polymorphonuclear and mononuclear leukocytes).

The same method described in Example 7 was used to obtain tumor cells from primary human tumors. Individual tumors and patient characteristics are shown in Table 36. Of each of the tumors shown in Table 36, four showed expression of MAP Kinases route after treatment with different non-peptide NK1 antagonists and four others did not express said route after treatment with different non-peptide NK1 antagonists. Similarly, inflammatory cells were obtained/immune cells (leukocytes cop and morfonuclears) to cultivate from skin samples obtained from the same patient from the surgical incision area that was made in the procedure performed to remove their tumor. Similarly to what was done in Example 7, a total of 6 wells containing tumor cells from tumors pathway integrity of MAP Kinases (ERK was absent after treatment with non-peptide NK1 receptor antagonists) were used as control for tumor cell survival of such tumors. Another 6 wells were used as control survival of tumor cells from tumors pathway impairment of MAP Kinases (ERK present after treatment with non-peptide NK1 receptors). Another 6 wells containing tumor cells from tumors pathway integrity of MAP Kinases (ERK was absent after treatment with non-peptide NK1 antagonists) were cultured in the presence of various non-peptidic antagonists of the NK1 receptor. Another 6 wells containing tumor cells from tumors pathway impairment of MAP kinases (ERK-presence after treatment with non-peptide NK1 antagonists) were cultured in the presence of various non-peptidic antagonists of the NK1 receptor. Another 6 wells containing tumor cells from tumors pathway integrity of MAP Kinases (ERK was absent after treatment with non-peptide NK1 antagonists) were cultured in the presence of stromal cells- fibroblasts- and various non-peptide NK1 receptor antagonists. Another 6 wells containing tumor cells from tumors pathway impairment of MAP Kinases (ERK presence after treatment with non-peptide NK1 receptor antagonists) cells were cultured in the presence of inflammatory/immune cells (poly and morfonuclears leukocytes) and various non-peptide NK1 receptor antagonists. Previously, it was found that the tumor cells and inflammatory cells/immune (poly and morfonuclears leukocytes) expressed NK1 receptor by Western blotting. Then a paraffin block with the content of each of the wells was prepared, as described in example 1. To test for proteins belonging to the route of MAP kinases in different cell cultures Western blotting was performed, using as primary antibody: Phospho-p44/42 MAPK (Erkl / 2) (Thr202/Tyr204) (D 13.14.4E) XP ^{®} Rabbit mAb (4370, Cell Signaling). On this occasion, in order to identify the different cell types in order to quantify them immunohistochemistry was performed with labeling with primary antibodies specific for inflammatory cells / immune cells (leukocytes cop and morfonucleares) human (Anti-smooth muscle actin), carcinoma cells (Anti-cytokeratin spectrum) Glioma (Anti-glial fibrillary acidic protein), Ewing's sarcoma (Anti-CD99), Melanoma (Anti-HMB45), leukemias and lymphomas (Anti-leukocyte common antigen) and Myeloma (anti-CD 138). All antibodies are distributed by Dako and used at the concentration at which they are supplied (supplied pre-diluted - "ready to use").

Tables 76 and 77 demonstrate that non-peptide receptor NK1 antagonist, Aprepitant, only inhibits the growth of cells in which said receptor acts through the MAP kinases pathway, whereas cells in which no inhibition is produced, there is no observed modification of this route. However, when tumor cells are co-cultured with cells of inflammation/immunity (polymorphonuclear and mononuclear leukocytes), said antagonist produces proliferation inhibition of tumor cells, whether they originate from tumors or where there is no integrity the route of MAP Kinases "downstream" of the receiver, is, whether or not acting receiver via said signaling pathway in tumor cells. We obtained the same results when other nonpeptide receptor NK1 was used: Vestipitant, Casopitant, L-733,060, L-732,138, L-703, 606, CP-100263, WIN 62,577, WIN 51708, CP-96345 and L- 760735.

**Table 76. Percentage of inhibition of proliferation of tumor cells from primary human tumors amending ERK (their presence is not detected after treatment with NK1 receptor antagonists - said receiver acts through the route of the MAP Kinases in tumor cells) in culture with this receptor antagonist Aprepitant (1µM) and culture with inflammatory/immune (mono and polymorphonuclear leukocytes) human cell and Aprepitant (1µM).**

| **Type of tumor** | **Tumor cells (control)** | **Tumor cells + Aprepitant** | **Tumor cells co-cultivated with leukocytes (control)** | **Tumor cells co-cultivated with leukocytes + Aprepitant** |
|---|---|---|---|---|
| Carcinoma of stomach. | 100 | 51±3 | 100 | 26±4 |
| Carcinoma of Colon. | 100 | 60±5 | 100 | 21±5 |
| Carcinoma of breast | 100 | 76±3 | 100 | 22±6 |
| Carcinoma of ovarian. | 100 | 61±4 | 100 | 23±7 |
| Carcinoma of endometrial | 100 | 65±5 | 100 | 24±5 |
| Carcinoma of human small cell lung | 100 | 67±4 | 100 | 26±6 |
| Carcinoma of human non-small cell lung | 100 | 64±5 | 100 | 22±6 |
| Glioma | 100 | 68±5 | 100 | 24±5 |
| Melanoma | 100 | 64±5 | 100 | 24±6 |

**Table 77. Percent inhibition/stimulation of the proliferation of human tumor cells from primary tumors in which ERK is unchanged (its presence is detected after treatment with NK1 receptor antagonists - said receiver does not act through the MAP Kinase pathway in tumor cells) in culture with said receptor antagonist Aprepitant (1µM) and culture with inflammatory/immune human cell and Aprepitant (1µM).**

| **Type of tumor** | **Tumor cells (control)** | **Tumor cells + Aprepitant** | **Tumor cells co-cultivated with leukocytes (control)** | **Tumor cells co-cultivated with leukocytes + Aprepitant** |
|---|---|---|---|---|
| Carcinoma of stomach. | 100 | 101±3 | 100 | 24±4 |
| Carcinoma of Colon. | 100 | 99±3 | 100 | 25±5 |
| Carcinoma of breast | 100 | 99±3 | 100 | 22±5 |
| Carcinoma of ovarian. | 100 | 98±4 | 100 | 23±4 |
| Carcinoma of endometrial | 100 | 99±4 | 100 | 21±6 |
| Carcinoma of human small cell lung | 100 | 100±4 | 100 | 22±6 |
| Carcinoma of human non-small cell lung | 100 | 98±5 | 100 | 20±6 |
| Glioma | 100 | 98±5 | 100 | 28±5 |
| Melanoma | 100 | 99±5 | 100 | 26±4 |

The results show that in the cases where there was an inhibition of proliferation, there was pathway integrity of the MAP kinases, ERK by determining. In these cases, where decreased proliferation is perceived, [absence is also appreciated ERK expression]. This means that NK1 receptor antagonists inhibit proliferation in this group of tumor cells through the MAP Kinase pathway. However, in cases where there was decreased proliferation after treatment with NK1 receptor antagonists, the presence of ERK was confirmed, which means that treatment with NK1 receptor antagonists does not inhibit the MAP Kinase pathway and therefore that the inhibition of proliferation of tumor cells by the aforementioned antagonists is caused by inhibition, "downstream" of the MAP Kinase pathway. However, when both types of tumor cells are grown (with integrity of the MAP kinases, and therefore with no ERK after treatment with NK1 receptor antagonists - and alteration of the MAP Kinase pathway - and therefore presence of ERK after treatment with NK1 receptor antagonists) with polymorphonuclear and mononuclear leukocytes obtained from the patient, inhibiting proliferation of tumor cells does appear, regardless of the state of the MAP Kinase pathway. This demonstrates that the non-peptide antagonists inhibit receptor NK1 survival of tumor cells by mechanisms different from those known in the prior art and related to blocking the secretion of substances produced by the interaction of these cells with other cells characteristic of the tumor microenvironment, specifically with the cells of inflammation / immunity (mononuclear and polymorphonuclear leukocytes).

### Example 17. Treatment with non-peptide NK1 receptors antagonists inhibits proliferation of tumor cells when the receptor acts exclusively through the PI3 Kinase pathway. Treatment with non-peptide NK1 receptor antagonists inhibits both the proliferation of these cells when cultured together with cells of inflammation/immunity (polymorphonuclear and mononuclear leukocytes).

The same method described in Example 7 was used to obtain tumor cells from primary human tumors. Individual tumors and patient characteristics are shown in Table 36. Of each of the tumors shown in Table 36, four showed expression of PI3 Kinase route after treatment with different non-peptide NK1 antagonists and four others did not express said route after treatment with different non-peptide NK1 antagonists. Similarly, inflammation/immunity cells (polymorphonuclear and mononuclear leukocytes) were obtained to cultivate from skin samples obtained from the same patient in the area of surgical incision during the procedure to remove their tumor. Similarly to what was done in Example 7, a total of 6 wells containing tumor cells from tumors pathway integrity of the PI3 Kinase (AKT was absent after treatment with non-peptide NK1 receptors antagonists) were used as control for tumor cell survival of such tumors. Another 6 wells were used as control survival of tumor cells derived from tumors with alterations via the PI3 Kinase (AKT presence after treatment with non-peptide NK1 receptor antagonists). Another 6 wells containing tumor cells from tumors pathway integrity of the PI3 Kinase (AKT was absent after treatment with non-peptide NK1 receptor antagonists) were cultured in the presence of various non-peptide NK1 receptor antagonists. Another 6 wells containing tumor cells from tumors with altered via the PI3 Kinase (with presence of AKT after treatment with non-peptide NK1 receptor antagonists) were cultured in the presence of various non-peptide NK1 receptor antagonists. Another 6 wells containing tumor cells from tumors pathway integrity of the PI3 Kinase (AKT was absent after treatment with non-peptide NK1 receptor antagonists) were cultured in the presence of stromal cells- fibroblasts- and various non-peptide NK1 receptor antagonists. Another 6 wells containing tumor cells from tumors with alterations via the PI3 Kinase (with presence of AKT after treatment with non-peptide NK1 receptor antagonists) cells were cultured in the presence of inflammation/immunity (polymorphonuclear and mononuclear leukocytes) and several non-peptide NK1 receptor antagonists.

Previously, it was found that the tumor cells and inflammatory cells/immunity (polymorphonuclear and mononuclear leukocytes) receptor expressed NK1 by Western blotting. Then, a paraffin block with the content of each of the wells was prepared, as described in Example 1. Western blotting was performed on AKT in different cell cultures (as described in Example 1-Western Blot-section), but using as the primary antibody with reference: Akt (pan) (11 E7) Rabbit mAb (4685, Cell Signalling). On this occasion, in order to identify the different cell types in order to quantify them, immunohistochemistry was performed with labeling with primary antibodies specific for cells of inflammation / immunity (mononuclear and polymorphonuclear leukocytes) human (Anti-muscle actin Smooth) carcinoma cells (Anti-cytokeratin spectrum) Glioma (Anti-glial fibrillary acidic protein), Ewing's sarcoma (Anti-CD99), Melanoma (Anti-HMB45), leukemias and lymphomas (Anti-leukocyte common antigen ) and myeloma (anti-CD138). All antibodies are distributed by Dako and used at the concentration at which they are supplied (supplied pre-diluted - "ready to use").

Tables 78 and 79 demonstrate that as non-peptide antagonist of the NK1 receptor, Aprepitant only inhibits the growth of cells in which said receptor acts through the PI3 Kinase pathway, whereas in cells that do not produce inhibition, modification of this route is not observed. However, when tumor cells are co-cultured with cells of inflammation/immunity (polymorphonuclear and mononuclear leukocytes), said antagonist produces a proliferation of inhibition of tumor cells, whether they originate from tumors [or where there is no integrity the route of the PI3 Kinase "downstream" of the receiver, is, whether or not acting receiver via said signaling pathway in tumor cells.

We obtained the same results when other non-peptide NK1 receptor antagonists were used: Vestipitant, Casopitant, L-733,060, L-732, 138, L-703,606, CP-100263, WIN 62,577, WIN 51708, CP-96345 and L- 760735.

**Table 78. Percentage inhibition/stimulation of the proliferation of tumor cells from primary human tumors in amending AKT (their presence is not detected after treatment with NK1 receptor antagonists - said receiver acts through the PI3 Kinase pathway in tumor cells) in culture with this receptor antagonist Aprepitant (1µM) and cell culture with inflammation/immunity (mononuclear and polymorphonuclear leukocytes) and human Aprepitant (1µM).**

| **Type of tumor** | **Tumor cells (control)** | **Tumor cells + Aprepitant** | **Tumor cells co-cultivated with leukocytes (control)** | **Tumor cells co-cultivated with leukocytes + Aprepitant** |
|---|---|---|---|---|
| Carcinoma of stomach. | 100 | 56±5 | 100 | 27±5 |
| Carcinoma of Colon. | 100 | 61±6 | 100 | 25±6 |
| Carcinoma of breast | 100 | 75±7 | 100 | 25±7 |
| Carcinoma of ovarian. | 100 | 64±6 | 100 | 29±4 |
| Carcinoma of endometrial | 100 | 63±7 | 100 | 29±6 |
| Carcinoma of human small cell lung | 100 | 66±6 | 100 | 31±7 |
| Carcinoma of human non-small cell lung | 100 | 66±7 | 100 | 33±7 |
| Glioma | 100 | 67±6 | 100 | 34±7 |
| Melanoma | 100 | 66±7 | 100 | 37±6 |

**Table 79. Percentage inhibition / stimulation of the proliferation of human tumor cells from primary tumors in which AKT is unchanged (its presence is detected after treatment with NK1 receptor antagonists - said receiver does not act through the MAP Kinase pathway in tumor cells) in culture with said receptor antagonist Aprepitant (1µM) and cell culture with inflammatory/immunity (mono and polymorphonuclear leukocytes) cells and Aprepitant (1µM).**

| **Type of tumor** | **Tumor cells (control)** | **Tumor cells + Aprepitant** | **Tumor cells co-cultivated with leukocytes (control)** | **Tumor cells co-cultivated with leukocytes + Aprepitant** |
|---|---|---|---|---|
| Carcinoma of stomach. | 100 | 100±5 | 100 | 32±5 |
| Carcinoma of Colon. | 100 | 100±4 | 100 | 26±4 |
| Carcinoma of breast | 100 | 99±5 | 100 | 26±6 |
| Carcinoma of ovarian. | 100 | 98±7 | 100 | 27±7 |
| Carcinoma of endometrial | 100 | 99±8 | 100 | 27±7 |
| Carcinoma of human small cell lung | 100 | 98±6 | 100 | 27±6 |
| Carcinoma of human non-small cell lung | 100 | 99±6 | 100 | 26±7 |
| Glioma | 100 | 100±6 | 100 | 26±8 |
| Melanoma | 100 | 98±6 | 100 | 27±7 |
| Neuroblastoma | 100 | 100±5 | 100 | 32±5 |

The results show that in the cases where there was an inhibition of proliferation there was pathway integrity of PI3 Kinase, by determining AKT. In these cases, where decreased proliferation is perceived, absence of expression of AKT is also noted. This means that NK1 receptor antagonists inhibit proliferation in this group of tumor cells, through the route of the PI3 Kinase. However, in cases where there was decreased proliferation after treatment with NK1 receptor antagonists, the presence of AKT was confirmed, which means that treatment with NK1 receptor antagonists that does not inhibit the PI3 Kinase pathway and therefore that the inhibition of proliferation of tumor cells by the aforementioned antagonists is caused by inhibition, "downstream" of the PI3 Kinase pathway. However, when both types of tumor cells are grown (with integrity PI3-kinases and thus with no AKT after treatment with NK1 receptor antagonists and altering the path of the PI3 Kinase - and therefore AKT is present after treatment with NK1 receptor antagonists) together with polymorphonuclear and mononuclear leukocytes obtained from the patient, inhibition in the proliferation of tumor cells does appear, regardless of the state of the PI3 Kinase pathway. This demonstrates that the non-peptide antagonists of NK1 receptors inhibit the survival of tumor cells by mechanisms different from those known in the prior art and related to blocking the secretion of substances produced by the interaction of these cells with other cells characteristic of the tumor microenvironment, specifically with the cells of inflammation/immunity (mononuclear and polymorphonuclear leukocytes).

### Example 18. Treatment with non-peptide NK1 receptors antagonists inhibits proliferation of tumor cells when the receptor acts exclusively via the MAP Kinases pathway, but not when it acts through this route. Treatment with non-peptide NK1 receptor antagonists inhibits both the proliferation of these cells when cultured together with cells of inflammation/immunity (macrophages).

The same method described in Example 7 was used to obtain tumor cells from primary human tumors. Individual tumors and patient characteristics are shown in Table 36. Of each of the tumors shown in the Table 36, four showed expression of MAP Kinases route after treatment with different non-peptide NK1 antagonists and four others did not express said route after treatment with different non-peptide NK1 antagonists. Similarly, cells were obtained inflammation/immunity (macrophages) to cultivate from skin samples obtained from the same patient in the area of surgical incision during the procedure performed to remove their tumor. Similarly to what was conducted in Example 7, a total of 6 wells containing tumor cells from tumors pathway integrity of MAP Kinases (ERK was absent after treatment with non-peptide NK1 receptors antagonists) were used as control for tumor cell survival of such tumors. Another 6 wells were used as control survival of tumor cells from tumors pathway impairment of MAP Kinases (ERK present after treatment with non-peptide receptor NK1 antagonists). Another 6 wells containing tumor cells from tumors pathway integrity of MAP Kinases (ERK was absent after treatment with non-peptide NK1 receptor antagonists) were cultured in the presence of various non-peptide NK1 receptor antagonists. Another 6 wells containing tumor cells from tumors pathway impairment of MAP Kinases (ERK-presence after treatment with non-peptide NK1 receptor antagonists) were cultured in the presence of various non-peptide NK1 receptor antagonists. Another 6 wells containing tumor cells from tumors pathway integrity of MAP Kinases (ERK was absent after treatment with non-peptide NK1 receptor antagonists) were cultured in the presence of stromal cells-fibroblasts- and various non-peptide NK1 receptor antagonists. Another 6 wells containing tumor cells from tumors pathway impairment of MAP Kinases (ERK-presence after treatment with non-peptide NK1 receptor antagonists) cells were cultured in the presence of inflammation/immunity (macrophages) and various non-peptide NK1 receptor antagonists.

Previously, it was found that the tumor cells and inflammatory/immunity (macrophages) cells expressed NK1 receptor antagonists by Western blotting. Then a paraffin block with the content of each of the wells was prepared, as described in Example 1. Western blotting was performed to test for proteins belonging to the route of MAP kinases in different cell cultures, using as primary antibody: Phospho-p44/42 MAPK (Erkl / 2) (Thr202/Tyr204) (D13 .14.4 E) XP ^{®} Rabbit mAb (4370, Cell Signaling). On this occasion, in order to identify the different cell types in order to quantify them, immunohistochemistry was performed with labeling with primary antibodies specific for inflammatory cells / immunity (macrophages) cells (Anti-smooth muscle actin), cells carcinoma (Anti-cytokeratin spectrum) Glioma (Anti-glial fibrillary acidic protein), Ewing's sarcoma (Anti-CD99), Melanoma (Anti-HMB45), leukemias and lymphomas (Anti-leukocyte common antigen) and myeloma (anti -CD138). All antibodies are distributed by Dako and used at the concentration at which they are supplied (supplied pre-diluted - "ready to use").

In tables 80 and 81, it can be seen that non-peptide NK1 receptor antagonist Aprepitant only inhibits the growth of cells in which said receptor acts through the MAP Kinases pathway, whereas in cells that do not produce inhibition, modification of this route is not observed. However, when tumor cells are co-cultured with inflammation/immunity (macrophages) cells, said antagonist produces a proliferation of inhibition of tumor cells, [whether they originate from tumors or where there is no path integrity of MAP Kinases "downstream" of the receiver, is, whether or not acting receiver via said signaling pathway in tumor cells].

We obtained the same results when other non-peptide NK1 receptor antagonists were used: Vestipitant, Casopitant, L-733,060, L-732,138, L-703,606, CP-100263, WIN 62,577, WIN 51708, CP-96345 and L- 760735.

**Table 80. Percentage inhibition/proliferation of tumor cells from primary human tumors amending ERK (their presence is not detected after treatment with NK1 receptor antagonists - said receiver acts through the MAP Kinase pathway in tumor cells ) in culture with this receptor antagonist Aprepitant (1µM) and 1 culture with inflammatory/immunity (macrophages) human cells and Aprepitant (1µM).**

| **Type of tumor** | **Tumor cells (control)** | **Tumor cells + Aprepitant** | **Tumor cells co-cultivated with macrophages (control)** | **Tumor cells co-cultivated with macrophages + Aprepitant** |
|---|---|---|---|---|
| Carcinoma of stomach. | 100 | 56±6 | 100 | 27±5 |
| Carcinoma of Colon. | 100 | 64±5 | 100 | 24±6 |
| Carcinoma of breast | 100 | 74±7 | 100 | 23±7 |
| Carcinoma of ovarian. | 100 | 67±5 | 100 | 24±6 |
| Carcinoma of endometrial | 100 | 67±4 | 100 | 25±6 |
| Carcinoma of human small cell lung | 100 | 65±7 | 100 | 25±7 |
| Carcinoma of human non-small cell lung | 100 | 66±6 | 100 | 25±5 |
| Glioma | 100 | 64±6 | 100 | 25±6 |
| Melanoma | 100 | 66±7 | 100 | 25±7 |

**Table 81. Percentage inhibition/stimulation of the proliferation of human tumor cells from primary tumors in which ERK is unchanged (its presence is detected after treatment with NK1 receptor antagonists - said receiver does not act through the MAP Kinase pathway in tumor cells) in culture with said receptor antagonist Aprepitant (1µM) and culture with inflammatory/immunity (macrophage) cells and Aprepitant (1µM).**

| **Type of tumor** | **Tumor cells (control)** | **Tumor cells + Aprepitant** | **Tumor cells co-cultivated with macrophages (control)** | **Tumor cells co-cultivated with macrophages + Aprepitant** |
|---|---|---|---|---|
| Carcinoma of stomach. | 100 | 100±5 | 100 | 27±5 |
| Carcinoma of Colon. | 100 | 100±7 | 100 | 28±6 |
| Carcinoma of breast | 100 | 101±6 | 100 | 29±6 |
| Carcinoma of ovarian. | 100 | 99±3 | 100 | 29±7 |
| Carcinoma of endometrial | 100 | 98±7 | 100 | 29±4 |
| Carcinoma of human small cell lung | 100 | 101±6 | 100 | 28±5 |
| Carcinoma of human non-small cell lung | 100 | 102±6 | 100 | 29±5 |
| Glioma | 100 | 99±7 | 100 | 27±7 |
| Melanoma | 100 | 98±6 | 100 | 32±5 |

The results show that in the cases where there was an inhibition of proliferation there was integrity of the MAP Kinase pathway by determining ERK. In these cases, where decreased proliferation is perceived, absence of ERK expression is also noted. This means that NK1 receptor antagonists inhibit proliferation in this group of tumor cells through the MAP Kinase pathway. However, in cases where there was decreased proliferation after treatment with NK1 receptor antagonists, the presence of ERK is confirmed, which means that treatment with NK1 receptor antagonists that does not inhibit the MAP Kinase pathway and therefore that the inhibition of proliferation of tumor cells by the aforementioned antagonists is caused by inhibition, "downstream" of the MAP Kinase pathway. However, when both types of tumor cells are grown (with integrity of the MAP kinases, and therefore with no ERK after treatment with NK1 receptor antagonists - and alteration of the MAP Kinase pathway - and therefore presence of ERK after treatment with NK receptor antagonists I) together with polymorphonuclear and mononuclear leukocytes obtained from the patient, inhibition of proliferation of tumor cells does appear, regardless of the state of the MAP Kinase pathway. This demonstrates that the non-peptide NK1 antagonists receptor inhibit survival of tumor cells by mechanisms different from those known in the prior art and related to blocking the secretion of substances produced by the interaction of these cells with other cells characteristic of the tumor microenvironment, specifically with the cells of inflammation/immunity (macrophages).

### Example 19. Treatment with non-peptide NK1 receptors antagonists inhibits proliferation of tumor cells when the receptor acts exclusively through the PI3 Kinase pathway. Treatment with non-peptide NK1 receptor antagonists inhibits both the proliferation of these cells when cultured together with cells of inflammation/immunity (macrophages).

The same method described in Example 7 was used to obtain tumor cells from primary human tumors. Individual tumors and patient characteristics are shown in Table 36. Of each of the tumors shown in Table 36, four showed expression of PI3 Kinase route after treatment with different non-peptide NK1 antagonists and four others not expressing said route after treatment with different non-peptide NK1 antagonists. Similarly, inflammation/immunity cells (polymorphonuclear and mononuclear leukocytes) were obtained to cultivate from skin samples obtained from the same patient in the area of surgical incision during the procedure to remove their tumor. Similarly to what was conducted in Example 7, a total of 6 wells containing tumor cells from tumors pathway integrity of the PI3 Kinase (AKT was absent after treatment with non-peptide NK1 receptor antagonists) were used as control for tumor cell survival of such tumors. Another 6 wells were used as control survival of tumor cells derived from tumors with alterations via the PI3 Kinase (AKT presence after treatment with non-peptide NK1 receptor antagonists). Another 6 wells containing tumor cells from tumors pathway integrity of the PI3 Kinase (AKT was absent after treatment with non-peptide NK1 receptor antagonists) were cultured in the presence of various non-peptide NK1 receptor antagonists. Another 6 wells containing tumor cells from tumors with alterations via the PI3 Kinase (with presence of AKT after treatment with non-peptide NK1 receptor antagonists) were cultured in the presence of various non-peptide NK1 receptor antagonists. Another 6 wells containing tumor cells from tumors pathway integrity of the PI3 Kinase (AKT was absent after treatment with non-peptide NK1 receptor antagonists) were cultured in the presence of stromal cells, fibroblasts, and various non-peptide antagonists NK1 receptor. Another 6 wells containing tumor cells from tumors with alterations via the PI3 Kinase (with presence of AKT after treatment with non-peptide NK1 receptor antagonists) were cultured in the presence of stromal cells-fibroblasts, and various non-peptide NK1 receptor antagonists.

Previously, it was found that the tumor cells and inflammatory/immunity cells (polymorphonuclear and mononuclear leukocytes) expressed NK1 receptor by Western blotting. Then a paraffin block with the content of each of the wells was prepared, as described in Example 1. Western blotting was performed on AKT in different cell cultures (as described in Example 1-Western Blot-section), but using as the primary antibody with reference: Akt (pan) (11 E7) Rabbit mAb (4685, Cell Signalling). On this occasion, in order to identify the different cell types in order to quantify them, immunohistochemistry was performed with labeling with primary antibodies specific for cells of inflammation immunity (mononuclear and polymorphonuclear leukocytes) human (Anti-muscle actin Smooth), carcinoma cells (Anti-cytokeratin spectrum), Glioma (Anti-glial fibrillary acidic protein), Ewing's sarcoma (Anti-CD99), Melanoma (Anti-HMB45), leukemias and lymphomas (Anti-leukocyte common antigen) and myeloma (anti-CD138). All antibodies are distributed by Dako and used at the concentration at which they are supplied (supplied pre-diluted - "ready to use").

Tables 82 and 83 show that as non-peptide antagonist of the NK1 receptor, Aprepitant only inhibits the growth of cells in which said receptor acts through the PI3 Kinase pathway, whereas in cells that do not produce inhibition, modification of this route is not observed. However, when tumor cells are co-cultured with cells of inflammation/immunity (macrophages), said antagonist produces a proliferation of inhibition of tumor cells, whether they originate from tumors or where there is no path integrity of the PI3 Kinase "downstream" of that receptor, is, whether or not acting receiver via said signaling pathway in tumor cells. We obtained the same results when used other nonpeptide NK1 receptor: Vestipitant, Casopitant, L-733,060, L-732,138, L-703,606, CP-100263, WIN 62,577, WIN 51708, CP-96345 and L-760735.

**Table 82. Percentage inhibition of the proliferation of human tumor cells from primary tumors amending AKT (their presence is not detected after treatment with NK1 receptor antagonists - said receiver acts through the route of the PI3 kinases in cells tumor) in culture with this receptor antagonist aprepitant (1µM) and one culture with inflammatory/immunity human cells (macrophages) and Aprepitant (1µM).**

| **Type of tumor** | **Tumor cells (control)** | **Tumor cells + Aprepitant** | **Tumor cells co-cultivated with macrophages (control)** | **Tumor cells co-cultivated with macrophages + Aprepitant** |
|---|---|---|---|---|
| Carcinoma of stomach. | 100 | 58±7 | 100 | 29±6 |
| Carcinoma of Colon. | 100 | 61±8 | 100 | 29±5 |
| Carcinoma of breast | 100 | 69±9 | 100 | 28±5 |
| Carcinoma of ovarian. | 100 | 69±5 | 100 | 28±5 |
| Carcinoma of endometrial | 100 | 68±6 | 100 | 27±7 |
| Carcinoma of human small cell lung | 100 | 68±6 | 100 | 28±6 |
| Carcinoma of human non-small cell lung | 100 | 69±7 | 100 | 31±6 |
| Glioma | 100 | 68±5 | 100 | 32±7 |
| Melanoma | 100 | 67±6 | 100 | 34±5 |

**Table 83. Percentage inhibition/stimulation of the proliferation of human tumor cells from primary tumors in which AKT is unchanged (its presence is detected after treatment with NK1 receptor antagonists - said receiver does not act through the PI3 Kinase pathway in tumor cells) in culture with said receptor antagonist Aprepitant (1µM) and culture with inflammatory/immunity (macrophage) human cells and Aprepitant (1 µM).**

| **Type of tumor** | **Tumor cells (control)** | **Tumor cells + Aprepitant** | **Tumor cells co-cultivated with macrophages (control)** | **Tumor cells co-cultivated with macrophages + Aprepitant** |
|---|---|---|---|---|
| Carcinoma of stomach. | 100 | 101±6 | 100 | 33±4 |
| Carcinoma of Colon. | 100 | 101±6 | 100 | 34±7 |
| Carcinoma of breast | 100 | 99±7 | 100 | 35±7 |
| Carcinoma of ovarian. | 100 | 100±6 | 100 | 29±7 |
| Carcinoma of endometrial | 100 | 99±8 | 100 | 33±8 |
| Carcinoma of human small cell lung | 100 | 100±7 | 100 | 29±6 |
| Carcinoma of human non-small cell lung | 100 | 100±8 | 100 | 32±8 |
| Glioma | 100 | 100±7 | 100 | 31±6 |
| Melanoma | 100 | 99±7 | 100 | 30±7 |

The results show that in the cases where there was an inhibition of proliferation PI3 Kinase pathway integrity was present, by determining AKT. In these cases where decreased proliferation is perceived, the absence of expression of AKT is also noted. This means that NK1 receptor antagonists inhibit proliferation in this group of tumor cells, through the route of the PI3 Kinase. However, in cases where there was decreased proliferation after treatment with NK1 receptor antagonists, the presence of AKT is confirmed, which means that treatment with NK1 receptor antagonists does not inhibit the PI3 Kinase pathway and therefore that the inhibition of proliferation of tumor cells by the aforementioned antagonists is caused by inhibition, "downstream" of the PI3 Kinase pathway. However, when both types of tumor cells are grown (with integrity PI3-kinases and thus with no AKT after treatment with NK1-receptor antagonists and altering the path of the PI3 Kinase - and therefore AKT is present after treatment with N K1 receptor antagonist) together with polymorphonuclear and mononuclear leukocytes obtained from the patient, inhibition of proliferation of tumor cells does appear, regardless of the state of the PI3 Kinase pathway. This demonstrates that the non-peptide antagonists inhibit receptor NK1 survival of tumor cells by mechanisms different from those known in the prior art and related to blocking the secretion of substances produced by the interaction of these cells with other cells characteristic of the tumor microenvironment, specifically with the cells of inflammation/immunity (macrophages).

### Example 20. The non-peptide NK1 receptor antagonists at low dose do not inhibit the proliferation of tumor cells, however inhibition occurs when cultured in the presence of human fibroblasts and cells of the immune and inflammatory systems.

As explained in the preceding examples, the interaction between tumor cells and stromal cells-fibroblasts-, and immune system cells/inflammatory (mononuclear leucocytes, polymorphonuclear and macrophages leukocytes) alters the microenvironment, by stimulating the secretion of these cells to substances that promote the progression of cancer. As is known in the state of the art, NK1 receptor antagonists can inhibit the proliferation of tumor cells at high doses, however, this effect does not occur when using low doses. In this example it is demonstrated that these cells, when cultured together with stromal cells - fibroblasts, and immune system /inflammatory cells (mononuclear leukocytes, polymorphonuclear leukocytes and macrophages), such antagonists can inhibit proliferation of these tumor cells.

Table 36 presents the tumor cells used, specifying in each case the tumor type to which they correspond. Said tumor cells and fibroblasts (human primary) were obtained according to the methods used and described herein. Before conducting the experiments, it was found that all these cell lines showed NK1 receptor by Western blotting.

In Table 84 it can be seen that non-peptide antagonist of the NK1 receptor, Aprepitant, does not inhibit the growth of tumor cells at low dose (5 nanomolar), however, when tumor cells are co-cultured with stromal- fibroblast- and immune system/inflammatory cells (mononuclear leucocytes, polymorphonuclear leukocytes and macrophages), said antagonist produces a proliferation of inhibition of tumor cells, irrespective of it being used at low doses (no inhibition occurs when these tumor cells were cultured alone in the presence of Aprepitant).

**Table 84. Percentage inhibition/proliferation of primary human tumor cells in culture with said receptor antagonist Aprepitant (5nM) and culture with stromal cells - fibroblasts-, or inflammation/immunity human cells (mononuclear leucocytes, polymorphonuclear leukocytes and macrophages) and Aprepitant (5 nM).**

| **Type of tumor** | **Tumor cells (control)** | **Tumor cells + Aprepitant** | **Tumor cells co-cultivated with fibroblast, leukocytes, macrophages (control)** | **Tumor cells co-cultivated with fibroblast, leukocytes, macrophages + Aprepitant** |
|---|---|---|---|---|
| Carcinoma of stomach. | 100 | 100±2 | 100 | 69±3 |
| Carcinoma of Colon. | 100 | 101±4 | 100 | 68±5 |
| Renal carcinoma | 100 | 99±4 | 100 | 78±4 |
| Carcinoma of breast. | 100 | 100±3 | 100 | 67±5 |
| Carcinoma of ovarian. | 100 | 99±4 | 100 | 78±6 |
| Carcinoma of endometrial | 100 | 99±5 | 100 | 56±6 |
| Carcinoma of human small cell lung | 100 | 101±4 | 100 | 78±6 |
| Carcinoma of human non-small cell lung | 100 | 99±5 | 100 | 79±5 |
| Glioma | 100 | 98±5 | 100 | 81±6 |
| Melanoma | 100 | 101±5 | 100 | 82±5 |

Therefore, the interaction between tumor cells and themselves stromal cells - fibroblast- and immune system/inflammatory cells (mononuclear leucocytes, polymorphonuclear leukocytes and macrophages) modifying the tumor microenvironment, promotes cancer progression. The NK1 receptor antagonists can inhibit tumor cell proliferation by modulating the secretion of substances of importance for survival and cancer progression produced by stromal cells, even at doses which "per se" do not produce direct inhibition in the proliferation of these tumor cells.

### REFERENCES

- Adams GP, Weiner LM. Monoclonal antibody therapy of cancer. Nat Biotechnol. 2005 Sep;23(9):1147-57.
- Barker R. Tachykinins, neurotrophism and neurodegenerative diseases: a critical review on the possible role of tachykinins in the aetiology of CNS diseases. Neurosci. Res., 1996, 7, 187-214.
- Berzofsky JA, et al., J Clin Invest. 2004; 113:1515-1525
- Bigioni M, Benzo A, Irrissuto C, Maggi CA, Goso C. Role of NK-1 and NK-2 tachykinin receptor antagonism on the growth of human breast carcinoma cell line MDA-MB-231. Anticancer Drugs. 2005,16(10):1083-9.
- Bunn PA Jr, Chan D, Stewart J, Gera L, Tolley R, Jewett P, Tagawa M, Alford C, Mochzuki T, Yanaihara N. Effects of neuropeptide analogues on calcium flux and proliferation in lung cancer cell lines. Cancer Res. 1994;54(13):3602-10.
- Carmeliet P, Jain RK. Angiogenesis in cancer and other diseases. Nature. 2000 Sep 14;407(6801):249-57.
- Condeelis J, Pollard JW. Macrophages: obligate partners for tumor cell migration, invasion, and metastasis. Cell. 2006 Jan 27;124(2):263-6.
- Cox G, Jones JL, O'Byrne KJ. Matrix metalloproteinase 9 and the epidermal growth factor signal pathway in operable non-small cell lung cancer. Clin Cancer Res. 2000;6(6):2349-55.
- Coussens LM, Werb Z. Inflammation and cancer. Nature. 2002; 19-26;420(6917):860-7.
- De Bari C, Dell'Accio F, Tylzanowski P, Luyten FP. Multipotent mesenchymal stem cells from adult human synovial membrane. Arthritis Rheum. 2001;44(8):1928-42.
- de Visser KE, Eichten A, Coussens LM. Paradoxical roles of the immune system during cancer development. Nat Rev Cancer. 2006;6(1):24-37.
- Doi T, Kamo I, Imai S, Okanishi S, Ishimaru T, Ikeura Y, Natsugari H. Effects of TAK- 637, a tachykinin receptor antagonist, on lower urinary tract function in the guinea pig. Eur J Pharmacol. 1999;383(3):297-303.
- Fieldng et al., Procc ASCO 2000
- Giardina GA, Gagliardi S, Martinelli M. Antagonists at the neurokinin receptors-Recent patent literature. IDrugs. 2003; 6(8):758-72.
- Hagemann T, Lawrence T. Investigating macrophage and malignant cell interactions in vitro. Methods Mol Biol. 2009;512:325-32.
- Hanahan D, Weinberg RA. The hallmarks of cancer. Cell. 2000 Jan 7;100(1):57-70.
- Ikushima H, Miyazono K. TGF-β signalling: a complex web in cancer progression. Nat Rev Cancer. 2010;10(6):415-24.
- Kramer MS, Cutler N, Feighner J, Shrivastava R, Carman J, Sramek JJ, Reines SA, Liu G, Snavely D, Wyatt-Knowles E, Hale JJ, Mills SG, MacCoss M, Swain CJ, Harrison T, Hill RG, Hefti F, Scolnick EM, Cascieri MA, Chicchi GG, Sadowski S, Williams AR, Hewson L, Smith D, Carlson EJ, Hargreaves RJ, Rupniak NM. Distinct mechanism for antidepressant activity by blockade of central substance P receptors. Science. 1998. 11;281(5383):1640-5.
- Lin WW, Karin M. A cytokine-mediated link between innate immunity, inflammation, and cancer. J Clin Invest. 2007 May;117(5):1175-83.
- Maggi CA, Patacchini R, Rovero R, Giachetti A. Tachykinin Receptor and Tachykinin Receptor Antagonist. Journal of Autonomic Pharmacology. 1993 (13):23-93.
- McAllister SS, Weinberg RA. Tumor-host interactions: a far-reaching relationship. J Clin Oncol. 2010;28(26):4022-8.
- Muñoz M, Rosso M, Robles-Frias MJ, Salinas-Martín MV, Rosso R, González-Ortega A, Coveñas R. The NK-1 receptor is expressed in human melanoma and is involved in the antitumor action of the NK-1 receptor antagonist Aprepitant on melanoma cell lines. Lab Invest. 2010; 90(8):1259-69
- Orosz A, Schrett J, Nagy J, Bartha L, Schön I, Nyéki O. New short-chain analogs of a substance-P antagonist inhibit proliferation of human small-cell lung-cancer cells in vitro and in vivo. Int J Cancer. 1995;60(1):82-7.
- Palma C, Maggi CA. The role of tachykinins via NK1 receptors in progression of human gliomas. Life Sci. 2000;67(9):985-1001.
- Puphanich et al., Procc ASCO 2001
- Quartara L, Maggi CA. The tachykinin NK1 receptor. Part II: Distribution and pathophysiological roles. Neuropeptides. 1998;32(1):1-49.
- Rosemurgy et al., Procc ASCO 1999
- Rosso M, Robles-Frías MJ, Coveñas R, Salinas-Martín MV, Muñoz M. The NK-1 receptor is expressed in human primary gastric and colon adenocarcinomas and is involved in the antitumor action of L-733,060 and the mitogenic action of substance P on human gastrointestinal cancer cell lines. Tumour Biol. 2008;29(4):245-54.
- Singh D, Joshi DD, Hameed M, Qian J, Gascón P, Maloof PB, Mosenthal A, Rameshwar P. Increased expression of preprotachykinin-I and neurokinin receptors in human breast cancer cells: implications for bone marrow metastasis. Proc Natl Acad Sci USA. 2000;97(1):388-93.
- Sparano et al., Procc ASCO 2002
- Welt S, et al., J Clin Oncol 1994

## Claims

1. Use of at least one modifying peritumoral environment agent selected from:
(i) an agent capable of inhibiting vascular cell lineage mediated neoangiogenesis and/or
(ii) an agent capable of inhibiting the synthesis, by cells of the fibroblastic lineage, of the markers selected from any one of the following: TGF-α , TGF-β 1, TGF-β 2, TGF-β 3, SPARC, MMP-3; MMP-7, MMP-9, MMP-11, MMP-13 and MMP-14 and/or
(iii) an agent capable of inhibiting the synthesis, by cells of the immune and/or inflammatory lineage, of the markers selected from any one of the following: TGF-β, NF-kB, EGF, MMP-9, VEGF and TNF-α,
or combinations thereof, for the manufacture of a medicament useful in the treatment of cancer.

2. Use according to claim 1 wherein the modifying peritumoral environment agent is a non-peptide NK1 receptor antagonist.

3. Use according to claim 2 **characterized in that** the non-peptide NK1 receptors antagonists are selected from any one of the following: Aprepitant, Vestipitant, Casopitant, Vofopitant, Ezlopitant, Lanepitant, LY-686017, L-733,060, L-732,138, L-703,606, WIN 62,577, CP-122721, , TAK-637, and R673, CP-100263, WIN 51708, CP-96345, L-760735, CP-122721, L-758298, L-741671, L-742694, CP-99994, T-2328.

4. Use according to claim 3 **characterized in that** the non-peptide NK1 receptor antagonist is selected from any one of the following: Aprepitant, Vestipitant, Casopitant, Vofopitant, Ezlopitant and Lanepitant.

5. Use according to any of claims 1 to 4 **characterized in that** the medicament useful in treating cancer further comprising at least one active substance which induces apoptosis in tumor cells.

6. Use according to claim 5 **characterized in that** the active principle that induces apoptosis in tumor cells is selected from any of one the following: Chlorambucil, Melphalan, Aldesleukin, 6- Mercaptopurine, 5-Fluorouracil, Ara-c, Bexarotene, Bleomycin, Capecitabine, Carboplatin, Cisplatin, Docetaxel, Doxorubicin, Epirubicin, Fludarabine, Irinotecan, Methotrexate, Mitoxantrone Oxaliplatin, Paclitaxel, Rituximab , Vinblastine, Etoposide, Teniposide, Vincristine, Vinorelbine, Imatinib, Erlotinib, Cetuximab and Trastuzumab, or combinations thereof.

7. Use according to any of claims 1 to 6 **characterized in that** the medicament useful in the treatment of cancer is administered simultaneous, separate or sequentially, with at least another anticancer agent chosen from any of the following: a chemotherapy agent or radiotherapy agent.

8. Use according to any of claims 1 to 7 **characterized in that** the vascular lineage cells are vascular endothelial cells, the fibroblast lineage cells are fibroblasts and the immune and/or inflammatory lineage is selected from: mononuclear leucocytes, polymorphonuclear leucocytes and/or macrophages.

9. Use according to any of claims 1 to 8 **characterized in that** the cancer is selected from any one of the following : gastric carcinoma, colon carcinoma, pancreatic carcinoma, breast carcinoma, ovarian carcinoma, endometrial carcinoma, choriocarcinoma, cervix carcinoma, lung carcinoma, thyroid carcinoma, bladder carcinoma, prostate carcinoma, glial CNS carcinoma, sarcoma, melanoma, embryonal carcinoma and hematologic cancers.

10. Composition comprising at least one modifying peritumoral environment agent selected from:
(i) an agent capable of inhibiting vascular cell lineage mediated neoangiogenesis and/or
(ii) an agent capable of inhibiting the synthesis, by cells of the fibroblastic lineage, of markers selected from any one of the following: TGF-α , TGF-β 1, TGF-β 2, TGF-β 3, SPARC, MMP-3; MMP-7, MMP-9, MMP-11, MMP-13 and MMP-14 and/or
(iii) an agent capable of inhibiting the synthesis, by cells of the immune and/or inflammatory lineage, of markers selected from any one of the following: TGF-β, NF-kB, EGF, MMP-9, VEGF and TNF-α, or combinations thereof.

11. Composition according to claim 10 **characterized in that** the modifying peritumoral environment agent is a non-peptide NK1 receptors antagonist.

12. Composition according to claim 11 **characterized in that** the non-peptide NK1 receptors antagonists are selected from any one of the following: Aprepitant, Vestipitant, Casopitant, Vofopitant, Ezlopitant, Lanepitant, LY-686017, L-733,060, L-732,138, L-703,606, WIN 62,577, CP-122721, TAK-637, and R673, CP- 100263, WIN 51708, CP-96345, L-760735, , CP-122721, L-758298, L-741671, L-742694, CP-99994, T-2328.

13. The composition of claim 11 **characterized in that** the non-peptide NK1 receptor antagonist is selected from any one of the following: Aprepitant, Vestipitant, Casopitant, Vofopitant, Ezlopitant and Lanepitant.

14. The composition according to any of claims 10 to 13 **characterized in that** it is a pharmaceutical composition.

15. The composition according to any of claims 10-14 **characterized by** further comprising a pharmaceutically acceptable carrier.

16. The composition according to any of claims 10 to 15 **characterized by** further comprising at least another active ingredient which induces apoptosis in tumor cells.

17. The composition of claim 16 **characterized in that** the active principle that induces apoptosis in tumor cells is selected from any one of the following: Chlorambucil, Melphalan, Aldesleukin, 6- Mercaptopurine, 5-Fluorouracil, Ara-c, Bexarotene, Bleomycin, Capecitabine, Carboplatin, Cisplatin, Docetaxel, Doxorubicin, Epirubicin, Fludarabine, Irinotecan, Methotrexate, Mitoxantrone Oxaliplatin, Paclitaxel, Rituximab, Vinblastine, Etoposide, Teniposide, Vincristine, Vinorelbine, Imatinib, Erlotinib, Cetuximab and Trastuzumab, or combinations thereof.

18. The composition according to any of claims 10-17 **characterized in that** it is administered simultaneous, separate or sequentially, with at least another anticancer agent selected from any of the following: a chemotherapy agent or a radiotherapy agent.

19. The composition according to any of claims 10 to 18 **characterized in that** the cancer is selected from any of the following: gastric carcinoma, colon carcinoma, pancreatic carcinoma, breast carcinoma, ovarian carcinoma, endometrial carcinoma, choriocarcinoma, cervix carcinoma, lung carcinoma, thyroid carcinoma, bladder carcinoma, prostate carcinoma, glial CNS carcinoma, sarcoma, melanoma, embryonal carcinoma and hematologic cancers.

20. Pharmaceutical form comprising a composition according to any one of claims 10 to 19.

21. Use of the composition according to any of claims 10 to 19 or the pharmaceutical form according to claim 20 in the manufacture of a medicament for treating cancer.

22. Combined preparation comprising:
(a) at least one modifying peritumoral environment agent selected from:
(i) an agent capable of inhibiting vascular cell lineage mediated neoangiogenesis and/or
(ii) an agent capable of inhibiting the synthesis, by cells of the fibroblastic lineage, of markers selected from any one of the following: TGF-α , TGF-β 1, TGF-β 2, TGF-β 3, SPARC, MMP-3; MMP-7, MMP-9, MMP-11, MMP-13 and MMP-14 and/or
(iii) an agent capable of inhibiting the synthesis, by cells of the immune and/or inflammatory lineage, of markers selected from any one of the following: TGF-β, NF-kB, EGF, MMP-9, VEGF and TNF-α,
(b) at least a second active ingredient which induces apoptosis in tumor cells.

23. Combined preparation according to claim 22 **characterized in that** the modifying peritumoral environment agent is a non-peptide NK1 receptor antagonist.

24. Combined preparation according to claim 23 **characterized in that** the non-peptide NK1 receptors antagonists are selected from any one of the following: Aprepitant, Vestipitant, Casopitant, Vofopitant, Ezlopitant, Lanepitant, LY-686017, L-733,060, L-732,138, L-703,606, WIN 62,577, CP-122721, TAK-637, and R673, CP-100263, WIN 51708, CP-96345, L-760735, CP-122721, L-758298, L-741671, L-742694, CP-99994, T-2328.

25. Combined preparation according to claim 24 **characterized in that** the non-peptide NK1 receptor antagonist is selected from any one of the following: Aprepitant, Vestipitant, Casopitant, Vofopitant, Ezlopitant and Lanepitant.

26. Combined preparation according to any one of claims 22 to 25 **characterized in that** the active principle that induces apoptosis in tumor cells is selected from any of one the following: Chlorambucil, Melphalan, Aldesleukin, 6- Mercaptopurine, 5-Fluorouracil, Ara-c, Bexarotene, Bleomycin, Capecitabine, Carboplatin, Cisplatin, Docetaxel, Doxorubicin, Epirubicin, Fludarabine, Irinotecan, Methotrexate, Mitoxantrone, Oxaliplatin, Paclitaxel, Rituximab , Vinblastine, Etoposide, Teniposide, Vincristine, Vinorelbine, Imatinib, Erlotinib, Cetuximab and Trastuzumab, or combinations thereof.

27. Combined preparation according to any one of claims 22 to 25 **characterized in that** it is administered simultaneous, separate or sequentially with at least one other anticancer agent selected from any of the following: a chemotherapy agent or a radiotherapy agent.

28. Use simultaneous, separate or sequentially of the active ingredients of the combined preparation according to any one of claims 22 to 27 in the manufacture of a medicament for treating cancer.
